# EUROPEAN PATENT APPLICATION

(11) **EP 2 233 468 A1**
(43) Date of publication of application: **29.09.2010**
(21) Application number: 08858485.9
(22) Date of filing: 10.12.2008
(51) Int. Cl.: C07C 251/24, C07B 53/00, C07B 61/00, C07C 269/00, C07C 269/06, C07C 271/22, C07D 307/33, C07D 307/54, C07D 333/24, C07F 15/04

(54) **OPTICALLY ACTIVE DINICKEL COMPLEX AND METHOD FOR PRODUCING OPTICALLY ACTIVE AMINE USING THE OPTICALLY ACTIVE DINICKEL COMPLEX AS CATALYST**

(30) Priority: 10.12.2007 JP 2007317977; 30.09.2008 JP 2008254293
(71) Applicant: Nissan Chemical Industries, Ltd., Chiyoda-ku Tokyo 101-0054 (JP)
(72) Inventor: SHIBASAKI, Masakatsu, Mitaka-shi Tokyo 181-0013 (JP); MATSUNAGA, Shigeki, Tokyo 130-0005 (JP); CHEN, Zhihua, Tokyo 104-0052 (JP)
(74) Representative: HOFFMANN EITLE
(86) International application number: PCT/JP2008/072411
(87) International publication number: WO 2009/075291

(57) **Abstract**

There is provided a novel optically active dinickel complex and/or a production method of an optically active amine by an asymmetric Mannich reaction using the dinickel complex as a catalyst. An optically active dinickel complex of Formula (I) or Formula (I'): 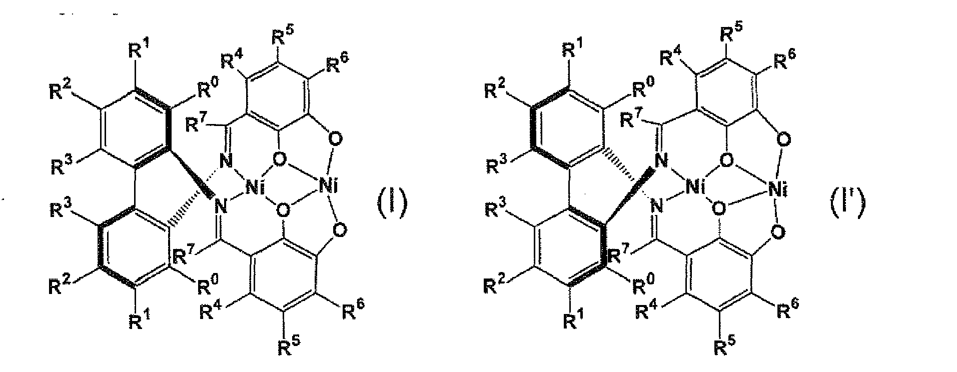 [where R⁰, R¹, R², R³, R⁴, R⁵, R⁶, and R⁷ are each independently a hydrogen atom, a halogen atom, a C₁₋₁₀ alkyl group or a C₁₋₁₀ alkoxy group, etc., R² and R³ together form, together with a benzene ring bonded to them, a naphthalene ring, etc. A novel production method of an optically active amine by an asymmetric Mannich reaction using the dinickel complex as a catalyst.

## Description

### TECHNICAL FIELD

The present invention relates to a novel optically active dinickel complex and/or a production method of an optically active amine by an asymmetric Mannich reaction using the dinickel complex as a catalyst.

### BACKGROUND ART

A salen skeleton is known to form a complex with various transition metals and a salen-metal complex has a catalyst activity for an organic synthesis reaction, so that the utilization of the salen-metal complex is attracting attention. An optically active moiety can be introduced into the salen skeleton, so that a compound in which a metal compound is coordinated or bonded to an optically active salen compound is effective as a catalyst for the production of an optically active compound. Particularly, an optically active salen-metal complex in which two nitrogen atoms and two oxygen atoms are coordination moieties or bonding moieties to a central metal is extremely useful in an asymmetric synthesis reaction (Non-patent Documents 1 and 2).
On the other hand, optically active amino acids and derivatives thereof are used as a raw material for about 20% of the top 500 types of medicines by sales launched in the market. Many medicines are designed based on structures of in vivo molecules, particularly proteins, so that an amino acid analogue that is a component of the proteins is frequently utilized in medicines. It is considered that the ammo acid analogue becomes increasingly significant in the future.
A β-amino-α-substituted ester such as an optically active β-amino-α-nitro ester is useful as a precursor of α, β-diamino acids or a β-amino acid which are a key structure of many physiologically active substances (Non-patent Documents 3 and 4). During the synthesis, high diastereoselectivity or high enantioselectivity is required, and, particularly, the above group of compounds such as an α,α-di-substituted amino acid having a quaternary carbon in the adjacent position is regarded as difficult to be synthesized (Non-patent Document 5).
Conventionally, as a synthesis method of these compounds, a method using mainly an asymmetric Mannich reaction is known (Non-patent Document 6). Recently, the synthesis method is also vigorously studied and, as a catalyst used for the synthesis method, the followings are known.
As an organic base catalyst, a di-functional group cinchonine derivative reported by Dixon et al. (Non-patent Document 7), cinchona alkaloids reported and filed by Boston University (Non-patent Document 8 and Patent Document 1), a dihydroquinidine catalyst reported by Jorgensen et al. (Non-patent Document 9), and the like are known.
As a metal catalyst, a copper-(1,1,1-tris(oxazolinyl)-ethane complex reported by Gade et al. (Non-patent Document 10), a palladium-BINAP derivative complex reported by Sodeoka et al. (Non-patent Document 11), a copper-bisoxazoline complex reported by Jorgensen et al. (Non-patent Documents 12 and 13), and the like are known.
An example of combining a copper-bisoxazoline complex reported by Jorgensen et al. with an organic base catalyst is also known (Non-patent Document 14).
Here, conventionally, some salen complexes containing nickel in the molecule thereof are known (Non-patent Documents 15 and 16). However, an optically active dinickel complex having two nickel atoms in the molecule thereof as in the present invention is not known.

Non-patent Document 1: Chemical Reviews, Vol. 105, No. 5, 1563-1602 (2005)
Non-patent Document 2: Chemical Society Reviews, Vol. 35, 269 (2006)
Non-patent Document 3: Enantioselective Synthesis of β-Amino Acids, Wiley VCH, New York (1997)
Non-patent Document 4: Chemical Reviews, Vol. 105, 3167 (2005)
Non-patent Document 5: Angewandte Chemie International Edition, 40, 4591 (2001)
Non-patent Document 6: Comprehensive Asymmetric Catalysis, Supplement 1, Chapter 29.5, p. 143(2004)
Non-patent Document 7: Chemical Communications, 1191-1193 (2006)
Non-patent Document 8: Angewandte Chemie International Edition, 44, 2896-2899 (2005)
Non-patent Document 9: Organic Letters, Vol. 18, No. 10,2003-2006 (2006)
Non-patent Document 10: Chemical Communications, 5115-5117 (2005)
Non-patent Document 11: Angewandte Chemie International Edition, 44, 1525-1529 (2005)
Non-patent Document 12: Organic Biomolecular Chemistry, Vol. 3, 804-808 (2005)
Non-patent Document 13: Chemistry - A European Journal, Vol. 9, 2359-2367 (2003)
Non-patent Document 14: Organic Biomolecular Chemistry, Vol. 3, 1362-1364 (2005)
Non-patent Document 15: Bulletin of the Chemical Society of Japan, Vol. 76, 327-334 (2003)
Non-patent Document 16: The Journal of Organic Chemistry, Vol. 68, 1973-1981 (2003)
Patent Document 1: WO 2007/011910

### DISCLOSURE OF THE INVENTION

### Problem to be Solved by the Invention

A catalyst used in a conventional synthesis method has had many such problems for synthesizing a β-amino-α-substituted ester such as a β-amino-α-nitro ester, or a β-ainino-α-substituted ketone with high diastereoselectivity or high enantioselectivity that the catalyst has poor substrate versatility, that a low reaction temperature is necessary, and that a protective group used is atypical.

### Means for Solving the Problem

As a result of assiduous research intended to overcome these disadvantages, the inventors of the present invention have found a novel optically active dinickel complex that is stable in air for a long period and is easily handled as the result of designing and synthesizing an optically active salen-metal complex in which two nitrogen atoms and four oxygen atoms are coordinated or bonded to two nickel metal atoms by introducing a 1,2-diphenol structure into a salen structure, and have found that the complex of the present invention exhibits high diastereoselectivity and high enantioselectivity that a conventional dimetal-salen complex cannot exhibit, in an asymmetric Mannich reaction using the complex of the present invention as a catalyst, and provides a product having high optical purity.
Particularly, the inventors of the present invention have found that the catalyst of the present invention has high selectivity and high versatility in comparison with a conventional catalyst in the synthesis of optically active amine analogues having a quaternary asymmetric center such as optically active β-amino-α-substituted esters, β-amino-α-substituted ketones, and β-arnina-α-substituted-cyclic esters, and have completed the present invention. That is, the present invention provides the followings.

### (1)

An optically active dinickel complex of Formula (I) or Formula (I'): 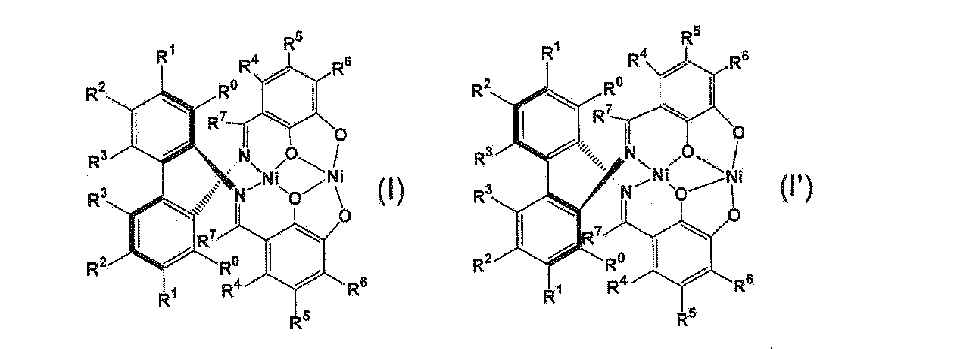 [where R⁰, R¹, R², R³, R⁴, R⁵, R⁶, and R⁷ are each independently a hydrogen atom, a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group, a C₁₋₁₀ alkylcarbonylamino group, a mono or di C₁₋₁₀ alkylamino group, a C₁₋₁₀) alkylsulfonyl group, a C₁₋₁₀ alkylaminasulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₁₋₁₀ alkyl group, the C₂-₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, the C₂₋₉ heterocyclyl group, the C₂₋₁₄ aryl group, and the C₂₋₁₄ aryloxy group are unsubstituted or substituted with one or more substituent(s) that are the same as or different from each other selected from a group V¹ of substituents and a group V² of substituents) or
two adjacent substituents of R⁰ and R¹, R¹ and R², R² and R³, R⁴ and R⁵, or R⁵ and R⁶ together with a benzene ring to which these substituents are bonded form a C₆₋₁₄ polycyclic fused ring (the C₆₋₁₄ polycyclic fused ring is unsubstituted or substituted with one or more substituent(s) that are the same as or different from each other selected from a group V¹ of substituents and a group V² of substituents),
where the group V¹ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V² of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V¹ of substituents)].

### (2)

The optically active dinickel complex according to (1) of Formula (II) or Formula (II'): 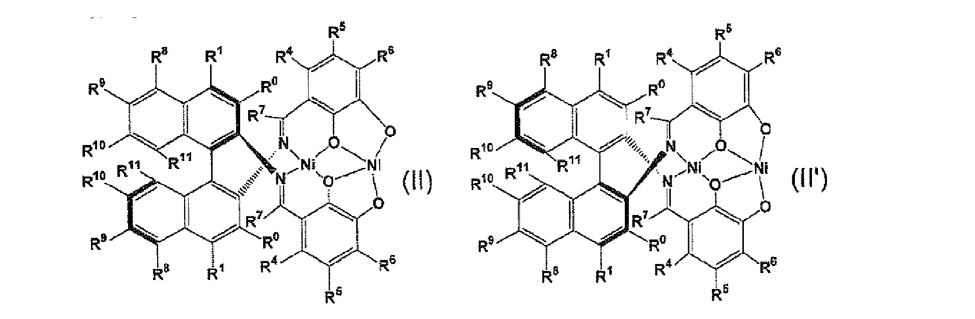 [where R⁰, R¹, R⁴, R⁵, R⁶, R⁷, R⁸, R⁹, R¹⁰, and R¹¹ are each independently a hydrogen atom, a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, the C₂₋₉ heterocyclyl group, the C₂₋₁₄ aryl group, and the C₂₋₁₄ aryloxy group are unsubstituted or substituted with one or more substituent(s) that are the same as or different from each other selected from the group V¹ of substituents and the group V² of substituents) or
two adjacent substituents of R⁰ and R¹, R⁴ and R⁵, R⁵ and R⁶, R⁸ and R⁹, R⁹ and R¹⁰, or R¹⁰ and R¹¹ together with a benzene ring to which these substituents are bonded form a C₆₋₁₄ polycyclic fused ring (the C₆₋₁₄ polycyclic fused ring is unsubstituted or substituted with one or more substituent(s) that are the same as or different from each other selected from the group V¹ of substituents and the group V² of substituents)
and the group V¹ of substituents and the group V² of substituents are the same as those defined in (1)],
two adjacent substituents of R² and R³ together with a benzene ring to which R² and R³ are bonded form a naphthalene ring.

### (3)

The optically active dinickel complex according to (2), wherein R⁰, R¹, R⁸, R⁹, R¹⁰, and R¹¹ are each independently a hydrogen atom, a halogen atom, a C₁₋₁₀ alkyl group, or a C₁₋₁₀ alkoxy group.

### (4)

The optically active dinickel complex according to (3), wherein R⁰, R¹, R⁸, R⁹, R¹⁰, and R¹¹ are a hydrogen atom.

### (5)

The optically active dinickel complex according to any one of (1) to (4), wherein R⁴, R⁵, R⁶, and R⁷ are a hydrogen atom.

### (6)

The optically active dinickel complex according to (1) or (2), wherein R⁴ an R⁵ together form, together with a benzene ring to which R⁴ and R⁵ are bonded, a ring structure of Formula (III) below; and R⁶ and R⁷ are a hydrogen atom.

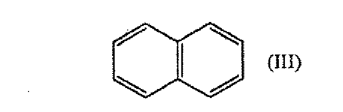

### (7)

The optically active dinickel complex according to (2), wherein R⁴ and R⁵ together form, together with a benzene ring to which R⁴ and R⁵ are bonded, a ring structure of Formula (III) below; R⁶ and R⁷ are a hydrogen atom; and R⁰, R¹, R⁸, R⁹, R¹⁰, and R¹¹ are each independently a hydrogen atom, a halogen atom, a C₁₋₁₀ alkyl group, or a C₁₋₁₀ alkoxy group.

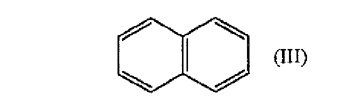

### (8)

The optically active dinickel complex according to (7), wherein R⁰, R¹, R⁸, R⁹, R¹⁰, and R¹¹ are a hydrogen atom.

### (9)

The optically active dinickel complex according to (1), wherein R⁴, R⁵, R⁶, and R⁷ are a hydrogen atom; and R⁰, R¹, R², and R³ are each independently a hydrogen atom, a halogen atom, a C₁₋₁₀ alkyl group, or a C₁₋₁₀ alkoxy group.

### (10)

The optically active dinickel complex according to (1), wherein R⁰, R¹, R⁴, R⁵, R⁶, and R⁷ are a hydrogen atom; and R² and R³ together form, together with a benzene ring to which R² and R³ are bonded, a ring structure of Formula (IV) or Formula (V) below.

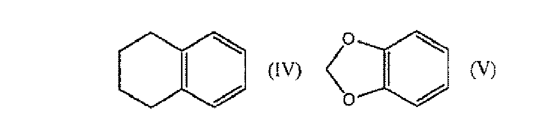

### (11)

The optically active dinickel complex according to (1), wherein R⁰, R¹, R⁶, and R⁷ are a hydrogen atom; R⁴ and R⁵ together form, together with a benzene ring to which R⁴ and R⁵ are bonded, a ring structure of Formula (III) below; and R² and R³ together form, together with a benzene ring to which R² and R³ are bonded, a ring structure of Formula (IV) or Formula (V) below.

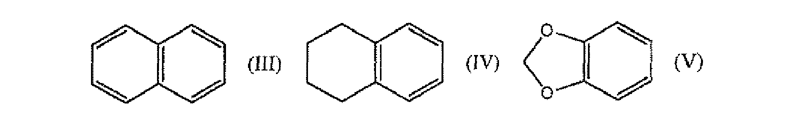

### (12)

A production method of an optically active amine (VIII), characterized by including reacting a N-substituted imine of Formula (VI) with a compound of Formula (VII) using an optically active dinickel complex of Formula (I), (I'), (II), or (II') as a catalyst. 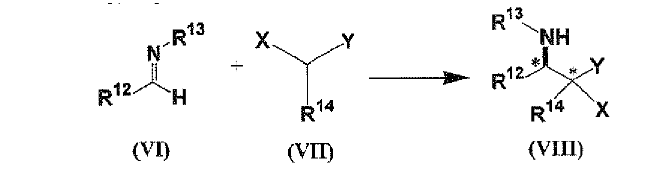 [where
R¹² is a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, a C₇₋₁₄ aralkyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, the C₇₋₁₄ aralkyl group, and the C₂₋₁₄ aryl group are unsubstituted or substituted with one or more substituent(s) that are the same as or different from each other selected from a group V³ of substituents and a group V⁴ of substituents);
R¹³ is a protective group for an amino group, a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents);
R¹⁴ is a hydrogen atom, a halogen atom, a C₁₋₁₀ alkyl group, a C₂₋₆ alkenyl group, a C₂₋₆ alkynyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents);
X and Y are each independently a nitro group, a cyano group, -C(O)R¹⁵, -C(O)OR¹⁵, - C(O)SR¹⁵, -C(S)OR¹⁵, -C(O)NR¹⁵R¹⁶, -S(O)₂R¹⁵, -S(O)₂NR¹⁵R¹⁶, -S(O)R¹⁵, or - P(O)(OR¹⁵)(OR¹⁶)
(where R¹⁵ and R¹⁶ are each independently a hydrogen atom, a C₁₋₁₀ alkyl group, a C₂₋₆ alkenyl group, a C₂₋₆ alkynyl group, a C₁₋₃ halogen-substituted alkyl group, a C₁₋₃ halogen-substituted alkoxy group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₃ halogen-substituted alkyl group, the C₁₋₃ halogen-substituted alkoxy group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents), or R¹⁵ and R¹⁶ together are -(CR¹⁷R¹⁸)ₘ₁-G-(CR¹⁹R²⁰)ₘ₂- (where R¹⁷, R¹⁸, R¹⁹, and R²⁰ are each independently a hydrogen atom, a C₁₋₁₀ alkyl group, a C₁₋₃ halogen-substituted alkyl group, a C₁₋₃ halogen-substituted alkoxy group, a C₂₋₁₄ aryl group, a C₁₋₁₀ alkoxy group, a C₂₋₁₄ aryloxy group, a hydroxy group, or a protected hydroxy group; G is an oxygen atom, a sulfur atom, -CR²¹R²²- (where R²¹ and R²² are each independently a hydrogen atom, a C₁₋₁₀ alkyl group, a C₂₋₁₄ aryl group, a C₁₋₁₀ alkoxy group, a C₂₋₁₄ aryloxy group, a hydroxy group, or a protected hydroxy group), or - NR²³ (where R²³ is a hydrogen atom, a hydroxy group, a formal group, a protective group for an amino group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, and the C₁₋₁₀ alkylcarbonyl group are unsubstituted or substituted with a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, or a C₂₋₁₄ aryloxy group (where the C₂₋₁₄ aryl group and the C₂₋₁₄ aryloxy group are unsubstituted or substituted with a C₁₋₆ alkyl group (the C₁₋₆ alkyl group is unsubstituted or substituted with a halogen atom), or a halogen atom)), or a C₂₋₁₄ aryl group (the C₂₋₁₄ aryl group is unsubstituted or substituted with a C₁₋₁₀ alkyl group (the C₁₋₁₀ alkyl group is unsubstituted or substituted with a halogen atom), a C₂₋₆ alkenyl group, a C₂₋₆ alkynyl group, a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, or a C₂₋₁₄ aryloxy group)); and m1 and m2 are each independently an integer of 0 to 5, where m1+m2 is 3, 4, or 5)),
where the group V³ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀
thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V⁴ of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V¹ of substituents), and the absolute configuration of a carbon atom marked with * means R or S].

### (13)

The production method of the optically active amine according to (12), wherein X is a nitro group and Y is -C(O)OR¹⁵ (where R¹⁵ is the same as defined in (12)).

### (14)

The production method of the optically active amine according to (12), wherein X is -C(O) R¹⁵ and Y is -C(O)OR¹⁵ (where R¹⁵ is the same as defined in (12) and R¹⁵ in X and R¹⁵ in Y are each independently defined).

### (15)

The production method of the optically active amine according to (12), wherein X is a cyano group and Y is -C(O)OR¹⁵ (where R¹⁵ is the same as defined in (12)).

### (16)

The production method of the optically active amine according to (12), wherein X is -P(O)(OR¹⁵)(OR¹⁶) and Y is -C(O)OR¹⁵ (where R¹⁵ and R¹⁶ are the same as defined in (12), and R¹⁵ in X and R¹⁵ in Y are each independently defined).

### (17)

The production method of the optically active amine according to (12), wherein X is -C(O)OR¹⁵ and Y is -C(O)OR¹⁵ (where R¹⁵ is the same as defined in (12), and R¹⁵ in X and R¹⁵ in Y are each independently defined).

### (18)

The production method of the optically active amine according to (12), wherein X is a nitro group and Y is -C(O)R¹⁵ (where R¹⁵ is the same as defined in (12)).

### (19)

The production method of the optically active amine according to (12), wherein X is a cyano group and Y is -C(O)R¹⁵ (where R¹⁵ is the same as defined in (12)).

### (20)

The production method of the optically active amine according to (12), wherein X is -P(O)(OR¹⁵)(OR¹⁶) and Y is -C(O)R¹⁵ (where R¹⁵ and R¹⁶ are the same as defined in (12), and R¹⁵ in X and R¹⁵ in Y are each independently defined).

### (21)

The production method of the optically active amine according to (12), wherein X is -C(O)R¹⁵ and Y is -C(O)R¹⁵ (where R¹⁵ is the same as defined in (12), and R¹⁵ in X and R¹⁵ in Y are each independently defined).

### (22)

A production method of an optically active β-amine-cyclic carbonyl compound (X), characterized by including reacting a N-substituted imine of Formula (VI) with a cyclic compound of Formula (IX) below using an optically active dinickel complex of formula (I), (I'), (II), or (II') as a catalyst. 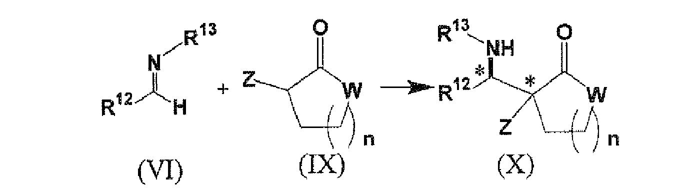 [where R¹² is a C₁₋₁₀ alkyl group, a C₂₋₆ alkynyl group, a C₂₋₆ alkenyl group, a C₁₋₁₀
alkoxycarbonyl group, a C₂₋₉ heterocyclyl group, a C₇₋₁₄ aralkyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, the C₇₋₁₄ aralkyl group, and the C₂₋₁₄ aryl group are unsubstituted or substituted with one or more substituent(s) that are the same as or different from each other selected from a group V⁵ of substituents and a group V⁶ of substituents);
R¹³ is a protective group for an amino group, a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, and the C₂₋₁₄ aryl group are unsubstituted or substituted with one or more substituent(s) that are the same as or different from each other selected from the group V⁵ of substituents and the group V⁶ of substituents);
Z is a nitro group, a cyano group, -C(O)R¹⁵, -C(O)OR¹⁵, -C(O)SR¹⁵, -C(S)OR¹⁵,-C(O)NR¹⁵R¹⁶, -S(O)²R¹⁵, -S(O)²NR¹⁵R¹⁶, -S(O)R¹⁵, or -P(O)(OR¹⁵)(OR¹⁶)
(where R¹⁵ and R¹⁶ are each independently a hydrogen atom, a C₁₋₁₀ alkyl group, a C₂-₆ alkenyl group, a C₂₋₆ alkynyl group, a C₁₋₃ halogen-substituted alkyl group, a C₁₋₃ halogen-substituted alkoxy group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₃ halogen-substituted alkyl group, the C₁₋₃ halogen-substituted alkoxy group, the C₂₋₉ heterocyclyl group, and the C₂₋₁₄ aryl group are unsubstituted or substituted with one or more substituent(s) that are the same as or different from each other selected from the group V⁵ of substituents and the group V⁶ of substituents),
or
R¹⁵ and R¹⁶ together are -(CR¹⁷R¹⁸)ₘ₁-G-(CR¹⁹R²⁰)ₘ₂- (where R¹⁷, R¹⁸, R¹⁹, and R²⁰ are each independently a hydrogen atom, a C₁₋₁₀ alkyl group, a C₂₋₁₄ aryl group, a C₁₋₁₀ alkoxy group, a C₂₋₁₄ aryloxy group, a hydroxy group, or a protected hydroxy group; G is an oxygen atom, a sulfur atom, -CR²¹R²²- (where R²¹ and R²² are each independently a hydrogen atom, a C₁₋₁₀ alkyl group, a C₂₋₁₄ aryl group, a C₁₋₁₀ alkoxy group, a C₁₋₁₄ aryloxy group, a hydroxy group, or a protected hydroxy group), or - NR²³ (where R²³ is a hydrogen atom, a hydroxy group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, and the C₁₋₁₀ alkylcarbonyl group are unsubstituted or substituted with a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, or a C₂₋₁₄ aryloxy group (where the C₂₋₁₄ aryl group and the C₂₋₁₄ aryloxy group are unsubstituted or substituted with a C₁₋₆ alkyl group (the C₁₋₆ alkyl group is unsubstituted or substituted with a halogen atom) or a halogen atom)), or a C₂₋₁₄ aryl group (the C₂₋₁₄ aryl group is unsubstituted or optionally substituted with one or a plurality of substituent(s) selected from a C₁₋₁₀ alkyl group (the C₁₋₁₀ alkyl group is unsubstituted or substituted with a halogen atom), a C₂₋₆ alkenyl group, a C₂₋₆ alkynyl group, a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, and a C₂₋₁₄ aryloxy group)); and m1 and m2 are each independently an integer of 0 to 5, where m1+m2 is 3, 4, or 5));
W is an oxygen atom, a sulfur atom, -NR²⁴, or -C(R²⁴R²⁵)- (where R²⁴ and R²⁵ are a hydrogen atom, a C₁₋₁₀ alkyl group, a C₂₋₆ alkenyl group, a C₂₋₆ alkynyl group, a C₂₋₁₄ aryl group, or a C₁₋₁₀ alkoxycarbonyl group (where the C₁₋₁₀ alkyl group, the C₂₋₆ alkenyl group, the C₂₋₆ alkynyl group, the C₂₋₁₄ aryl group, and the C₁₋₁₀ alkoxycarbonyl group are unsubstituted or substituted with one or more substituent(s) that are the same as or different from each other selected from the group V⁵ of substituents and the group V⁶ of substituents)); and
n is 1, 2, or 3,
where the group V⁵ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V⁶ of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V⁵ of substituents), and the absolute configuration of a carbon atom marked with * means R or S.].

### (23)

The production method of the optically active amine according to (22), wherein Z is a nitro group.

### (24)

The production method of the optically active amine according to (22), wherein Z is a cyano group.

### (25)

The production method of the optically active amine according to (22), wherein Z is -C(O)R¹⁵ (where R¹⁵ is the same as defined in (22)).

### (26)

The production method of the optically active amine according to (22), wherein Z is -C(O)OR¹⁵ (where R¹⁵ is the same as defined in (22)).

### (27)

The production method of the optically active amine according to (22), wherein Z is -P(O)(OR¹⁵)(OR¹⁶) (where R¹⁵ and R¹⁶ are the same as defined in (22)).

### (28)

The production method according to any one of (22) to (27), wherein W is - CH₂- and n is 1 or 2.

### (29)

The production method according to any one of (22) to (27), wherein W is an oxygen atom and n is 1 or 2.

### (30)

The production method according to any one of (12) to (29), wherein, R¹³ in the N-substituted imine of Formula (VI), is a Boc group.

### (31)

The production method according to any one of (12) to (29), wherein, R¹³ in the N-substituted imine of Formula (VI), is a Cbz group.

### (32)

The production method according to any one of (12) to (31), **characterized in that** the reaction is carried out in the presence of an additive having a dehydration activity.

### (33)

The production method according to any one of (12) to (32), wherein the catalyst used in the reaction is the optically active dinickel complex of (1).

### (34)

The production method according to any one of (12) to (32), wherein the catalyst used in the reaction is the optically active dinickel complex as described in (2).

### (35)

The production method according to any one of (12) to (32), wherein the catalyst used in the reaction is the optically active dinickel complex as described in (3).

### (36)

The production method according to any one of (12) to (32), wherein the catalyst used in the reaction is the optically active dinickel complex as described in (4).

### (37)

The production method according to any one of (12) to (32), wherein the catalyst used in the reaction is the optically active dinickel complex as described in (5).

### (38)

The production method according to any one of (12) to (32), wherein the catalyst used in the reaction is the optically active dinickel complex as described in (6).

### (39)

The production method according to any one of (12) to (32), wherein the catalyst used in the reaction is the optically active dinickel complex as described in (7).

### (40)

The production method according to any one of (12) to (32), wherein the catalyst used in the reaction is the optically active dinickel complex as described in (8).

### (41)

The production method according to any one of (12) to (32), wherein the catalyst used in the reaction is the optically active dinickel complex as described in (9).

### (42)

The production method according to any one of (12) to (32), wherein the catalyst used in the reaction is the optically active dinickel complex as described in (10).

### (43)

The production method according to any one of (12) to (32), wherein the catalyst used in the reaction is the optically active dinickel complex as described in (11).

### Effects of the Invention

The present invention can provide a novel optically active dinickel complex that is stable in air for a long period and can be easily handled, and the present invention can further provide a novel production method of an optically active amine having high diastereoselectivity and high enantioselectivity by an asymmetric Mannich reaction using the complex of the present invention as a catalyst.

### BEST MODES FOR CARRYING OUT THE INVENTION

In the compounds used in the present invention, "n" means normal; "i" means iso; "s" means secondary; "tert" and "t" mean tertiary; "c" means cyclo; "o" means ortho; "m" means meta; "p" means para; "Ph" means phenyl; "Me" means methyl; "Et" means ethyl; "Pr" means propyl; and "Bu" means butyl.

First, the terms in the individual substituents described in the present specification such as substituents R¹ to R²⁵ and V¹ to V⁶ are described.

Examples of the halogen atom include fluorine, chlorine, bromine, and iodine.

Examples of the C₁₋₃ alkyl group may include linear C₁₋₃ alkyl groups, branched C₁₋₃ alkyl groups, and C₃ cycloalkyl groups, and include methyl, ethyl, n-propyl, i-propyl, and c-propyl.

Examples of theC₁₋₆alkyl group may include linear C₁₋₆ alkyl groups, branched C₁₋₆ alkyl groups, and C₃₋₆ cycloalkyl groups, and include, besides the above examples, n-butyl, i-butyl, s-butyl, t-butyl, c-butyl, 1-methyl-c-propyl, 2-methyl-c-propyl, n-pentyl, 1-methyl-n-butyl, 2-methyl-n-butyl, 3-methyl-n-butyl, 1,1-diethyl-n-propyl, 1,2-dimethyl-n-propyl, 2,2-dimethyl-n-propyl, 1-ethyl-n-propyl, c-pentyl, 1-methyl-c-butyl, 2-methyl-c-butyl, 3-methyl-c-butyl, 1,2-dimethyl-c-propyl, 2,3-dimethyl-c-propyl, 1-ethyl-c-propyl, 2-ethyl-c-propyl, n-hexyl, 1-methyl-n-pentyl, 2-methyl-n-pentyl, 3-methyl-n-pentyl, 4-methyl-n-pentyl, 1,1-dimethyl-n-butyl, 1,2-dimethyl-n-butyl, 1,3-dimethyl-n-butyl, 2,2-dimethyl-n-butyl, 2,3-dimethyl-n-butyl, 3,3-dimethyl-n-butyl, 1-ethyl-n-butyl, 2-ethyl-n-butyl, 1,1,2-trimethyl-n-propyl, 1,2,2-trimethyl-n-propyl, 1-ethyl-1-methyl-n-propyl, 1-ethyl-2-methyl-n-propyl, c-hexyl, 1-methyl-c-pentyl, 2-methyl-c-pentyl, 3-methyl-c-pentyl, 1-ethyl-c-butyl, 2-ethyl-c-butyl, 3-ethyl-c-butyl, 1,2-dimethyl-c-butyl, 1,3-dimethyl-c-butyl, 2,2-dimethyl-c-butyl, 2,3-dimethyl-c-butyl, 2,4-dimethyl-c-butyl, 3,3-dimethyl-c-butyl, 1-n-propyl-c-propyl, 2-n-propyl-c-propyl, 1-i-prnpyl-c-propyl, 2-i-propyl-c-propyl, 1,2,2-trimethyl-c-propyl, 1,2,3-trimethyl-c-propyl, 2,2,3-trimethyl-c-propyl, 1-ethyl-2-methyl-c-propyl, 2-ethyl-1-methyl-c-propyl, 2-ethyl-2-methyl-c-propyl, and 2-ethyl-3-methyl-c-propyl.

Examples of the C₁₋₁₀ alkyl group may include linear C₁₋₁₀ alkyl groups, branched C₁₋₁₀ alkyl groups, and C₃₋₁₀ cycloalkyl groups, and include, besides the above examples, 1-methyl-1-ethyl-n-pentyl, 1-heptyl, 2-heptyl, 1-ethyl-1,2-dimethyl-n-propyl, 1-ethyl-2,2-dimethyl-n-propyl, 1-octyl, 3-octyl, 4-methyl-3-n-heptyl, 6-methyl-2-n-heptyl, 2-propyl-1-n-heptyl, 2,4,4-trimethyl-1-n-pentyl, 1-nonyl, 2-nonyl, 2,6-dimethyl-4-n-heptyl, 3-ethyl-2,2-dimethyl-3-n-pentyl, 3,5,5-trimethyl-1-n-hexyl, 1-decyl, 2-decyl, 4-decyl, 3,7-dimethyl-1-n-octyl, and 3,7-dimethyl-3-n-octyl.

The C₁₋₃ halogen-substituted alkyl group means a C₁₋₃ alkyl group in which hydrogen atom(s) on a carbon atom is (are) substituted with halogen atom(s), and the alkyl group may be linear C₁₋₃ alkyl groups, branched C₁₋₃ alkyl groups, or C₃ cycloalkyl groups. Specific examples thereof include trifluoromethyl, difluoromethyl, trichloromethyl, dichloromethyl, difluorochloromethyl, pentafluoroethyl, n-heptafluoropropyl, i-heptafluoropropyl, and c-pentafluoropropyl.

Examples of the C₂₋₆ alkynyl group include ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 1-n-propyl-2-propynyl, 2-ethyl-3-butynyl, 1-methyl-1-ethyl-2-propynyl, and 1-i-propyl-2-propynyl.

Examples of the C₂₋₆ alkenyl group may include linear C₂₋₆ alkenyl groups, branched C₂₋₆ alkenyl groups, and C₃₋₆ cycloalkenyl groups, and include ethenyl, 1-propenyl, 2-propenyl, 1-methyl-1-ethenyl, 1-butenyl, 2-butenyl, 3-butenyl, 2-methyl-1-propenyl, 2-methyl-2-propenyl, 1-ethyl ethenyl, 1-methyl-1-propenyl, 1-methyl-2-propenyl, 1-pentenyl, 2-pentenyl, 3-pentenyl, 4-pentenyl, 1-n-propyl ethenyl, 1-methyl-1-butenyl, 1-methyl-2-butenyl, 1-methyl-3-butenyl, 2-ethyl-2-propenyl, 2-methyl-1-butenyl, 2-methyl-2-butenyl, 2-methyl-3-butenyl, 3-methyl-1-butenyl, 3-methyl-2-butenyl, 3-methyl-3-butenyl, 1,1-dimethyl-2-propenyl, 1-i-propyl ethenyl, 1,2-dimethyl-1-propenyl, 1,2-dimethyl-2-propenyl, 1-c-pentenyl, 2-c-pentenyl, 3-c-pentenyl, 1-hexenyl, 2-hexenyl, 3-hexenyl, 4-hexenyl, 5-hexenyl, 1-methyl-1-pentenyl, 1-methyl-2-pentenyl, 1-methyl-3-pentenyl, 1-methyl-4-pentenyl, 1-n-butyl ethenyl, 2-methyl-1-pentenyl, 2-methyl-2-pentenyl, 2-methyl-3-pentenyl, 2-methyl-4-pentenyl, 2-n-propyl-2-propenyl, 3-methyl-1-pentenyl, 3-methyl-2-pentenyl, 3-methyl-3-pentenyl, 3-methyl-4-pentenyl, 3-ethyl-3-butenyl, 4-methyl-1-pentenyl, 4-methyl-2-pentenyl, 4-methyl-3-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1-methyl-2-ethyl-2-propenyl, 1-s-butyl ethenyl, 1,3-dimethyl-1-butenyl, 1,3-dimethyl-2-butenyl, 1,3-dimethyl-3-butenyl, 1-i-butyl ethenyl, 2,2-dimethyl-3-butenyl, 2,3-dimethyl-1-butenyl, 2,3-dimethyl-2-butenyl, 2,3-dimethyl-3-butenyl, 2-i-propyl-2-propenyl, 3,3-dimethyl-1-butenyl, 1-ethyl-1-butenyl, 1-ethyl-2-butenyl, 1-ethyl-3-butenyl, 1-n-propyl-1-propenyl, 1-n-propyl-2-propenyl, 2-ethyl-1-butenyl, 2-ethyl-2-butenyl, 2-ethyl-3-butenyl, 1,1,2-trimethyl-2-propenyl, 1-t-butyl ethenyl, 1-methyl-1-ethyl-2-propenyl, 1 -ethyl-2-methyl-1-propenyl, 1-ethyl-2-methyl-2-propenyl, 1-i-propyl-1-propeny, 1-i-propyl-2-propenyl, 1-methyl-2-c-pentenyl, 1-methyl-3-c-pentenyl, 2-methyl-1-c-pentenyl, 2-methyl-2-c-pentenyl, 2-methyl-3-c-pentenyl, 2-methyl-4-c-pentenyl, 2-methyl-5-c-pentenyl, 2-methylene-c-pentyl, 3-methyl-1-c-pentenyl, 3-methyl-2-c-pentenyl, 3-methyl-3-c-pentenyl, 3-methyl-4-c-pentenyl, 3-methyl-5-c-pentenyl, 3-methylene-c-pentyl, 1-c-hexenyl, 2-c-hexenyl, and 3-c-hexenyl.

The C₂₋₁₄ aryl group includes C₆₋₁₄ aryl groups which contain no hetero atom as a ring member atom, C₂₋₉ aromatic heterocycle groups, and C₂₋₁₄ fused polycyclic groups. The C₂₋₉ aromatic heterocycle group includes C₂₋₆ monocyclic heterocycle groups having a 5- to 7-membered ring and capable of containing one to three atom(s) of an oxygen atom, a nitrogen atom, and/or a sulfur atom individually or in combination thereof, and C₅₋₉ fused bicyclic heterocycle groups having the number of ring member atoms of 8 to 10.

Examples of the C₆₋₁₄ aryl group containing no hetero atom include a phenyl group, a 1-indenyl group, a 2-indenyl group, a 3-indenyl group, a 4-indenyl group, a 5-indenyl group, a 6-indenyl group, a 7-indenyl group, an α-naphthyl group, a β-naphthyl group, a 1-tetrahydronaphthyl group, a 2-tetrahydronaphthyl group, a 5-tetrahydronaphthyl group, a 6-tetrahydronaphthyl group, an o-biphenylyl group, a m-biphenylyl group, a p-biphenylyl group, a 1-anthryl group, a 2-anthryl group, a 9-anthryl group, a 1-phenanthryl group, a 2-phenanthryl group, a 3-phenanthryl group, a 4-phenanthryl group, and a 9-phenanthryl group.

Examples of the C₂₋₆ monocyclic heterocycle group having a 5- to 7-membered ring include a 2-thienyl group, a 3-thienyl group, a 2-furyl group, a 3-furyl group, a 2-pyranyl group, a 3-pyranyl group, a 4-pyranyl group, a 1-pyrrolyl group, a 2-pyrrolyl group, a 3-pyrrolyl group, a 1-imidazolyl group, a 2-imidazolyl group, a 4-imidazolyl group, a 1-pyrazolyl group, a 3-pyrazolyl group, a 4-pyrazolyl group, a 2-thiazolyl group, a 4-thiazolyl group, a 5-thiazolyl group, a 3-isothiazolyl group, a 4-isothiazolyl group, a 5-isothiazolyl group, a 1-1,2,4-triazole group, a 3-1,2,4-triazole group, a 5-1,2,4-triazole group, a 1-1,2,3-triazole group, a 4-1,2,3-triazole group, a 5-1,2,3-triazole group, a 2-oxazolyl group, a 4-oxazolyl group, a 5-oxazolyl group, a 3-isoxazolyl group, a 4-isoxazolyl group, a 5-isoxazolyl group, a 2-pyridyl group, a 3-pyridyl group, a 4-pyridyl group, a 2-pyrazinyl group, a 2-pyrimidinyl group, a 4-pyrimidinyl group, a 5-pyrimidinyl group, a 3-pyridazinyl group, a 4-pyridazinyl group, a 2-1,3,4-oxadiazolyl group, a 2-1,3,4-thiadiazolyl group, a 3-1,2,4-oxadiazolyl group, a 5-1,2,4-oxadiazolyl group, a 3-1,2,4-thiadiazolyl group, a 5-1,2,4-thiadiazolyl group, a 3-1,2,5-oxadiazolyl group, and a 3-1,2,5-thiadiazolyl group.

Examples of the C₅₋₉ fused bicyclic heterocycle group having the number of ring member atoms of 8 to 10 include a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 4-isobenzofuranyl group, a 5-isobenzofuranyl group, a 2-benzothienyl group, a 3-benzothienyl group, a 4-benzothienyl group, a 5-benzothienyl group, a 6-benzothienyl group, a 7-benzothienyl group, a 1-isobenzothienyl group, a 4-isobenzothienyl group, a 5-isobenzothienyl group, a 2-chromenyl group, a 3-chromenyl group, a 4-chromenyl group, a 5-chromenyl group, a 6-chromenyl group, a 7-chrormenyl group, a 8-chromenyl group, a 1-indolizinyl group, a 2-indolizinyl group, a 3-indolizinyl group, a 5-indolizinyl group, a 6-indolizinyl group, a 7-indolizinyl group, a 8-indolizinyl group, a 1-isoindolyl group, a 2-isoindolyl group, a 4-isoindolyl group, a 5-isoindolyl group, a 1-indolyl group, a 2-indolyl group, a 3-indolyl group, a 4-indolyl group, a 5-indolyl group, a 6-indolyl group, a 7-indolyl group, a 1-indazolyl group, a 2-indazolyl group, a 3-indazolyl group, a 4-indazolyl group, a 5-indazolyl group, a 6-indazolyl group, a 7-indazolyl group, a 1-purinyl group, a 2-purinyl group, a 3-purinyl group, a 6-purinyl group, a 7-purinyl group, a 8-purinyl group, a 2-quinolyl group, a 3-quinolyl group, a 4-quinolyl group, a 5-quinolyl group, a 6-quinolyl group, a 7-quinolyl group, a 8-quinolyl group, a 1-isoquinolyl group, a 3-isoquinolyl group, a 4-isoquinolyl group, a 5-isoquinolyl group, a 6-isoquinolyl group, a 7-isoquinolyl group, a 8-isoquinolyl group, a 1-phthalazinyl group, a 5-phthalazinyl group, a 6-phthalazinyl group, a 1-2,7-naphthyridinyl group, a 3-2,7-naphthyridinyl group, a 4-2,7-naphthyridinyl group, a 1-2,6-naphthyridinyl group, a 3-2,6-naphthyridinyl group, a 4-2,6-naphthyridinyl group, a 2-1,8-naphthyridinyl group, a 3-1,8-naphthyridinyl group, a 4-1,8-naphthyridinyl group, a 2-1,7-naphthyridinyl group, a 3-1,7-naphthyridinyl group, a 4-1,7-naphthyridinyl group, a 5-1,7-naphthyridinyl group, a 6-1,7-naphthyridinyl group, a 8-1,7-naphthyridinyl group, a 2-1,6-naphthyridinyl group, a 3-1,6-naphthyridinyl group, a 4-1,6-naphthyridinyl group, a 5-1,6-naphthyridinyl group, a 7-1,6-naphthyridinyl group, a 8-1,6-naphthyridinyl group, a 2-1,5-naphthyridinyl group, a 3-1,5-naphthyridinyl group, a 4-1,5-naphthyridinyl group, a 6-1,5-naphthyridinyl group, a 7-1,5-naphthyridinyl group, a 8-1,5-naphthyridinyl group, a 2-quinoxalinyl group, a 5-quinoxalinyl group, a 6-quinoxalinyl group, a 2-quinazolinyl group, a 4-quinazolinyl group, a 5-quinazolinyl group, a 6-quinazolinyl group, a 7-quinazolinyl group, a 8-quinazolinyl group, a 3-cinnolinyl group, a 4-cinnolinyl group, a 5-cinnolinyl group, a 6-cinnolinyl group, a 7-cinnolinyl group, a 8-cinnolinyl group, a 2-pteridinyl group, a 4-pteridinyl group, a 6-pteridinyl group, and a 7-pteridinyl group.

The C₂₋₁₄ fused polycyclic group is a fused bicyclic group or a fused tricyclic group in which a C₂₋₉ heterocyclyl group is ring-fused to the C₆₋₁₄ aryl group containing no hetero atom and having the number of ring member carbons of 12 or less or to a C₂₋₉ aromatic heterocycle group, and specific examples thereof include:

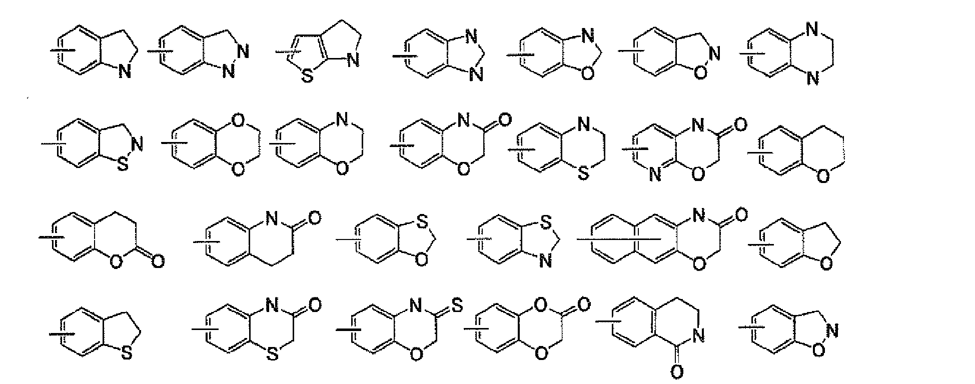

The C₇₋₁₄ aralkyl group is a linear or branched alkyl group having, as a substituent, a monocyclic or polycyclic aromatic hydrocarbon group, or a group that is produced by a ring-fusion of a monocyclic or polycyclic aromatic hydrocarbon group with a linear or branched alkyl group and that has a total number of carbon atoms of 7 to 14, and examples thereof include a benzyl group, a phenethyl group, a phenylpropyl group, a naphthylmethyl group, an indanyl group, a (2H)-indenyl group, and a tetrahydronaphthyl group. Examples of the C₂₋₁₄ aryloxy group include a C₆₋₁₄ aryloxy group containing no hetero atom as a ring member atom and a C₂₋₉ aromatic heterocycle oxy group, and examples of the C₂₋₉ aromatic heterocycle oxy group include a C₂₋₆ monocyclic heterocycle oxy group having a 5- to 7-membered ring and capable of containing one to three atom(s) of an oxygen atom, a nitrogen atom, and/or a sulfur atom individually or in combination thereof, and a C₅₋₉ fused bicyclic heterocycle oxy group having the number of ring member atoms of 8 to 10.

Examples of the C₆₋₁₄ aryloxy group containing no hetero atom include a phenyloxy group, a 1-indenyloxy group, a 2-indenyloxy group, a 3-indenyloxy group, a 4-indenyloxy group, a 5-indenyloxy group, a 6-indenyloxy group, a 7-indenyloxy group, an α-naphthyloxy group, a β-naphthyloxy group, a 1-tetrahydronaphthyloxy group, a 2-tetrahydronaphthyloxy group, a 5-tetrahydronaphthyloxy group, a 6-tetrahydronaphthyloxy group, an o-biphenylyloxy group, a m-biphenylyloxy group, a p-biphenylyloxy group, a 1-anthryloxy group, a 2-anthryloxy group, a 9-anthryloxy group, a 1-phenanthryloxy group, a 2-phenanthryloxy group, a 3-phenanthryloxy group, a 4-phenanthryloxy group, and a 9-phenanthryloxy group.

Examples of the C₂₋₆ monocyclic heterocycle oxy group having a 5- to 7-membered ring include a 2-thienyloxy group, a 3-thienyloxy group, a 2-furyloxy group, a 3-furyloxy group, a 2-pyranyloxy group, a 3-pyranyloxy group, a 4-pyranyloxy group, a 1-pyrrolyloxy group, a 2-pyrrolyloxy group, a 3-pyrrolyloxy group, a 1-imidazolyloxy group, a 2-imidazolyloxy group, a 4-imidazolyloxy group, a 1-pyrazolyloxy group, a 3-pyrazolyloxy group, a 4-pyrazolyloxy group, a 2-thiazolyloxy group, a 4-thiazolyloxy group, a 5-thiazolyloxy group, a 3-isothiazolyloxy group, a 4-isothiazolyloxy group, a 5-isothiazolyloxy group, a 2-oxazolyloxy group, a 4-oxazolyloxy group, a 5-oxazolyloxy group, a 3-isoxazolyloxy group, a 4-isoxazolyloxy group, a 5-isoxazolyloxy group, a 2-pyridyloxy group, a 3-pyridyloxy group, a 4-pyridyloxy group, a 2-pyrazinyloxy group, a 2-pyrimidinyloxy group, a 4-pyrimidinyloxy group, a 5-pyrimidinyloxy group, a 3-pyridazinyloxy group, a 4-pyridazinyloxy group, a 2-1,3,4-oxadiazolyloxy group, a 2-1,3,4-thiadiazolyloxy group, a 3-1,2,4-oxadiazolyloxy group, a 5-1,2,4-oxadiazolyloxy group, a 3-1,2,4-thiadiazolyloxy group, a 5-1,2,4-thiadiazolyloxy group, a 3-1,2,5-oxadiazolyloxy group, and a 3-1,2,5-thiadiazolyloxy group.

Examples of the C₅₋₉ fused bicyclic heterocycle oxy group having the number of ring member atoms of 8 to 10 include a 2-benzofuranyloxy group, a 3-benzofuranyloxy group, a 4-benzofuranyloxy group, a 5-benzofuranyloxy group, a 6-benzofuranyloxy group, a 7-benzofuranyloxy group, a 1-isobenzofuranyloxy group, a 4-isobenzofuranyloxy group, a 5-isobenzofuranyloxy group, a 2-benzothienyloxy group, a 3-benzothienyloxy group, a 4-benzothienyloxy group, a 5-benzothienyloxy group, a 6-benzothienyloxy group, a 7-benzothienyloxy group, a 1-isobenzothienyloxy group, a 4-isobenzothienyloxy group, a 5-isobenzothienyloxy group, a 2-chromenyloxy group, a 3-chromenyloxy group, a 4-chromenyloxy group, a 5-chromenyloxy group, a 6-chromenyloxy group, a 7-chromenyloxy group, a 8-chromenyloxy group, a 1-indolizinyloxy group, a 2-indolizinyloxy group, a 3-indolizinyloxy group, a 5-indolizinyloxy group, a 6-indolizinyloxy group, a 7-indolizinyloxy group, a 8-indolizinyloxy group, a 1-isomdolyloxy group, a 2-isoindolyloxy group, a 4-isoindolyloxy group, a 5-isoindolyloxy group, a 1-indolyloxy group, a 2-indolyloxy group, a 3-indolyloxy group, a 4-indolyloxy group, a 5-indolyloxy group, a 6-indolyloxy group, a 7-indolyloxy group, 1-indazolyloxy group, a 2-indazolyloxy group, a 3-indazolyloxy group, a 4-indazolyloxy group, a 5-indazolyloxy group, a 6-indazolyloxy group, a 7-indazolyloxy group, a 1-purinyloxy group, a 2-purinyloxy group, a 3-purinyloxy group, a 6-purinyloxy group, a 7-purinyloxy group, a 8-purinyloxy group, a 2-quinolyloxy group, a 3-quinolyloxy group, a 4-quinolyloxy group, a 5-quinolyloxy group, a 6-quinolyloxy group, a 7-quinolyloxy group, a 8-quinolyloxy group, a 1-isoquinolyloxy group, a 3-isoquinolyloxy group, a 4-isoquinolyloxy group, a 5-isoquinolyloxy group, a 6-isoquinolyloxy group, a 7-isoquinolyloxy group, a 8-isoquinolyloxy group, a 1-phthalazinyloxy group, a 5-phthalazinyloxy group, a 6-phthalazinyloxy group, a 1-2,7-naphthyridinyloxy group, a 3-2,7-naphthyridinyloxy group, a 4-2,7-naphthyridinyloxy group, a 1-2,6-naphthyridinyloxy group, a 3-2,6-naphthyridinyloxy group, a 4-2,6-naphthyridinyloxy group, a 2-1,8-naphthyridinyloxy group, a 3-1,8-naphthyridinyloxy group, a 4-1,8-naphthyridinyloxy group, a 2-1,7-naphthyridinyloxy group, a 3-1,7-naphthyridinyloxy group, a 4-1,7-naphthyridinyloxy group, a 5-1,7-naphthyridinyloxy group, a 6-1,7-naphthyridinyloxy group, a 8-1,7-naphthyridinyloxy group, 2-1,6-naphthyridinyloxy group, a 3-1,6-naphthyridinyloxy group, a 4-1,6-naphthyridinyloxy group, a 5-1,6-naphthyridinyloxy group, a 7-1,6-naphthyridinyloxy group, a 8-1,6-naphthyridinyloxy group, a 2-1,5-naphthyridinyloxy group, a 3-1,5-naphthyridinyloxy group, a 4-1,5-naphthyridinyloxy group, a 6-1,5-naphthyridinyloxy group, a 7-1,5-naphthyridinyloxy group, a 8-1,5-naphthyridinyloxy group, a 2-quinoxalinyloxy group, a 5-quinoxalinyloxy group, a 6-quinoxalinyloxy group, a 2-quinazolinyloxy group, a 4-quinazolinyloxy group, a 5-quinazolinyloxy group, a 6-quinazolinyloxy group, a 7-quinazolinyloxy group, a 8-quinazolinyloxy group, a 3-cinnolinyloxy group, a 4-cinnolinyloxy group, a 5-cinnolinyloxy group, a 6-cinnolinyloxy group, a 7-cinnolinyloxy group, a 8-cinnolinyloxy group, a 2-pteridinyloxy group, a 4-pteridinyloxy group, a 6-pteridinyloxy group, and a 7-pteridinyloxy group.
Examples of the C₂₋₉ heterocyclyl group include monocyclic and fused-ring bicyclic heterocycle groups containing one or more atom(s) optionally selected from a nitrogen atom, an oxygen atom, and a sulfur atom and two to nine carbon atoms, and specific examples thereof include:

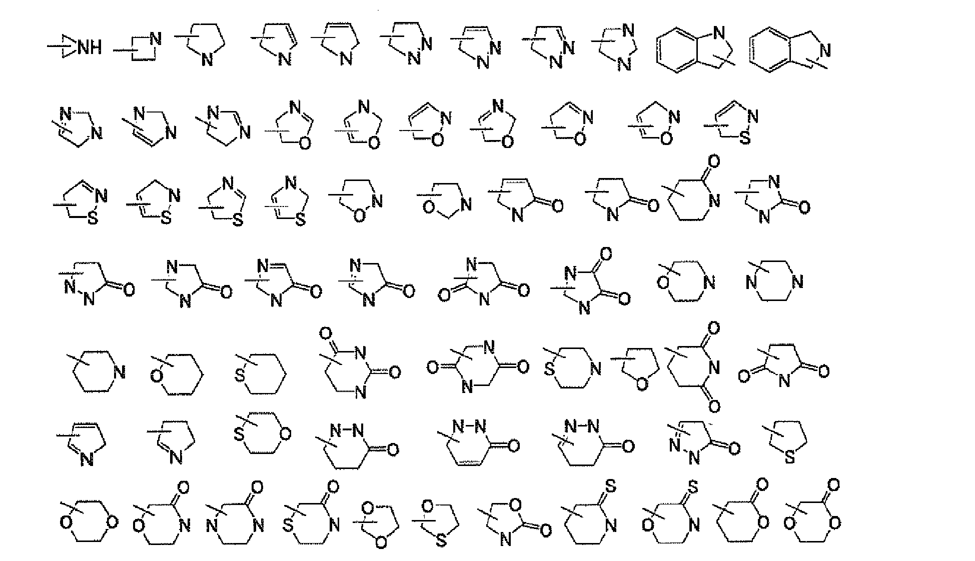

Examples of the C₆₋₁₄ polycyclic fused-ring include C₆₋₁₄ fused polycyclic rings and C₆₋₉ aromatic heterocycles both of which contain no hetero atom as a ring member atom. Examples of the C₆₋₉ aromatic heterocycle include rings in which a 5-to 7-membered ring capable of containing one to three atom(s) of an oxygen atom, a nitrogen atom, and/or a sulfur atom individually or in combination thereof is ring-fused to a benzene ring. Examples of the C₆₋₁₄ polycyclic fused-ring also include fused bicyclic groups and fused tricyclic groups in which a C₂₋₉ heterocyclyl group is ring-fused to a benzene ring, and specific examples thereof include:

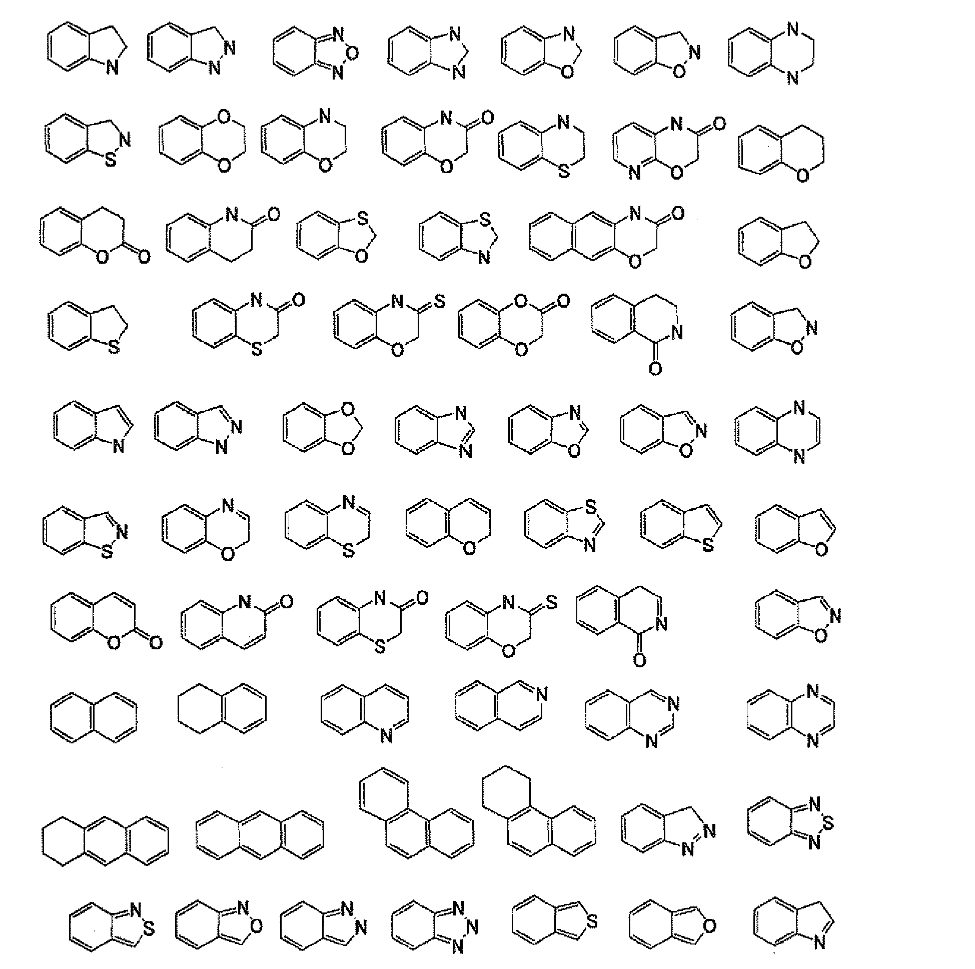

Examples of the C₁₋₁₀ alkylcarbonyl group may include linear C₁₋₁₀ alkylcarbonyl groups, branched C₁₋₁₀ alkylcarbonyl groups, and C₃₋₁₀ cycloalkylcarbonyl groups, and include methylcarbonyl, ethylcarbonyl, n-propylcarbonyl, i-propylcarbonyl, c-propylcarbonyl, n-butylcarbonyl, i-butylcarbonyl, s-butylcarbonyl, t-butylcarbonyl, c-butylcarbonyl, 1-methyl-c-propylcarbonyl, 2-methyl-c-propylcarbonyl, n-pentylcarbonyl, 1-methyl-n-butylcarbonyl, 2-methyl-n-butylcarbonyl, 3-methyl-n-butylcarbonyl, 1,1-dimethyl-n-propylcarbonyl, 1,2-dimethyl-n-propylcarbonyl, 2,2-dimethyl-n-propylcarbonyl, 1-ethyl-n-propylcarbonyl, c-pentylcarbonyl, 1-methyl-c-butylcarbonyl, 2-methyl-c-butylcarbonyl, 3-methyl-c-butylcarbonyl, 1,2-dimethyl-c-propylcarbonyl, 2,3-dimethyl-c-propylcarbonyl, 1-ethyl-c-propylcarbonyl, 2-ethyl-c-propylcarbonyl, n-hexylcarbonyl, 1-methyl-n-pentylcarbonyl, 2-methyl-n-pentylcarbonyl, 3-methyl-n-pentylearbonyl, 4-methyl-n-pentylcarbonyl, 1,1-dimethyl-n-butylcarbonyl, 1,2-dimethyl-n-butylcarbonyl, 1,3-dimethyl-n-butylcarbonyl, 2,2-dimethyl-n-butylcarbonyl, 2,3-dimethyl-n-butylcarbonyl, 3,3-dimethyl-n-butylcarbonyl, 1-ethyl-n-butylcarbonyl, 2-ethyl-n-butylcarbonyl, 1,1,2-trimethyl-n-propylcarbonyl, 1,2,2-trimethyl-n-propylcarbonyl, 1-ethyl-1-methyl-n-propylcarbonyl, 1-ethyl-2-methyl-n-propylcarbonyl, c-hexylcarbonyl, 1-methyl-c-pentylcarbonyl, 2-methyl-c-pentylcarbonyl, 3-methyl-c-pentylcarbonyl, 1-ethyl-c-butylcarbonyl, 2-ethyl-c-butylcarbonyl, 3-ethyl-c-butylcarbonyl, 1,2-dimethyl-c-butylcarbonyl, 1,3-dimethyl-c-butylcarbonyl, 2,2-dimethyl-c-butylcarbonyl, 2,3-dimethyl-c-butylcarbonyl, 2,4-dimethyl-c-butylcarbonyl, 3,3-dimethyl-c-butylcarbonyl, 1-n-propyl-c-propylcarbonyl, 2-n-propyl-c-propylcarbonyl, 1-i-propyl-c-propylcarbonyl, 2-i-propyl-c-propylcarbonyl, 1,2,2-trimethyl-c-propylcarbonyl, 1,2,3-trimethyl-c-propylcarbonyl, 2,2,3-trimethyl-c-propylcarbonyl, 1-ethyl-2-methyl-c-propylcarbonyl, 2-ethyl-1-methyl-c-propylcarbonyl, 2-ethyl-2-methyl-c-propylcarbonyl, 2-ethyl-3-methyl-c-propylcarbonyl, 1I-methyl-1-ethyl-n-pentylcarbonyl, 1-heptylcarbonyl, 2-heptylcarbonyl, 1-ethyl-1,2-dimethyl-n-propylcarbonyl, 1-ethyl-2,2-dimethyl-n-propylcarbonyl, 1-octylcarbonyl, 3-octylcarbonyl, 4-methyl-3-n-heptylcarbonyl, 6-methyl-2-n-heptylcarbonyl, 2-propyl-1-n-heptylcarbonyl, 2,4,4-trimethyl-1-n-pentylcarbonyl, 1-nonylcarbonyl, 2-nonylcarbonyl, 2,6-dimethyl-4-n-heptylcarbonyl, 3-ethyl-2,2-dimethyl-3-n-pentylcarbonyl, 3,5,5-trimethyl-1-n-hexylcarbonyl, 1-decylcarbonyl, 2-decylcarbonyl, 4-decylcarbonyl, 3,7-dimethyl-1-n-octylcarbonyl, and 3,7-dimethyl-3-n-octylcarbonyl.

Examples of the C₁₋₁₀ thioalkyl group may include linear C₁₋₁₀ thioalkyl groups, branched C₁₋₁₀ thioalkyl groups, and C₃₋₁₀ cyclothioalkyl groups, and include methylthio, ethylthio, n-propylthio, i-propylthio, c-propylthio, n-butylthio, i-butylthio, s-butylthio, t-butylthio, c-butylthio, 1-methyl-c-propylthio, 2-methyl-c-propylthio, n-pentylthio, 1-methyl-n-butylthio, 2-methyl-n-butylthio, 3-methyl-n-butylthio, 1,1-dimethyl-n-propylthio, 1,2-dimethyl-n-propylthio, 2,2-dimethyl-n-propylthio, 1-ethyl-n-propylthio, c-pentylthio, 1-methyl-c-butylthio, 2-methyl-c-butylthio, 3-methyl-c-butylthio, 1,2-dimethyl-c-propylthio, 2,3-dimethyl-c-propylthio, 1-ethyl-c-propylthio, 2-ethyl-c-propylthio, n-hexylthio, 1-methyl-n-pentylthio, 2-methyl-n-pentylthio, 3-methyl-n-pentylthio, 4-methyl-n-pentylthio, 1,1-dimethyl-n-butylthio, 1,2-dimethyl-n-butylthio, 1,3-dimethyl-n-butylthio, 2,2-dimethyl-n-butylthio, 2,3-dimethyl-n-butylthio, 3,3-dimethyl-n-butylthio, 1-ethyl-n-butylthio, 2-ethyl-n-butylthio, 1,1,2-trimethyl-n-propylthio, 1,2,2-trimethyl-n-propylthio, 1-ethyl-1-methyl-n-propylthio, 1-ethyl-2-methyl-n-propylthio, c-hexylthio, 1-methyl-c-pentylthio, 2-methyl-c-pentylthio, 3-methyl-c-pentylthio, 1-ethyl-c-butylthio, 2-ethyl-c-butylthio, 3-ethyl-c-butylthio, 1,2-dimethyl-c-butylthio, 1,3-dimethyl-c-butylthio, 2,2-dimethyl-c-butylthio, 2,3-dimethyl-c-butylthio, 2,4-dimethyl-c-butylthio, 3,3-dimethyl-c-butylthio, 1-n-propyl-c-propylthio, 2-n-propyl-c-propylthio, 1-i-propyl-c-propylthio, 2-i-propyl-c-propylthio, 1,2,2-trimethyl-c-propylthio, 1,2,3-trimethyl-c-propylthio, 2,2,3-trimethyl-c-propylthio, 1-ethyl-2-methyl-c-propylthio, 2-ethyl-1-methyl-c-propylthio, 2-ethyl-2-methyl-c-propylthio, 2-ethyl-3-methyl-c-propylthio, 1-methyl-1-ethyl-n-pentylthio, 1-heptylthio, 2-heptylthio, 1-ethyl-1,2-dimethyl-n-propylthio, 1-ethyl-2,2-dimethyl-n-propylthio, 1-octylthio, 3-octylthio, 4-methyl-3-n-heptylthio, 6-methyl-2-n-heptylthio, 2-propyl-1-n-heptylthio, 2,4,4-trimethyl-1-n-pentylthio, 1-nonylthio, 2-nonylthio, 2,6-dimethyl-4-n-heptylthio, 3-ethyl-2,2-dimethyl-3-n-pentylthio, 3,5,5-trimethyl-1-n-hexylthio, 1-decylthio, 2-decylthio, 4-decylthio, 3,7-dimethyl-1-n-octylthio, and 3,7-dimethyl-3-n-octylthio.
Examples of the C₁₋₃ alkylsulfonyl group may include linear C₁₋₃ alkylsulfonyl groups, branched C₁₋₃ alkylsulfonyl groups, and C₃ cycloalkylsulfonyl groups, and include methylsulfonyl, ethylsulfonyl, n-propylsulfonyl, i-propylsulfonyl, and c-propylsulfonyl.

Examples of the C₁₋₁₀ alkylsulfonyl group may include linear C₁₋₁₀ alkylsulfonyl groups, branched C₁₋₁₀ alkylsulfonyl groups, and C₃₋₁₀ cycloalkylsulfonyl groups, and include, besides the above C₁₋₃ alkylsulfonyl groups, n-butylsulfonyl, i-butylsulfonyl, s-butylsulfonyl, t-butylsulfonyl, c-butylsulfonyl, 1-methyl-c-propylsulfonyl, 2-methyl-c-propylsulfonyl, n-pentylsulfonyl, 1-methyl-n-butylsulfonyl, 2-methyl-n-butylsulfonyl, 3-methyl-n-butylsulfonyl, 1,1-dimethyl-n-propylsulfonyl, 1,2-dimethyl-n-propylsulfonyl, 2,2-dimethyl-n-propylsulfonyl, 1-ethyl-n-propylsulfonyl, c-pentylsulfonyl, 1-methyl-c-butylsulfonyl, 2-methyl-c-butylsulfonyl, 3-methyl-c-butylsulfonyl, 1,2-dimethyl-c-propylsulfonyl, 2,3-dimethyl-c-propylsulfonyl, 1-ethyl-c-propylsulfonyl, 2-ethyl-c-propylsulfonyl, n-hexylsulfonyl, 1-methyl-n-pentylsulfonyl, 2-methyl-n-pentylsulfonyl, 3-methyl-n-pentylsulfonyl, 4-methyl-n-pentylsulfonyl, 1,1-dimethyl-n-butylsulfonyl, 1,2-dimethyl-n-butylsulfonyl, 1,3-dimethyl-n-butylsulfonyl, 2,2-dimethyl-n-butylsulfonyl, 2,3-dimethyl-n-butylsulfonyl, 3,3-dimethyl-n-butylsulfonyl, 1-ethyl-n-butylsulfonyl, 2-ethyl-n-butylsulfonyl, 1,1,2-trimethyl-n-propylsulfonyl, 1,2,2-trimethyl-n-propylsulfonyl, 1-ethyl-1-methyl-n-propylsulfonyl, 1-ethyl-2-methyl-n-propylsulfonyl, c-hexylsulfonyl, 1-methyl-c-pentylsulfonyl, 2-methyl-c-pentylsulfonyl, 3-methyl-c-pentylsulfonyl, 1-ethyl-c-butylsulfonyl, 2-ethyl-c-butylsulfonyl, 3-ethyl-c-butylsulfonyl, 1,2-dimethyl-c-butylsulfonyl, 1,3-dimethyl-c-butylsulfonyl, 2,2-dimethyl-c-butylsulfonyl, 2,3-dimethyl-c-butylsulfonyl, 2,4-dimethyl-c-butylsulfonyl, 3,3-dimethyl-c-butylsulfonyl, 1-n-propyl-c-propylsulfonyl, 2-n-propyl-c-propylsulfonyl, 1-i-propyl-c-propylsulfonyl, 2-i-propyl-c-propylsulfonyl, 1,2,2-trimethyl-c-propylsulfonyl, 1,2,3-trimethyl-c-propylsulfonyl, 2,2,3-trimethyl-c-propylsulfonyl, 1-ethyl-2-methyl-c-propylsulfonyl, 2-ethyl-1-methyl-c-propylsulfonyl, 2-ethyl-2-methyl-c-propylsulfonyl, 2-ethyl-3-methyl-c-propylsulfonyl, 1-methyl-1-ethyl-n-pentylsulfonyl, 1-heptylsulfonyl, 2-heptylsulfonyl, 1-ethyl-1,2-dimethyl-n-propylsulfonyl, 1-ethyl-2,2-dimethyl-n-propylsulfonyl, 1-octylsulfonyl, 3-octylsulfonyl, 4-methyl-3-n-heptylsulfonyl, 6-methyl-2-n-heptylsulfonyl, 2-propyl-1-n-heplylsulfonyl, 2,4,4-trimethyl-1-pentylsulfonyl, 1-nonylsulfonyl, 2-nonylsulfonyl, 2,6-dimethyl-4-n-heptylsulfonyl, 3-ethyl-2,2-dimethyl-3-n-pentylsulfonyl, 3,5,5-trimethyl-1-n-hexylsulfonyl, 1-decylsulfonyl, 2-decylsulfonyl, 4-decylsulfonyl, 3,7-dimethyl-1-n-octylsulfonyl, and 3,7-dimethyl-3-n-octylsulfonyl.

Examples of the C₁₋₁₀ alkylsulfonylamino group may include linear C₁₋₁₀ alkylsulfonylamino groups, branched C₁₋₁₀ alkylsulfonylamino groups, and C₃₋₁₀ cycloalkylsulfonylamino groups, and include methylsulfonylamino, ethylsulfonylamino, n-propylsulfonylamino, i-propylsulfonylamino, c-propylsulfonylamino, n-butylsulfonylamino, i-butylsulfonylamino, s-butylsulfonylamino, t-butylsulfonylamino, c-butylsulfonylamino, 1-methyl-c-propylsulfonylamino, 2-methyl-c-propylsulfonylamino, n-pentylsulfonylamino, 1-methyl-n-butylsulfonylamino, 2-methyl-n-butylsulfonylamino, 3-methyl-n-butylsulfonylamino, 1,1-dimethyl-n-propylsulfonylamino, 1,2-dimethyl-n-propylsulfonylamino, 2,2-dimethyl-n-propylsulfonylamino, 1-ethyl-n-propylsulfonylamino, c-pentylsulfonylamino, 1-methyl-c-butylsulfonylamino, 2-methyl-c-butylsulfonylamino, 3-methyl-c-butylsulfonylamino, 1,2-dimethyl-c-propylsulfonylamino, 2,3-dimethyl-c-propylsulfonylamino, 1-ethyl-c-propylsulfonylamino, 2-ethyl-c-propylsulfonylamino, n-hexylsulfonylamino, 1-methyl-n-pentylsulfonylamino, 2-methyl-n-pentylsulfonylamino, 3-methyl-n-pentylsulfonylamino, 4-methyl-n-pentylsulfonylamino, 1,1-dimethyl-n-butylsulfonylamino, 1,2-dimethyl-n-butylsulfonylamino, 1,3-dimethyl-n-butylsulfonylamino, 2,2-dimethyl-n-butylsulfonylamino, 2,3-dimethyl-n-butylsulfonylamino, 3,3-dimethyl-n-butylsulfonylamino, 1-ethyl-n-butylsulfonylamino, 2-ethyl-n-butylsulfonylamino, 1,1,2-trimethyl-n-propylsulfonylamino, 1,2,2-trimethyl-n-propylsulfonylamino, 1-ethyl-1-methyl-n-propylsulfonylamino, 1-ethyl-2-methyl-n-propylsulfonylamino, c-hexylsulfonylamino, 1-methyl-c-pentylsulfonylamino, 2-methyl-c-pentylsulfonylamino, 3-methyl-c-pentylsulfonylamino, 1-ethyl-c-butylsulfonylamino, 2-ethyl-c-butylsulfonylamino, 3-ethyl-c-butylsulfonylamino, 1,2-dimethyl-c-butylsulfonylamino, 1,3-dimethyl-c-butylsulfonylamino, 2,2-dimethyl-c-butylsulfonylamino, 2,3-dimethyl-c-butylsulfonylamino, 2,4-dimethyl-c-butylsulfonylamino, 3,3-dimethyl-c-butylsulfonylamino, 1-n-propyl-c-propylsulfonylamino, 2-n-propyl-c-propylsulfonylamino, 1-i-propyl-c-propylsulfonylamino, 2-i-propyl-c-propylsulfonylamino, 1,2,2-trimethyl-c-propylsulfonylamino, 1,2,3-trimethyl-c-propylsulfonylamino, 2,2,3-trimethyl-c-propylsulfonylamino, 1-ethyl-2-methyl-c-propylsulfonylamino, 2-ethyl-1-methyl-c-propylsulfonylamino, 2-ethyl-2-methyl-c-propylsulfonylamino, 2-ethyl-3-methyl-c-propylsulfonylamino, 1-methyl-1-ethyl-n-pentylsulfonylamino, 1-heptylsulfonylamino, 2-heptylsulfonylamino, 1-ethyl-1,2-dimethyl-n-propylsulfonylamino, 1-ethyl-2,2-dimethyl-n-propylsulfonylamino, 1-octylsulfonylamino, 3-octylsulfonylamino, 4-methyl-3-n-heptylsulfonylamino, 6-methyl-2-n-heptylsulfonylamino, 2-propyl-I-n-heptylsulfonylamino, 2,4,4-trimethyl-1-n-pentylsulfonylamino, 1-nonylsulfonylamino, 2-nonylsulfonylamino, 2,6-dimethyl-4-n-heptylsulfonylamino, 3-ethyl-2,2-dimethyl-3-n-pentylsulfonylamino, 3,5,5-trimethyl-1-n-hexylsulfonylamino, 1-decylsulfonylamino, 2-decylsulfonylamino, 4-decylsulfonylamino, 3,7-dimethyl-1-n-octylsulfonylamino, 3,7-dimethyl-3-n-octylsulfonylamino, c-heptylsulfonylamino, c-octylsulfonylamino, 1-methyl-c-hexylsulfonylamino, 2-methyl-c-hexylsulfonylamino, 3-methyl-c-hexylsulfonylamino, 1,2-dimethyl-c-hexylsulfonylamino, 1-ethyl-c-hexylsulfonylamino, 1-methyl-c-pentylsulfonylamino, 2-methyl-c-pentylsulfonylamino, and 3-methyl-c-pentylsulfonylamino.

Examples of the C₁₋₃ alkoxy group may include linear C₁₋₃ alkoxy groups, branched C₁₋₃ alkoxy groups, and C₃ cycloalkoxy groups, and include methoxy, ethoxy, n-propoxy, i-propoxy, and c-propoxy.

Examples of the C₁₋₆ alkoxy group may include linear C₁₋₆ alkoxy groups, branched C₁₋₆ alkoxy groups, and C₃₋₆ cycloalkoxy groups, and include, besides the above C₁₋₃ alkoxy groups, n-butoxy, i-butoxy, s-butoxy, t-butoxy, c-butoxy, 1-methyl-c-propoxy, 2-methyl-c-propoxy, n-pentyloxy, 1-methyl-n-butoxy, 2-methyl-n-butoxy, 3-methyl-n-butoxy, 1,1-dimethyl-n-propoxy, 1,2-dimethyl-n-propoxy, 2,2-dimethyl-n-propoxy, 1-ethyl-n-propoxy, c-pentyloxy, 1-methyl-c-butoxy, 2-methyl-c-butoxy, 3-methyl-c-butoxy, 1,2-dimethyl-c-propoxy, 2,3-dimethyl-c-propoxy, 1-ethyl-c-propoxy, 2-ethyl-c-propoxy, n-hexyloxy, 1-methyl-n-pentyloxy, 2-methyl-n-pentyloxy, 3-methyl-n-pentyloxy, 4-methyl-n-pentyloxy, 1,1-dimethyl-n-butoxy, 1,2-dimethyl-n-butoxy, 1,3-dimethyl-n-butoxy, 2,2-dimethyl-n-butoxy, 2,3-dimethyl-n-butoxy, 3,3-dimethyl-n-butoxy, 1-ethyl-n-butoxy, 2-ethyl-n-butoxy, 1,1,2-trimethyl-n-propoxy, 1,2,2-trimethyl-n-propoxy, 1-ethyl-1-methyl-n-propoxy, 1-ethyl-2-methyl-n-propoxy, c-hexyloxy, 1-methyl-c-pentyloxy, 2-methyl-c-pentyloxy, 3-methyl-c-pentyloxy, 1-ethyl-c-butoxy, 2-ethyl-c-butoxy, 3-ethyl-c-butoxy, 1,2-dimethyl-c-butoxy, 1,3-dimethyl-c-butoxy, 2,2-dimethyl-c-butoxy, 2,3-dimethyl-c-butoxy, 2,4-dimethyl-c-butoxy, 3,3-dimethyl-c-butoxy, 1-n-propyl-c-propoxy, 2-n-propyl-c-propoxy, 1-i-propyl-c-propoxy, 2-i-propyl-c-propoxy, 1,2,2-trimethyl-c-propoxy, 1,2,3-trimethyl-c-propoxy, 2,2,3-trimethyl-c-propoxy, 1-ethyl-2-methyl-c-propoxy, 2-ethyl-1-methyl-c-propoxy, 2-ethyl-2-methyl-c-propoxy, and 2-ethyl-3-methyl-c-propoxy.

Examples of the C₁₋₁₀ alkoxy group may include linear C₁₋₁₀ alkoxy groups, branched C₁₋₁₀ alkoxy groups, and C₃₋₁₀ cycloalkoxy groups, and include, besides the above C₁₋₃ alkoxy groups, n-butoxy, i-butoxy, s-butoxy, t-butoxy, c-butoxy, 1-methyl-c-propoxy, 2-methyl-c-propoxy, n-pentyloxy, 1-methyl-n-butoxy, 2-methyl-n-butoxy, 3-methyl-n-butoxy, 1,1-dimemyl-n-propoxy, 1,2-dimethyl-n-propoxy, 2,2-dimethyl-n-propoxy, 1-ethyl-n-propoxy, c-pentyloxy, 1-methyl-c-butoxy, 2-methyl-c-butoxy, 3-methyl-c-butoxy, 1,2-dimethyl-c-propoxy, 2,3-dimethyl-c-propoxy, 1-ethyl-c-propoxy, 2-ethyl-c-propoxy, n-hexyloxy, 1-methyl-n-pentyloxy, 2-methyl-n-pentyloxy, 3-methyl-n-pentyloxy, 4-methyl-n-pentyloxy, 1,1-dimethyl-n-butoxy, 1,2-dimethyl-n-butoxy, 1,3-dimethyl-n-butoxy, 2,2-dimethyl-n-butoxy, 2,3-dimethyl-n-butoxy, 3,3-dimethyl-n-butoxy, 1-ethyl-n-butoxy, 2-ethyl-n-butoxy, 1,1,2-trimethyl-n-propoxy, 1,2,2-trimethyl-n-propoxy, 1-ethyl-1-methyl-n-propoxy, 1-ethyl-2-methyl-n-propoxy, c-hexyloxy, 1-methyl-c-pentyloxy, 2-methyl-c-pentyloxy, 3-methyl-c-pentyloxy, 1-ethyl-c-butoxy, 2-ethyl-c-butoxy, 3-ethyl-c-butoxy, 1,2-dimethyl-c-butoxy, 1,3-dimethyl-c-butoxy, 2,2-dimethyl-c-butoxy, 2,3-dimethyl-c-butoxy, 2,4-dimethyl-c-butoxy, 3,3-dimethyl-c-butoxy, 1-n-propyl-c-propoxy, 2-n-propyl-c-propoxy, 1-i-propyl-c-propoxy, 2-i-propyl-c-propoxy, 1,2,2-trimethyl-c-propoxy, 1,2,3-trimethyl-c-propoxy, 2,2,3-trimethyl-c-propoxy, 1-ethyl-2-methyl-c-propoxy, 2-ethyl-1-methyl-c-propoxy, 2-ethyl-2-methyl-c-propoxy, 2-ethyl-3-methyl-c-propoxy, 1-methyl-1-ethyl-n-pentyloxy, 1-heptyloxy, 2-heptyloxy, 1-ethyl-1,2-dimethyl-n-propyloxy, 1-ethyl-2,2-dimethyl-n-propyloxy, 1-octyloxy, 3-octyloxy, 4-methyl-3-n-heptyloxy, 6-methyl-2-n-heptyloxy, 2-propyl-1-n-heptyloxy, 2,4,4-trimethyl-1-n-pentyloxy, 1-nonyloxy, 2-nonyloxy, 2,6-dimethyl-4-n-heptyloxy, 3-ethyl-2,2-dimethyl-3-n-pentyloxy, 3,5,5-trimethyl-1-n-hexyloxy, 1-decyloxy, 2-decyloxy, 4-decyloxy, 3,7-dimethyl-1-n-octyloxy, and 3,7-dimethyl-3-n-octyloxy.

The C₁₋₃ halogen-substituted alkoxy group means a C₁₋₃ alkoxy group in which a hydrogen atom on a carbon atom is substituted with a halogen atom, and the alkoxy group may be linear alkoxy groups, branched alkoxy groups, and C₃ cycloalkyl groups. Specific examples thereof include trifluoromethoxy, difluoromethoxy, trichloromethoxy, dichloromethoxy, difluorochloromethoxy, pentafluoroethoxy, n-heptafluoropropoxy, i-heptafluoropropoxy, and c-pentafluoropropoxy.

Examples of the C₁₋₁₀ alkoxycarbonyl group may include linear C₁₋₁₀ alkoxycarbonyl groups, branched C₁₋₁₀ alkoxycarbonyl groups, and C₃₋₁₀ cycloalkoxycarbonyl groups, and include methoxycarbonyl, ethoxycarbonyl, n-propoxycarbonyl, i-propoxycarbonyl, c-propoxycarbonyl, n-butoxycarbonyl, i-butoxycarbonyl, s-butoxycarbonyl, t-butoxycarbonyl, c-butoxycarbonyl, 1-methyl-c-propoxycarbonyl, 2-methyl-c-propoxycarbonyl, n-pentyloxycarbonyl, 1-methyl-n-butoxycarbonyl, 2-methyl-n-butoxycarbonyl, 3-methyl-n-butoxycarbonyl, 1,1-dimethyl-n-propoxycarbonyl, 1,2-dimethyl-n-propoxycarbonyl, 2,2-dimethyl-n-propoxycarbonyl, 1-ethyl-n-propoxycarbonyl, c-pentyloxycarbonyl, 1-methyl-c-butoxycarbonyl, 2-methyl-c-butoxycarbonyl, 3-methyl-c-butoxycarbonyl, 1,2-dimethyl-c-propoxycarbonyl, 2,3-dimethyl-c-propoxycarbonyl, 1-ethyl-c-propoxycarbonyl, 2-ethyl-c-propoxycarbonyl, n-hexyloxycarbonyl, 1-methyl-n-pentyloxycarbonyl, 2-methyl-n-pentyloxycarbonyl, 3-methyl-n-pentyloxycarbonyl, 4-methyl-n-pentyloxycarbonyl, 1,1-dimethyl-n-butoxycarbonyl, 1,2-dimethyl-n-butoxycarbonyl, 1,3-dimethyl-n-butoxycarbonyl, 2,2-dimethyl-n-butoxycarbonyl, 2,3-dimethyl-n-butoxycarbonyl, 3,3-dimethyl-n-butoxycarbonyl, 1-ethyl-n-butoxycarbonyl, 2-ethyl-n-butoxycarbonyl, 1,1,2-trimethyl-n-propoxycarbonyl, 1,2,2-trimethyl-n-propoxycarbonyl, 1-ethyl-1-methyl-n-propoxycarbonyl, 1-ethyl-2-methyl-n-propoxycarbonyl, c-hexyloxycarbonyl, 1-methyl-c-pentyloxycarbonyl, 2-methyl-c-pentyloxycarbonyl, 3-methyl-c-pentyloxycarbonyl, 1-ethyl-c-butoxycarbonyl, 2-ethyl-c-butoxycarbonyl, 3-ethyl-c-butoxycarbonyl, 1,2-dimethyl-c-butoxycarbonyl, 1,3-dimethyl-c-butoxycarbonyl, 2,2-dimethyl-c-butoxycarbonyl, 2,3-dimethyl-c-butoxycarbonyl, 2,4-dimethyl-c-butoxycarbonyl, 3,3-dimethyl-c-butoxycarbonyl, 1-n-propyl-c-propoxycarbonyl, 2-n-propyl-c-propoxycarbonyl, 1-i-propyl-c-propoxycarbonyl, 2-i -propyl-c-propoxycarbonyl, 1,2,2-trimethyl-c-propoxycarbonyl, 1,2,3-trimethyl-c-propoxycarbonyl, 2,2,3-trimethyl-c-propoxycarbonyl, 1-ethyl-2-methyl-c-propoxycarbonyl, 2-ethyl-1-methyl-c-propoxycarbonyl, 2-ethyl-2-methyl-c-propoxycarbonyl, 2-ethyl-3-methyl-c-propoxycarbonyl, 1-methyl-1-ethyl-n-pentyloxycarbonyl, 1-heptyloxycarbonyl, 2-heptyloxycarbonyl, 1-ethyl-1,2-dimethyl-n-propyloxycarbonyl, 1-ethyl-2,2-dimethyl-n-propyloxycarbonyl, 1-octyloxycarbonyl, 3-octyloxycarbonyl, 4-methyl-3-n-heptyloxycarbonyl, 6-methyl-2-n-heptyloxycarbonyl, 2-propyl-1-n-heptyloxycarbonyl, 2,4,4-trimethyl-1-n-pentyloxycarbonyl, 1-nonyloxycarbonyl, 2-nonyloxycarbonyl, 2,6-dimethyl-4-n-heptyloxycarbonyl, 3-ethyl-2,2-dimethyl-3-n-pentyloxycarbonyl, 3,5,5-trimethyl-1-n-hexyloxycarbonyl, 1-decyloxycarbonyl, 2-decyloxycarbonyl, 4-decyloxycarbonyl, 3,7-dimethyl-1-n-octyloxycarbonyl, and 3,7-dimethyl-3-n-octyloxycarbonyl.

Examples of the C₁₋₁₀ alkylcarbonyloxy group may include linear C₁₋₁₀ alkylcarbonyloxy groups, branched C₁₋₁₀ alkylcarbonyloxy groups, and C₃₋₁₀ cycloalkylcarbonyloxy groups, and include methylcarbonyloxy, ethylcarbonyloxy, n-propylcarbonyloxy, i-propylcarbonyloxy, c-propylcarbonyloxy, n-butylcarbonyloxy, i-butylcarbonyloxy, s-butylcarbonyloxy, t-butylcarbonyloxy, c-butylcarbonyloxy, 1-methyl-c-propylcarbonyloxy, 2-methyl-c-propylcarbonyloxy, n-pentylcarbonyloxy, 1-methyl-n-butylcarbonyloxy, 2-methyl-n-butylcarbonyloxy, 3-methyl-n-butylcarbonyloxy, 1,l-dimethyl-n-propylcarbonyloxy, 1,2-dimethyl-n-propylcarbonyloxy, 2,2-dimethyl-n-propylcarbonyloxy, 1-ethyl-n-propylcarbonyloxy, c-pentylcarbonyloxy, 1-methyl-c-butylcarbonyloxy, 2-methyl-c-butylcarbonyloxy, 3-methyl-c-butylcarbonyloxy, 1,2-dimethyl-c-propylcarbonyloxy, 2,3-dimethyl-c-propylcarbonyloxy, 1-ethyl-c-propylcarbonyloxy, 2-ethyl-c-propylcarbonyloxy, n-hexylcarbonyloxy, 1-methyl-n-pentylcarbonyloxy, 2-methyl-n-pentylearbonyloxy, 3-methyl-n-pentylcarbonyloxy, 4-methyl-n-pentylcarbonyloxy, 1,1-dimethyl-n-butylcarbonyloxy, 1,2-dimethyl-n-butylcarbonyloxy, 1,3-dimethyl-n-butylcarbonyloxy, 2,2-dimethyl-n-butylcarbonyloxy, 2,3-dimethyl-n-butylcarbonyloxy, 3,3-dimethyl-n-butylcarbonyloxy, 1-ethyl-n-butylcarbonyloxy, 2-ethyl-n-butylcarbonyloxy, 1,1,2-trimethyl-n-propylcarbonyloxy, 1,2,2-trimethyl-n-propylcarbonyloxy, 1-ethyl-1-methyl-n-propylcarbonyloxy, 1-ethyl-2-methyl-n-propylcarbonyloxy, c-hexylcarbonyloxy, 1-methyl-c-pentylcarbonyloxy, 2-methyl-c-pentylcarbonyloxy, 3-methyl-c-pentylcarbonyloxy, 1-ethyl-c-butylcarbonyloxy, 2-ethyl-c-butylcarbonyloxy, 3-ethyl-c-butylcarbonyloxy, 1,2-dimethyl-c-butylcarbonyloxy, 1,3-dimethyl-c-butylcarbonyloxy, 2,2-dimethyl-c-butylcarbonyloxy, 2,3-dimethyl-c-butylcarbonyloxy, 2,4-dimethyl-c-butylcarbonyloxy, 3,3-dimethyl-c-butylcarbonyloxy, 1-n-propyl-c-propylcarbonyloxy, 2-n-propyl-c-propylcarbonyloxy, 1-i-propyl-c-propylcarbonyloxy, 2-i-propyl-c-propylcarbonyloxy, 1,2,2-trimethyl-c-propylcarbonyloxy, 1,2,3-trimethyl-c-propylcarbonyloxy, 2,2,3-trimethyl-c-propylcarbonyloxy, 1-ethyl-2-methyl-c-propylcarbonyloxy, 2-ethyl-1-methyl-c-propylcarbonyloxy, 2-ethyl-2-methyl-c-propylcarbonyloxy, 2-ethyl-3-methyl-c-propylcarbonyloxy, 1-methyl-1-ethyl-n-pentylcarbonyloxy, 1-heptylcarbonyloxy, 2-heptylcarbonyloxy, 1-ethyl-1,2-dimethyl-n-propylcarbonyloxy, 1-ethyl-2,2-dimethyl-n-propylcarbonyloxy, 1-octylcarbonyloxy, 3-octylcarbonyloxy, 4-methyl-3-n-heptylcarbonyloxy, 6-methyl-2-n-heptylcarbonyloxy, 2-propyl-1-n-heptylcarbonyloxy, 2,4,4-trimethyl-1-n-pentylcarbonyloxy, 1-nonylcarbonyloxy, 2-nonylcarbonyloxy, 2,6-dimethyl-4-n-heptylcarbonyloxy, 3-ethyl-2,2-dimethyl-3-n-pentylcarbonyloxy, 3,5,5-trimethyl-1-n-hexylcarbonyloxy, 1-decylcarbonyloxy, 2-decylcarbonyloxy, 4-decylcarbonyloxy, 3,7-dimethyl-1-n-octylcarbonyloxy, and 3,7-dimethyl-3-n-octylcarbonyloxy.

Examples of the C₁₋₁₀ alkylcarbonylamino group may include linear C₁₋₁₀ alkylcarbonylamino groups, branched C₁₋₁₀ alkylcarbonylamino groups, and C₃₋₁₀ cycloalkylcarbonylamino groups, and include methylcarbonylamino, ethylcarbonylamino, n-propylcarbonylamino, i-propylcarbonylamino, c-propylcarbonylamino, n-butylcarbonylamino, i-butylcarbonylamino, s-butylcarbonylamino, t-butylcarbonylamino, c-butylcarbonylamino, 1-methyl-c-propylcarbonylamino, 2-methyl-c-propylcarbonylamino, n-pentylcarbonylamino, 1-methyl-n-butylcarbonylamino, 2-methyl-n-butylcarbonylamino, 3-methyl-n-butylcarbonylamino, 1,1-dimethyl-n-propylcarbonylamino, 1,2-dimethyl-n-propylcarbonylamino, 2,2-dimethyl-n-propylcarbonylamino, 1-ethyl-n-propylcarbonylamino, c-pentylcarbonylamino, 1-methyl-c-butylcarbonylamino, 2-methyl-c-butylcarbonylamino, 3-methyl-c-butylcarbonylamino, 1.2-dimethyl-c-propylcarbonylamino, 2,3-dimethyl-c-propylcarbonylamino, 1-ethyl-c-propylcarbonylamino, 2-ethyl-c-propylcarbonylamino, n-hexylcarbonylamino, 1-methyl-n-pentylcarbonylamino, 2-methyl-n-pentylcarbonylamino, 3-methyl-n-pentylcarbonylamino, 4-methyl-n-pentylcarbonylamino, 1,1-dimethyl-n-butylcarbonylamino, 1,2-dimethyl-n-butylcarbonylamino, 1,3-dimethyl-n-butylcarbonylamino, 2,2-dimethyl-n-butylcarbonylamino, 2,3-dimethyl-n-butylcarbonylamino, 3,3-dimethyl-n-butylcarbonylamino, 1-ethyl-n-butylcarbonylamino, 2-ethyl-n-butylcarbonylamino, 1,1,2-trimethyl-n-propylcarbonylamino, 1,2,2-trimethyl-n-propylcarbonylamino, 1-ethyl-1-methyl-n-propylcarbonylamino, 1-ethyl-2-methyl-n-propylcarbonylamino, c-hexylcarbonylamino, 1-methyl-c-pentylcarbonylamino, 2-methyl-c-pentylcarbonylamino, 3-methyl-c-pentylcarbonylamino, 1-ethyl-c-butylcarbonylamino, 2-ethyl-c-butylcarbonylamino, 3-ethyl-c-butylcarbonylamino, 1,2-dimethyl-c-butylcarbonylamino, 1,3-dimethyl-c-butylcarbonylamino, 2,2-dimethyl-c-butylcarbonylamino, 2,3-dimethyl-c-butylcarbonylamino, 2,4-dimethyl-c-butylcarbonylamino, 3,3-dimethyl-c-butylcarbonylamino, 1-n-propyl-c-propylcarbonylamino, 2-n-propyl-c-propylcarbonylamino, 1-i-propyl-c-propylcarbonylamino, 2-i-propyl-c-propylcarbonylamino, 1,2,2-trimethyl-c-propylcarbonylamino, 1,2,3-trimethyl-c-propylcarbonylamino, 2,2,3-trimethyl-c-propylcarbonylamino, 1-ethyl-2-methyl-c-propylcarbonylamino, 2-ethyl-1-methyl-c-propylcarbonylamino, 2-ethyl-2-methyl-c-propylcarbonylamino, 2-ethyl-3-methyl-c-propylcarbonylamino, 1-methyl-1-ethyl-n-pentylcarbonylamino, 1-heptylcarbonylamino, 2-heptylcarbonylamino, 1-ethyl-1,2-dimethyl-n-propylcarbonylamino, 1-ethyl-2,2-dimethyl-n-propylcarbonylamino, 1-octylcarbonylamino, 3-octylcarbonylamino, 4-methyl-3-n-heptylcarbonylamino, 6-methyl-2-n-heptylcarbonylamino, 2-propyl-1-n-heptylcarbonylamino, 2,4,4-trimethyl-1-n-pentylcarbonylamino, 1-nonylcarbonylamino, 2-nonylcarbonylamino, 2,6-dimethyl-4-n-heptylcarbonylamino, 3-ethyl-2,2-dimethyl-3-n-pentylcarbonylamino, 3,5,5-trimethyl-1-n-hexylcarbonylamino, 1-decylcarbonylamino, 2-decylcarbonylamino, 4-decylcarbonylamino, 3,7-dimethyl-1-n-octylcarbonylamino, and 3,7-dimethyl-3-n-octylcarbonylamino.

Examples of the mono C₁₋₁₀ alkylamino group may include linear C₁₋₁₀ alkylamino groups, branched C₁₋₁₀ alkylamino groups, and C₃₋₁₀ cycloalkylamino groups, and include methylamino, ethylamino, n-propylamino, i-propylamino, c-propylamino, n-butylamino, i-butylamino, s-butylamino, t-butylamino, c-butylamino, 1-methyl-c-propylamino, 2-methyl-c-propylamino, n-pentylamino, 1-methyl-n-butylamino, 2-methyl-n-butylamino, 3-methyl-n-butylamino, 1,1-dimethyl-n-propylamino, 1,2-dimethyl-n-propylamino, 2,2-dimethyl-n-propylamino, 1-ethyl-n-propylamino, c-pentylamino, 1-methyl-c-butylamino, 2-methyl-c-butylamino, 3-methyl-c-butylamino, 1,2-dimethyl-c-propylamino, 2,3-dimethyl-c-propylamino, 1-ethyl-c-propylamino, 2-ethyl-c-propylamino, n-hexylamino, 1-methyl-n-pentylamino, 2-methyl-n-pentylamino, 3-methyl-n-pentylamino, 4-methyl-n-pentylamino, 1,1-dimethyl-n-butylamino, 1,2-dimethyl-n-butylamino, 1,3-dimethyl-n-butylamino, 2,2-dimethyl-n-butylamino, 2-3-dimethyl-n-butylamino, 3,3-dimethyl-n-butylamino, 1-ethyl-n-butylamino, 2-ethyl-n-butylamino, 1,1,2-trimethyl-n-propylamino, 1,2,2-trimethyl-n-propylamino, 1-ethyl-1-methyl-n-propylamino, 1-ethyl-2-methyl-n-propylamino, c-hexylamino, 1-methyl-c-pentylamino, 2-methyl-c-pentylamino, 3-methyl-c-pentylamino, 1-ethyl-c-butylamino, 2-ethyl-c-butylamino, 3-ethyl-c-butylamino, 1,2-dimethyl-c-butylamino, 1,3-dimethyl-c-butylamino, 2,2-dimethyl-c-butylamino, 2,3-dimethyl-c-butylamino, 2,4-dimethyl-c-butylamino, 3,3-dimethyl-c-butylamino, 1-n-propyl-c-propylamino, 2-n-propyl-c-propylamino, 1-i-propyl-c-propylamino, 2-i-propyl-c-propylamino, 1,2,2-trimethyl-c-propylamino, 1,2,3-trimethyl-c-propylamino, 2,2,3-trimethyl-c-propylamino, 1-ethyl-2-methyl-c-propylamino, 2-ethyl-1-methyl-c-propylamino, 2-ethyl-2-methyl-c-propylamino, 2-ethyl-3-methyl-c-propylamino, 1-methyl-1-ethyl-n-pentylamino, 1-heptylamino, 2-heptylamino, 1-ethyl-1,2-dimethyl-n-propylamino, 1-ethyl-2,2-dimethyl-n-propylamino, 1-octylamino, 3-actylamino, 4-methyl-3-n-heptylamino, 6-methyl-2-n-heptylamino, 2-propyl-1-n-heptylamino, 2,4,4-trimethyl-1-n-pentylamino, 1-nonylamino, 2-nonylamino, 2,6-dimethyl-4-n-heptylamino, 3-ethyl-2,2-dimethyl-3-n-pentylamino, 3,5,5-trimethyl-1-n-hexylamino, 1-decylamino, 2-decylamino, 4-decylamino, 3,7-dimethyl-1-n-octylamino, and 3,7-dimethyl-3-n-octylamino.

Examples of the di C₁₋₁₀ alkylamino group may include symmetric and asymmetric dialkylamino groups. Examples of the symmetric di C₁₋₁₀ alkylamino group may include linear C₁₋₁₀ alkylamino groups, branched C₁₋₁₀ alkylamino groups, and C₃₋₁₀ cycloalkylamino groups, and include dimethylamino, diethylamino, di-n-propylamino, di-i-propylamino, di-c-propylamino, di-n-butylamino, di-i-butylamino, di-s-butylamino, di-t-butylamino, di-c-butylamino, di-(1-methyl-c-propyl)amino, di-(2-methyl-c-propyl)amino, di-n-pentylamino, di-(1-methyl-n-butyl)amino, di-(2-methyl-n-butyl)amino, di-(3-methyl-n-butyl)amino, di-(1,1-dimethyl-n-propyl)amino, di-(1,2-dimethyl-n-propyl)amino, di-(2,2-dimethyl-n-propyl)amino, di-(1-ethyl-n-propyl)amino, di-c-pentylamino, di-(1-methyl-c-butyl)amino, di-(2-methyl-c-butyl)amino, di-(3-methyl-c-butyl)amino, di-(1,2-dimethyl-c-propyl)amino, di-(2,3-dimethyl-c-propyl)amino, di-(1-ethyl-c-propyl)amino, di-(2-ethyl-c-propyl)amino, din-hexylamino, di-(1-methyl-n-pentyl)amino, di-(2-methyl-n-pentyl)amino, di-(3-methyl-n-pentyl)amino, di-(4-methyl-n-pentyl)amino, di-(1,1-dimethyl-n-butyl)amino, di-(1,2-dimethyl-n-butyl)amino, di-(1,3-dimethyl-n-butyl)amino, di-(2,2-dimethyl-n-butyl)amino, di-(2,3-dimethyl-n-butyl)amino, di-(3,3-dimethyl-n-butyl)amino, di-(1-ethyl-n-butyl)amino, di-(2-ethyl-n-butyl)amino, di-(1,1,2-trimethyl-n-propyl)amino, di-(1,2,2-trimethyl-n-propyl)amino, di-(1-ethyl-1-methyl-n-propyl)amino, di-(1-ethyl-2-methyl-n-propyl)amino, di-c-hexylamino, di-(1-methyl-c-pentyl)amino, di-(2-methyl-c-pentyl)amino, di-(3-methyl-c-pentyl)amino, di-(l-ethyl-c-butyl)amino, di-(2-ethyl-c-butyl)amino, di-(3-ethyl-c-butyl)amino, di-(1,2-dimethyl-c-butyl)amino, di-(1,3-dimethyl-c-butyl)amino, di-(2,2-dimethyl-c-butyl)amino, di-(2,3-dimethyl-c-butyl)amino, di-(2,4-dimethyl-c-butyl)amino, di-(3,3-dimethyl-c-butyl)amino, di-(1-n-propyl-c-propyl)amino, di-(2-n-propyl-c-propyl)amino, di-(1-i-propyl-c-propyl)amino, di-(2-i-propyl-c-propyl)amino, di-(1,2,2-trimethyl-c-propyl)amino; di-(1,2,3-trimethyl-c-propyl)amino, di-(2,2,3-trimethyl-c-propyl)amino, di-(1-ethyl-2-methyl-c-propyl)amino, di-(2-ethyl-1-methyl-c-propyl)amino, di-(2-ethyl-2-methyl-c-propyl)amino, di-(2-ethyl-3-methyl-c-propyl)amino, di-(1-methyl-1-ethyl-n-pentyl)amino, di-(1-heptyl)amino, di-(2-heptyl)amino, di-(1-ethyl-1,2-dimethyl-n-propyl)amino, di-(1-ethyl-2,2-dimethyl-n-propyl)amino, di-(1-octyl)amino, di-(3-octyl)amino, di-(4-methyl-3-n-heptyl)amino, di-(6-methyl-2-n-heptyl)amino, di-(2-propyl-1-n-heptyl)amino, di-(2,4,4-trimethyl-1-n-pentyl)amino, di-(1-nonyl)amino, di-(2-nonyl)amino, di-(2,6-dimethyl-4-n-heptyl)amino, di-(3-ethyl-2,2-dimethyl-3-n-pentyl)amino, di-(3,5,5-trimethyl-1-n-hexyl)amino, di-(1-decyl)amino, di-(2-decyl)amino, di-(4-decyl)amino, di-(3,7-dimethyl-1-n-octyl)amino, and di-(3,7-dimethyl-3-n-octyl)amino.

Examples of the asymmetric di C₁₋₁₀ alkylamino group may include linear C₁₋₁₀ alkylamino groups, branched C₁₋₁₀ alkylamino groups, and C₃₋₁₀ cycloalkylamino groups, and include (methyl, ethyl)amino, (methyl, n-propyl)amino, (methyl, i-propyl)amino, (methyl, c-propyl)amino, (methyl, n-butyl)amino, (methyl, i-butyl)amino, (methyl, s-butyl)amino, (methyl, t-butyl)amino, (methyl, n-pentyl)amino, (methyl, c-pentyl)amino, (methyl, n-hexyl)amino, (methyl, c-hexyl)amino, (ethyl, n-propyl)amino, (ethyl, i-propyl)amino, (ethyl, c-propyl)amino, (ethyl, n-butyl)amino, (ethyl, i-butyl)amino, (ethyl, s-butyl)amino, (ethyl, t-butyl)amino, (ethyl, n-pentyl)amino, (ethyl, c-pentyl)amino, (ethyl, n-hexyl)amino, (ethyl, c-hexyl)amino, (n-propyl, i-propyl)amino, (n-propyl, c-propyl)amino, (n-propyl, n-butyl)amino, (n-propyl, i-butyl)amino, (n-propyl, s-butyl)amino, (n-propyl, t-butyl)amino, (n-propyl, n-pentyl)amino, (n-propyl, c-pentyl)amino, (n-propyl, n-hexyl)amino, (n-propyl, c-hexyl)amino, (i-propyl, c-propyl)amino, (i-propyl, n-butyl)amino, (i-propyl, i-butyl)amino, (i-propyl, s-butyl)amino, (i-propyl, t-butyl)amino, (i-propyl, n-pentyl)amino, (i-propyl, c-pentyl)amino, (i-propyl, n-hexyl)amino, (i-propyl, c-hexyl)amino, (c-propyl, n-butyl)amino, (c-propyl, i-butyl)amino, (c-propyl, s-butyl)amino, (c-prnpyl, t-butyl)amino, (c-propyl, n-pentyl)amino, (c-propyl, c-pentyl)amino, (c-propyl, n-hexyl)amino, (c-propyl, c-hexyl)amino, (n-butyl, i-butyl)amino, (n-butyl, s-butyl)amino, (n-butyl, t-butyl)amino, (n-butyl, n-pentyl)amino, (n-butyl, c-pentyl)amino, (n-butyl, n-hexyl)amino, (n-butyl, c-hexyl)amino, (i-butyl, s-butyl)amino, (i-butyl, t-butyl)amino, (i-butyl, n-pentyl)amino, (i-butyl, c-pentyl)amino, (i-butyl, n-hexyl)amino, (i-butyl, c-hexyl)amino, (s-butyl, t-butyl)amino, (s-butyl, n-pentyl)amino, (s-butyl, c-pentyl)amino, (s-butyl, n-hexyl)amino, (s-butyl, c-hexyl)amino, (t-butyl, n-pentyl)amino, (t-butyl, c-pentyl)amino, (t-butyl, n-hexyl)amino, (t-butyl, c-hexyl)amino, (n-pentyl, c-pentyl)amino, (n-pentyl, n-hexyl)amino, (n-pentyl, c-hexyl)amino, (c-pentyl, n-hexyl)amino, (c-pentyl, c-hexyl)amino, (n-hexyl, c-hexyl)amino, (methyl, n-heptyl)amino, (methyl, n-octyl)amino, (methyl, n-nonanyl)amino, (methyl, n-decyl)amino, (ethyl, n-heptyl)amino, (ethyl, n-octyl)amino, (ethyl, n-nonanyl)amino, and (ethyl, n-decyl)amino.

Examples of the C₁₋₁₀ alkylaminocarbonyl group may include linear C₁₋₁₀ alkylaminocarbonyl groups, branched C₁₋₁₀ alkylaminocarbonyl groups, and C₃₋₁₀ cycloalkylaminocarbonyl groups and di C₁₋₁₀ alkylaminocarbonyl groups, and include methylaminocarbonyl, ethylaminocarbonyl, n-propylaminocarbonyl, i-propylaminocarbonyl, c-propylaminocarbonyl, n-butylaminocarbonyl, i-butylaminocarbonyl, s-butylaminocarbonyl, t-butylaminocarbonyl, c-butylaminocarbonyl, 1-methyl-c-propylaminocarbonyl, 2-methyl-c-propylaminocarbonyl, n-pentylaminocarbonyl, 1-methyl-n-butylaminocarbonyl, 2-methyl-n-butylaminocarbonyl, 3-methyl-n-butylaminocarbonyl, 1,1-dimethyl-n-propylaminocarbonyl, 1,2-dimethyl-n-propylaminocarbonyl, 2,2-dimethyl-n-propylaminocarbonyl, 1-ethyl-n-propylaminocarbonyl, c-pentylaminocarbonyl, 1-methyl-c-butylaminocarbonyl, 2-methyl-c-butylaminocarbonyl, 3-methyl-c-butylaminocarbonyl, 1,2-dimethyl-c-propylaminocarbonyl, 2,3-dimethyl-c-propylaminocarbonyl, 1-ethyl-c-propylaminocarbonyl, 2-ethyl-c-propylaminocarbonyl, n-hexylaminocarbonyl, 1-methyl-n-penlylaminocarbonyl, 2-methyl-n-pentylaminocarbonyl, 3-methyl-n-pentylaminocarbonyl, 4-methyl-n-pentylaminocarbonyl, 1,1-dimethyl-n-butylaminocarbonyl, 1,2-dimethyl-n-butylaminocarbonyl, 1,3-dimethyl-n-butylaminocarbonyl, 2,2-dimethyl-n-butylaminocarbonyl, 2,3-dimethyl-n-butylaminocarbonyl, 3,3-dimethyl-n-butylaminocarbonyl, 1-ethyl-n-butylaminocarbonyl, 2-ethyl-n-butylaminocarbonyl, 1,1,2-trimethyl-n-propylaminocarbonyl, 1,2,2-trimethyl-n-propylaminocarbonyl, 1-ethyl-1-methyl-n-propylaminocarbonyl, 1-ethyl-2-methyl-n-propylaminocarbonyl, c-hexylaminocarbonyl, 1-methyl-c-pentylaminocarbonyl, 2-methyl-c-pentylaminocarbonyl, 3-methyl-c-pentylaminocarbonyl, 1-ethyl-c-butylaminocarbonyl, 2-ethyl-c-butylaminocarbonyl, 3-ethyl-c-butylaminocarbonyl, 1,2-dimethyl-c-butylaminocarbonyl, 1,3-dimethyl-c-butylaminocarbonyl, 2,2-dimethyl-c-butylaminocarbonyl, 2,3-dimethyl-c-butylaminocarbonyl, 2,4-dimethyl-c-butylaminocarbonyl, 3,3-dimethyl-c-butylaminocarbonyl, 1-n-propyl-c-propylaminocarbonyl, 2-n-propyl-c-propylaminocarbonyl, 1-i-propyl-c-propylaminocarbonyl, 2-i-propyl-c-propylaminocarbonyl, 1,2,2-trimethyl-c-propylaminocarbonyl, 1,2,3-trimethyl-c-propylaminocarbonyl, 2,2,3-trimethyl-c-propylaminocarbonyl, 1-ethyl-2-methyl-c-propylaminocarbonyl, 2-ethyl-1-methyl-c-propylaminocarbonyl, 2-ethyl-2-methyl-c-propylaminocarbonyl, 2-ethyl-3-methyl-c-propylaminocarbonyl, 1-methyl-1-ethyl-n-pentylaminocarbonyl, 1-heptylaminocarbonyl, 2-heptylaminocarbonyl, 1-ethyl-1,2-dimethyl-n-propylaminocarbonyl, 1-ethyl-2,2-dimethyl-n-propylaminocarbonyl, 1-octylaminocarbonyl, 3-octylaminocarbonyl, 4-methyl-3-n-heptylaminocarbonyl, 6-methyl-2-n-heptylaminocarbonyl, 2-propyl-1-n-heptylaminocarbonyl, 2,4,4-trimethyl-1-n-pentylaminocarbonyl, 1-nonylaminocarbonyl, 2-nonylaminocarbonyl, 2,6-dimethyl-4-n-heptylaminocarbonyl, 3-ethyl-2,2-dimethyl-3-n-pentylaminocarbonyl, 3,5,5-trimethyl-1-n-hexylaminocarbonyl, 1-decylaminocarbonyl, 2-decylaminocarbonyl, 4-decylaminocarbonyl, 3,7-dimethyl-1-n-octylaminocarbonyl, and 3,7-dimethyl-3-n-octylaminocarbonyl.
Examples of the di C₁₋₁₀ alkylaminocarbonyl group may include symmetric and asymmetric dialkylaminocarbonyl groups. Examples of the symmetric di C₁₋₁₀ alkylaminocarbonyl group may include linear C₁₋₁₀ alkylaminocarbonyl groups, branched C₁₋₁₀ alkylaminocarbonyl groups, and C₃₋₁₀ cycloalkylaminocarbonyl groups, and include dimethylaminocarbonyl, diethylaminocarbonyl, di-n-propylaminocarbonyl, di-i-propylaminocarbonyl, di-c-propylaminocarbonyl, di-n-butylaminocarbonyl, di-i-butylaminocarbonyl, di-s-butylaminocarbonyl, di-t-butylaminocarbonyl, di-c-butylaminocarbonyl, di-(1-methyl-c-propyl)aminocarbonyl, di-(2-methyl-c-propyl)aminocarbonyl, di-n-pentylaminocarbonyl, di-(1-methyl-n-butyl)aminocarbonyl, di-(2-methyl-n-butyl)aminocarbonyl, di-(3-methyl-n-butyl)aminocarbonyl, di-(1,1-dimethyl-n-propyl)aminocarbonyl, di-(1,2-dimethyl-n-propyl)aminocarbonyl, di-(2,2-dimethyl-n-propyl)aminocarbonyl, di-(1-ethyl-n-propyl)aminocarbonyl, di-c-pentylaminocarbonyl, di-(1-methyl-c-butyl)aminocarbonyl, di-(2-methyl-c-butyl)aminocarbonyl, di-(3-methyl-c-butyl)aminocarbonyl, di-(1,2-dimethyl-c-propyl)aminocarbonyl, di-(2,3-dimethyl-c-propyl)aminocarbonyl, di-(1-ethyl-c-propyl)aminocarbonyl, di-(2-ethyl-c-propyl)aminocarbonyl, di-n-hexylaminocarbonyl, di-(1-methyl-n-pentyl)aminocarbonyl, di-(2-methyl-n-pentyl)aminocarbonyl, di-(3-methyl-n-pentyl)aminocarbonyl, di-(4-methyl-n-pentyl)aminocarbonyl, di-(1,1-dimethyl-n-butyl)aminocarbonyl, di-(1,2-dimethyl-n-butyl)aminocarbonyl, di-(1,3-dimethyl-n-butyl)aminocarbonyl, di-(2,2-dimethyl-n-butyl)aminocarbonyl, di-(2,3-dimethyl-n-butyl)aminocarbonyl, di-(3,3-dimethyl-n-butyl)aminocarbonyl, di-(1-ethyl-n-butyl)aminocarbonyl, di-(2-ethyl-n-butyl)aminocarbonyl, di-(1,1,2-trimethyl-n-propyl)aminocarbonyl, di-(1,2,2-trimethyl-n-propyl)aminocarbonyl, di-(1-ethyl-1-methyl-n-propyl)aminocarbonyl, di-(1-ethyl-2-methyl-n-propyl)aminocarbonyl, di-c-hexylaminocarbonyl, di-(1-methyl-c-pentyl)aminocarbonyl, di-(2-methyl-c-pentyl)aminocarbonyl, di-(3-methyl-c-pentyl)aminocarbonyl, di-(1-ethyl-c-butyl)aminocarbonyl, di-(2-ethyl-c-butyl)aminocarbonyl, di-(3-ethyl-c-butyl)aminocarbonyl, di-(1,2-dimethyl-c-butyl)aminocarbonyl, di-(1,3-dimethyl-c-butyl)aminocarbonyl, di-(2,2-dimethyl-c-butyl)aminocarbonyl, di-(2,3-dimethyl-c-butyl)aminocarbonyl, di-(2,4-dimethyl-c-butyl)aminocarbonyl, di-(3,3-dimethyl-c-butyl)aminocarbonyl, di-(1-n-propyl-c-propyl)aminocarbonyl, di-(2-n-propyl-c-propyl)aminocarbonyl, di-(1-i-propyl-c-propyl)aminocarbonyl, di-(2-i-propyl-c-propyl)aminocarbonyl, di-(1,2,2-trimethyl-c-propyl)aminocarbonyl, di-(1,2,3-trimethyl-c-propyl)aminocarbonyl, di-(2,2,3-trimethyl-c-propyl)aminocarbonyl, di-(1-ethyl-2-methyl-c-propyl)aminocarbonyl, di-(2-ethyl-1-methyl-c-propyl)aminocarbonyl, di-(2-ethyl-2-methyl-c-propyl)aminocarbonyl, di-(2-ethyl-3-methyl-c-propyl)aminocarbonyl, di-(1-methyl-1-ethyl-n-pentyl)aminocarbonyl, di-(1-heptyl)aminocarbonyl, di-(2-heptyl)aminocarbonyl, di-(1-ethyl-1,2-dimethyl-n-propyl)aminocarbonyl, di-(1-ethyl-2,2-dimethyl-n-propyl)aminocarbonyl, di-(1-octyl)aminocarbonyl, di-(3-octyl)aminocarbonyl, di-(4-methyl-3-n-heptyl)aininocarbonyl, di-(6-methyl-2-n-heptyl)aminocarbonyl, di-(2-propyl-1-n-heptyl)aminocarbonyl, di-(2,4,4-trimethyl-1-n-pentyl)aminocarbonyl, di-(1-nonyl)aminocarbonyl, di-(2-nonyl)aminocarbonyl, di-(2,6-dimethyl-4-n-heptyl)aminocarbonyl, di-(3-ethyl-2,2-dimethyl-3-n-pentyl)aminocarbonyl, di-(3,5,5-trimethyl-1-n-hexyl)aminocarbonyl, di-(1-decyl)aminocarbonyl, di-(2-decyl)aminocarbonyl, di-(4-decyl)aminocarbonyl, di-(3,7-dimethyl-1-n-octyl)aminocarbonyl, and di-(3,7-dimethyl-3-n-octyl)aminocarbonyl.

Examples of the asymmetric di C₁₋₁₀ alkylaminocarbonyl group may include linear C₁₋₁₀ alkylaminocarbonyl groups, branched C₁₋₁₀ alkylaminocarbonyl groups, and C₃₋₁₀ cycloalkylaminocarbonyl groups, and include (methyl, ethyl)aminocarbonyl, (methyl, n-propyl)aminocarbonyl, (methyl, i-propyl)aminocarbonyl, (methyl, c-propyl)aminocarbonyl, (methyl, n-butyl)aminocarbonyl, (methyl, i-butyl)aminocarbonyl, (methyl, s-butyl)aminocarbonyl, (methyl, t-butyl)aminocarbonyl, (methyl, n-pentyl)aminocarbonyl, (methyl, c-pentyl)aminocarbonyl, (methyl, n-hexyl)aminocarbonyl, (methyl, c-hexyl)aminocarbonyl, (ethyl, n-propyl)aminocarbonyl, (ethyl, i-propyl)aminocarbonyl, (ethyl, c-propyl)aminocarbonyl, (ethyl, n-butyl)aminocarbonyl, (ethyl, i-butyl)aminocarbonyl, (ethyl, s-butyl)aminocarbonyl, (ethyl, t-butyl)aminocarbonyl, (ethyl, n-pentyl)aminocarbonyl, (ethyl, c-pentyl)aminocarbonyl, (ethyl, n-hexyl)aminocarbonyl, (ethyl, c-hexyl)aminocarbonyl, (n-propyl, i-propyl)aminocarbonyl, (n-propyl, c-propyl)aminocarbonyl, (n-propyl, n-butyl)aminocarbonyl, (n-propyl, i-butyl)aminocarbonyl, (n-propyl, s-butyl)aminocarbonyl, (n-propyl, t-butyl)aminocarbonyl, (n-propyl, n-pentyl)aminocarbonyl, (n-propyl, c-pentyl)aminocarbonyl, (n-propyl, n-hexyl)aminocarbonyl, (n-propyl, c-hexyl)aminocarbonyl, (i-propyl, c-propyl)aminocarbonyl, (i-propyl, n-butyl)aminocarbonyl, (i-propyl, i-butyl)aminocarbonyl, (i-propyl, s-butyl)aminocarbonyl, (i-propyl, t-butyl)aminocarbonyl, (i-propyl, n-pentyl)aminocarbonyl, (i-propyl, c-pentyl)aminocarbonyl, (i-propyl, n-hexyl)aminocarbonyl, (i-propyl, c-hexyl)aminocarbonyl, (c-propyl, n-butyl)aminocarbonyl, (c-propyl, i-butyl)aminocarbonyl, (c-propyl, s-butyl)aminocarbonyl, (c-propyl, t-butyl)aminocarbonyl, (c-propyl, n-pentyl)aminocarbonyl, (c-propyl, c-pentyl)aminocarbonyl, (c-propyl, n-hexyl)aminocarbonyl, (c-propyl, c-hexyl)aminocarbonyl, (n-butyl, i-butyl)aminocarbonyl, (n-butyl, s-butyl)aminocarbonyl, (n-butyl, t-butyl)aminocarbonyl, (n-butyl, n-pentyl)aminocarbonyl, (n-butyl, c-pentyl)aminocarbonyl, (n-butyl, n-hexyl)aminocarbonyl, (n-butyl, c-hexyl)aminocarbonyl, (i-butyl, s-butyl)aminocarbonyl, (i-butyl, t-butyl)aminocarbonyl, (i-butyl, n-pentyl)aminocarbonyl, (i-butyl, c-pentyl)aminocarbonyl, (i-butyl, n-hexyl)aminocarbonyl, (i-butyl, c-hexyl)aminocarbonyl, (s-butyl, t-butyl)aminocarbonyl, (s-butyl, n-pentyl)aminocarbonyl, (s-butyl, c-pentyl)aminocarbonyl, (s-butyl, n-hexyl)aminocarbonyl, (s-butyl, c-hexyl)aminocarbonyl, (t-butyl, n-pentyl)aminocarbonyl, (t-butyl, c-pentyl)aminocarbonyl, (t-butyl, n-hexyl)aminocarbonyl, (t-butyl, c-hexyl)aminocarbonyl, (n-pentyl, c-pentyl)aminocarbonyl, (n-pentyl, n-hexyl)aminocarbonyl, (n-pentyl, c-hexyl)aminocarbonyl, (c-pentyl, n-hexyl)aminocarbonyl, (c-pentyl, c-hexyl)aminocarbonyl, (n-hexyl, c-hexyl)aminocarbonyl, (methyl, n-heptyl)aminocarbonyl, (methyl, n-octyl)aminocarbonyl, (methyl, n-nonanyl)aminocarbonyl, (methyl, n-decyl)aminocarbonyl, (ethyl, n-heptyl)aminocarbonyl, (ethyl, n-octyl)aminocarbonyl, (ethyl, n-nonanyl)aminocarbonyl, and (ethyl, n-decyl)aminocarbonyl.

Examples of the C₁₋₁₀ alkylaminosulfonyl group may include linear C₁₋₁₀ alkylaminosulfonyl groups, branched C₁₋₁₀ alkylaminosulfonyl groups, and C₃₋₁₀ cycloalkylaminosulfonyl groups and di C₁₋₁₀ alkylaminosulfonyl groups, and include methylaminosulfonyl, ethylaminosulfonyl, n-propylaminosulfonyl, i-propylaminosulfonyl, c-propylaminosulfonyl, n-butylaminosulfonyl, i-butylaminosulfonyl, s-butylaminosulfonyl, t-butylaminosulfonyl, c-butylaminosulfonyl, 1-methyl-c-propylaminosulfonyl, 2-methyl-c-propylaminosulfonyl, n-pentylaminosulfonyl, 1-methyl-n-butylaminosulfonyl, 2-methyl-n-butylaminosulfonyl, 3-methyl-n-butylaminosulfonyl, 1,1-dimethyl-n-propylaminosulfonyl, 1,2-dimethyl-n-propylaminosulfonyl, 2,2-dimethyl-n-propylaminosulfonyl, 1-ethyl-n-propylaminosulfonyl, c-pentylaminosulfonyl, 1-methyl-c-butylaminosulfonyl, 2-methyl-c-butylaminosulfonyl, 3-methyl-c-butylaminosulfonyl, 1,2-dimethyl-c-propylaminosulfonyl, 2,3-dimethyl-c-propylaminosulfonyl, 1-ethyl-c-propylaminosulfonyl, 2-ethyl-c-propylaminosulfonyl, n-hexylaminosulfonyl, 1-methyl-n-pentylaminosulfonyl, 2-methyl-n-pentylaminosulfonyl, 3-methyl-n-pentylaminosulfonyl, 4-methyl-n-pentylaminosulfonyl, 1,1-dimethyl-n-butylaminosulfonyl, 1,2-dimethyl-n-butylaminosulfonyl, 1,3-dimethyl-n-butylaminosulfonyl, 2,2-dimethyl-n-butylaminosulfonyl, 2,3-dimethyl-n-butylaminosulfonyl, 3,3-dimethyl-n-butylaminosulfonyl, 1-ethyl-n-butylaminosulfonyl, 2-ethyl-n-butylaminosulfonyl, 1,1,2-trimethyl-n-propylaminosulfonyl, 1,2,2-trimethyl-n-propylaminosulfonyl, 1-ethyl-1-methyl-n-propylaminosulfonyl, 1-ethyl-2-methyl-n-propylaminosulfonyl, c-hexyl amino sulfonyl, 1-methyl-c-pentylaminosulfonyl, 2-methyl-c-pentylaminosulfonyl, 3-methyl-c-pentylaminosulfonyl, 1-ethyl-c-butylaminosulfonyl, 2-ethyl-c-butylaminosulfonyl, 3-ethyl-c-butylaminosulfonyl, 1,2-dimethyl-c-butylaminosulfonyl, 1,3-dimethyl-c-butylaminosulfonyl, 2,2-dimethyl-c-butylaminosulfonyl, 2,3-dimethyl-c-butylaminosulfonyl, 2,4-dimethyl-c-butylaminosulfonyl, 3,3-dimethyl-c-butylaminosulfonyl, 1-n-propyl-c-propylaminosulfonyl, 2-n-propyl-c-propylaminosulfonyl, 1-i-propyl-c-propylaminosulfonyl, 2-i-propyl-c-propylaminosulfonyl, 1,2,2-trimethyl-c-propylaminosulfonyl, 1,2,3-trimethyl-c-propylaminosulfonyl, 2,2,3-trimethyl-c-propylaminosulfonyl, 1-ethyl-2-methyl-c-propylaminosulfonyl, 2-ethyl-1-methyl-c-propylaminosulfonyl, 2-ethyl-2-methyl-c-propylaminosulfonyl, 1-methyl-1-ethyl-n-pentylaminosulfonyl, 1-heptylaminosulfonyl, 2-heptylaminosulfonyl, 1-methyl-1,2-dimethyl-n-propylaminosulfonyl, 1-ethyl-2,2-dimethyl-n-propylaminosulfonyl, 1-octylaminosulfonyl, 3-octylaminosulfonyl, 4-methyl-3-n-heptylaminosulfonyl, 6-methyl-2-n-heptylaminosulfonyl, 2-propyl-1-n-heptylaminosulfonyl, 2,4,4-trimethyl-1-n-pentylaminosulfonyl, 1-nonylaminosulfonyl, 2-nonylaminosulfonyl, 2,6-dimethyl-4-n-heptylaminosulfonyl, 3-ethyl-2,2-dimethyl-3-n-pentylaminosulfonyl, 3,5,5-trimethyl-1-n-hexylaminosulfonyl, 1-decylaminosulfonyl, 2-decylaminosulfonyl, 4-decylaminosulfonyl, 3,7-dimethyl-1-n-octylaminosulfonyl, 3,7-dimethyl-3-n-octylaminosulfonyl, c-heptylaminosulfonyl, c-octylaminosulfonyl, 1-methyl-c-hexylaminosulfonyl, 2-methyl-c-hexylaminosulfonyl, 3-methyl-c-hexylaminosulfonyl, 1,2-dimethyl-c-hexylaminosulfonyl, 1-ethyl-c-hexylaminosulfonyl, 1-methyl-c-pentylaminosulfonyl, 2-methyl-c-pentylaminosulfonyl, and 3-methyl-c-pentylaminosulfonyl.

Examples of the di C₁₋₁₀ alkylaminosulfonyl group may include symmetric and asymmetric dialkylaminosulfonyl groups. Examples of the symmetric di C₁₋₁₀ alkylaminosulfonyl group may include linear C₁₋₁₀ alkylaminosulfonyl groups, branched C₁₋₁₀ alkylaminosulfonyl groups, and C₃₋₁₀ cycloalkylaminosulfonyl groups, and include dimethylaminosulfonyl, diethylaminosulfonyl, di-n-propylaminosulfonyl, di-i-propylaminosulfonyl, di-c-propylaminosulfonyl, di-n-butylaminosulfonyl, di-i-butylaminosulfonyl, di-s-butylaminosulfonyl, di-t-butylaminosulfonyl, di-c-butyl amino sulfonyl, di-(1-methyl-c-propyl)aminosulfonyl, di-(2-methyl-c-propyl)aminosulfonyl, di-n-pentylaminosulfonyl, di-(1-methyl-n-butyl)aminosulfonyl, di-(2-methyl-n-butyl)aminosulfonyl, di-(3-methyl-n-butyl)aminosulfonyl, di-(1,1-dimethyl-n-propyl)aminosulfonyl, di-(1,2-dimethyl-n-propyl)aminosulfonyl, di-(2,2-dimethyl-n-propyl)aminosulfonyl, di-(1-ethyl-n-propyl)aminosulfonyl, di-c-pentylaminosulfonyl, di-(1-methyl-c-butyl)aminosulfonyl, di-(2-methyl-c-butyl)aminosulfonyl, di-(3-methyl-c-butyl)aminosulfonyl, di-(1,2-dimethyl-c-propyl)aminosulfonyl, di-(2,3-dimethyl-c-propyl)aminosulfonyl, di-(1-ethyl-c-propyl)aminosulfonyl, di-(2-ethyl-c-propyl)aminosulfonyl, di-n-hexylaminosulfonyl, di-(1-methyl-n-pentyl)aminosulfonyl, di-(2-methyl-n-pentyl)aminosulfonyl, di-(3-methyl-n-pentyl)aminosulfonyl, di-(4-methyl-n-pentyl)aminosulfonyl, di-(1,1-dimethyl-n-butyl)aminosulfonyl, di-(1,2-dimethyl-n-butyl)aminosulfonyl, di-(1,3-dimethyl-n-butyl)aminosulfonyl, di-(2,2-dimethyl-n-butyl)aminosulfonyl, di-(2,3-dimethyl-n-butyl)aminosulfonyl, di-(3,3-dimethyl-n-butyl)aminosulfonyl, di-(1-ethyl-n-butyl)aminosulfonyl, di-(2-ethyl-n-butyl)aminosulfonyl, di-(1,1,2-trimethyl-n-propyl)aminosulfonyl, di-(1,2,2-trimethyl-n-propyl)aminosulfonyl, di-(1-ethyl-1-methyl-n-propyl)aminosulfonyl, di-(1-ethyl-2-methyl-n-propyl)aminosulfonyl, di-c-hexylaminosulfonyl, di-(1-methyl-c-pentyl)aminosulfonyl, di-(2-methyl-c-pentyl)aminosulfonyl, di-(3-methyl-c-pentyl)aminosulfonyl, di-(1-ethyl-c-butyl)aminosulfonyl, di-(2-ethyl-c-butyl)aminosulfonyl, di-(3-ethyl-c-butyl)aminosulfonyl, di-(1,2-dimethyl-c-butyl)aminosulfonyl, di-(1,3-dimethyl-c-butyl)aminosulfonyl, di-(2,2-dimethyl-c-butyl)aminosulfonyl, di-(2,3-dimethyl-c-butyl)aminosulfonyl, di-(2,4-dimethyl-c-butyl)aminosulfonyl, di-(3,3-dimethyl-c-butyl)aminosulfonyl, di-(1-n-propyl-c-propyl)aminosulfonyl, di-(2-n-propyl-c-propyl)aminosulfonyl, di-(1-i-propyl-c-propyl)aminosulfonyl, di-(2-i-propyl-c-propyl)aminosulfonyl, di-(1,2,2-trimethyl-c-propyl)aminosulfonyl, di-(1,2,3-trimethyl-c-propyl)aminosulfonyl, di-(2,2,3-trimethyl-c-propyl)aminosulfonyl, di-(1-ethyl-2-methyl-c-propyl)aminosulfonyl, di-(2-ethyl-1-methyl-c-propyl)aminosulfonyl, di-(2-ethyl-2-methyl-c-propyl)aminosulfonyl, di-(2-ethyl-3-methyl-c-propyl)aminosulfonyl, di-(1-methyl-1-ethyl-n-pentyl)aminosulfonyl, di-(1-heptyl)aminosulfonyl, di-(2-heptyl)aminosulfonyl, di-(1-ethyl-1,2-dimethyl-n-propyl)aminosulfonyl, di-(1-ethyl-2,2-dimethyl-n-propyl)aminosulfonyl, di-(1-octyl)aminosulfonyl, di-(3-octyl)aminosulfonyl, di-(4-methyl-3-n-heptyl)aminosulfonyl, di-(6-methyl-2-n-heptyl)aminosulfonyl, di-(2-propyl-1-n-heptyl)aminosulfonyl, di-(2,4,4-trimethyl-1-n-pentyl)aminosulfonyl, di-(1-nonyl)aminosulfonyl, di-(2-nonyl)aminosulfonyl, di-(2,6-dimethyl-4-n-heptyl)aminosulfonyl, di-(3-ethyl-2,2-dimethyl-3-n-pentyl)aminosulfonyl, di-(3,5,5-trimethyl-1-n-hexyl)ammosulfonyl, di-(1-decyl)aminosulfonyl, di-(2-decyl)aminosulfonyl, di-(4-decyl)amininosulfonyl, di-(3,7-dimethyl-1-n-octyl)aminosulfonyl, and di-(3,7-dimethyl-3-n-octyl)aminosulfonyl.

Examples of the asymmetric di C₁₋₁₀ alkylaminosulfonyl group may include linear C₁₋₁₀ alkylaminosulfonyl groups, branched C₁₋₁₀ alkylaminosulfonyl groups, and C₃₋₁₀ cycloalkylaminosulfonyl groups, and include (methyl, ethyl)aminosulfonyl, (methyl, n-propyl)aminosulfonyl, (methyl, i-propyl)aminosulfonyl, (methyl, c-propyl)aminosulfonyl, (methyl, n-butyl)aminosulfonyl, (methyl, i-butyl)aminosulfonyl, (methyl, s-butyl)aminosulfonyl, (methyl, t-butyl)aminosulfonyl, (methyl, n-pentyl)aminosulfonyl, (methyl, c-pentyl)aminosulfonyl, (methyl, n-hexyl)aminosulfonyl, (methyl, c-hexyl)aminosulfonyl, (ethyl, n-propyl)aminosulfonyl, (ethyl, i-propyl)aminosulfonyl, (ethyl, c-propyl)aminosulfonyl, (ethyl, n-butyl)aminosulfonyl, (ethyl, i-butyl)aminosulfonyl, (ethyl, s-butyl)aminosulfonyl, (ethyl, t-butyl)aminosulfonyl, (ethyl, n-pentyl)aminosulfonyl, (ethyl, c-pentyl)aminosulfonyl, (ethyl, n-hexyl)aminosulfonyl, (ethyl, c-hexyl)aminosulfonyl, (n-propyl, i-propyl)aminosulfonyl, (n-propyl, c-propyl)aminosulfonyl, (n-propyl, n-butyl)aminosulfonyl, (n-propyl, i-butyl)aminosulfonyl, (n-propyl, s-butyl)aminosulfonyl, (n-propyl, t-butyl)aminosulfonyl, (n-propyl, n-pentyl)aminosulfonyl, (n-propyl, c-pentyl)aminosulfonyl, (n-propyl, n-hexyl)aminosulfonyl, (n-propyl, c-hexyl)aminosulfonyl, (i-propyl, c-propyl)aminosulfonyl, (i-propyl, n-butyl)aminosulfonyl, (i-propyl, i-butyl)aminosulfonyl, (i-propyl, s-butyl)aminosulfonyl, (i-propyl, t-butyl)aminosulfonyl, (i-propyl, n-pentyl)aminosulfonyl, (i-propyl, c-pentyl)aminosulfonyl, (i-propyl, n-hexyl)aminosulfonyl, (i-propyl, c-hexyl)aminosulfonyl, (c-propyl, n-butyl)aminosulfonyl, (c-propyl, i-butyl)aminosulfonyl, (c-propyl, s-butyl)aminosulfonyl, (c-propyl, t-butyl)aminosulfonyl, (c-propyl, n-pentyl)aminosulfonyl, (c-propyl, c-pentyl)aminosulfonyl, (c-propyl, n-hexyl)aminosulfonyl, (c-propyl, c-hexyl)aminosulfonyl, (n-butyl, i-butyl)aminosulfonyl, (n-butyl, s-butyl)aminosulfonyl, (n-butyl, t-butyl)aminosulfonyl, (n-butyl, n-pentyl)aminosulfonyl, (n-butyl, c-pentyl)aminosulfonyl, (n-butyl, n-hexyl)aminosulfonyl, (n-butyl, c-hexyl)aminosulfonyl, (i-butyl, s-butyl)aminosulfonyl, (i-butyl, t-butyl)aminosulfonyl, (i-butyl, n-pentyl)aminosulfonyl, (i-butyl, c-pentyl)aminosulfonyl, (i-butyl, n-hexyl)aminosulfonyl, (i-butyl, c-hexyl)aminosulfonyl, (s-butyl, t-butyl)aminosulfonyl, (s-butyl, n-pentyl)aminosulfonyl, (s-butyl, c-pentyl)aminosulfonyl, (s-butyl, n-hexyl)aminosulfonyl, (s-butyl, c-hexyl)aminosulfonyl, (t-butyl, n-pentyl)aminosulfonyl, (t-butyl, c-pentyl)aminosulfonyl, (t-butyl, n-hexyl)aminosulfonyl, (t-butyl, c-hexyl)aminosulfonyl, (n-pentyl, c-pentyl)aminosulfonyl, (n-pentyl, n-hexyl)aminosulfonyl, (n-pentyl, c-hexyl)aminosulfonyl, (c-pentyl, n-hexyl)aminosulfonyl, (c-pentyl, c-hexyl)aminosulfonyl, (n-hexyl, c-hexyl)aminosulfonyl, (methyl, n-heptyl)aminosulfonyl, (methyl, n-octyl)aminosulfonyl, (methyl, n-nonanyl)aminosulfonyl, (methyl, n-decyl)aminosulfonyl, (ethyl, n-heptyl)aminosulfonyl, (ethyl, n-octyl)aminosulfonyl, (ethyl, n-nonanyl)aminosulfonyl, and (ethyl, n-decyl)aminosulfonyl.

Examples of the protective group in the protected hydroxy group, the protected amino group, the protected thiol group, and the protective group for an amino group include protective groups described in, for example, Protective Groups in Organic Synthesis, Fourth edition, authored by T.W. Greene, John Wiley & Sons Inc. (2006). Specific examples thereof include a C₁₋₄ alkoxymethyl group (such as MOM: methoxymethyl, MEM: 2-methoxyethoxymethyl, ethoxymethyl, n-propoxymethyl, i-propoxymethyl, n-butoxymethyl, iBM: isobutyloxymethyl, BUM: t-butoxymethyl, POM: pivaloyloxymethyl, and SEM: trimethylsilylethoxymethyl, and preferred are a C₁₋₂ alkoxymethyl group and the like), an aryloxymethyl group (such as BOM: benzyloxymethyl, PMBM: p-methoxybenzyloxymethyl, and p-AOM: P-anisyloxymethyl, and preferred are benzyloxymethyl and the like), a C₁₋₄ alkylaminomethyl group (such as dimethylaminomethyl), a substituted-acetamidomethyl group (such as Acm; acetamidomethyl and Tacm: trimethylacetamidomethyl), a substituted-thiomethyl group (such as MTM: methylthiomethyl, PTM: phenylthiomethyl, and Btm: benzylthiomethyl), a carboxyl group, a C₁₋₇ acyl group (such as formyl, acetyl, fluoroacetyl, difluoroacetyl, trifluoroacetyl, chloroacetyl, dichloroacetyl, trichloroacetyl, propionyl, Pv: pivaloyl, and tigloyl), an arylcarbonyl group (such as benzoyl, p-bromobenzoyl, p-nitrobenzoyl, 2,4-dinitrobenzoyl, benzoylformyl, benzoylpropionyl, and phenylpropionyl), a C₁₋₄ alkoxycarbonyl group (such as methoxycarbonyl, ethoxycarbonyl, n-propoxycarbonyl, i-propoxycarbonyl, n-butoxycarbonyl, i-butoxycarbonyl, Boc: t-butoxycarbonyl, AOC: t-amyloxycarbonyl, VOC: vinyloxycarbonyl, AOC: allyloxycarbonyl, Teoc: 2-(trimethylsilyl)ethoxycarbonyl, and Troc: 2,2,2-trichloroethoxycarbonyl, and preferred are Boc and the like), an aryloxycarbonyl group (such as Cbz: benzyloxycarbonyl, p-nitrobenzyloxycarbonyl, and MOZ: p-methoxybenzyloxycarbonyl), a C₁₋₄ alkylaminocarbonyl group (such as methylcarbamoyl, Ec: ethylcarbamoyl, and n-propylcarbamoyl), an arylaminocarbonyl group (such as phenylcarbamoyl), a trialkylsilyl group (such as TMS: trimethylsilyl, TES: triethylsilyl, TIPS: triisopropylsilyl, DEIPS: diethylisopropylsilyl, DMIPS: dimethylisopropylsilyl, DTBMS: di-t-butylmethylsilyl, IPDMS: isopropyldimethylsilyl, TBDMS: t-butyldimethylsilyl, and TDS: thexyldimethylsilyl, and preferred are t-butyldimethylsilyl and the like), a trialkylarylsilyl group (such as DPMS: diphenylmethylsilyl, TBDPS: t-butyldiphenylsilyl, TBMPS: t-butyldimethoxyphenylsilyl, and TPS: triphenylsilyl), an alkylsulfonyl group (such as Ms: methanesulfonyl and ethanesulfonyl), and an arylsulfonyl group (such as benzenesulfonyl, Ts: p-toluenesulfonyl, p-chlorobenzenesulfonyl, MBS: p-methoxybenzenesulfonyl, m-nitrobenzenesulfonyl, o-nitrobenzenesulfonyl, p-nitrobenzenesulfonyl, 2,4-nitrobenzenesulfonyl, iMds: 2,6-dimethoxy-4-methylbenzenesulfonyl, Mds: 2,6-dimethyl-4-methoxybenzenesulfonyl, Mtb: 2,4,6-trimethoxybenzenesulfonyl, Mte: 2,3,5,6-tetramethyl-4-methoxybenzenesulfonyl, Mtr: 2,3,6-trimethyl-4-methoxybenzenesulfonyl, Mts: 2,4,6-trimethylbenzenesulfonyl, and Pme: pentamethylbenzenesulfonyl).
Besides them, specific examples of the protective group also include a 1-methyl-1-methoxyethyl group, a 1-ethoxyethyl group, a 2,2,2-trichloroethyl group, a 2-trimethylsilylethoxy group, a t-butyl group, an allyl group, a benzyl group, a p-methoxybenzyl group, a 2,4-dinitrophenyl group, a p-chlorophenyl group, a p-methoxyphenyl group, a tetrahydropyranyl group, and a tetrahydrofuranyl group.

The optically active dinickel complex of the present invention is described.

Preferred examples of R⁰, R¹, R², R³, R⁴, R⁵, and R⁶ in Formula (I) and Formula (I') include a hydrogen atom, a halogen atom, a C₁₋₁₀ alkyl group, a C₂₋₆ alkenyl group, a C₂₋₆ alkynyl group, a C₁₋₁₀ alkoxy group, a C₂₋₁₄ aryl group or a C₂₋₁₄ aryloxy group, a halogen atom, a nitro group, and a cyano group. Other preferred examples thereof include the ring structures below formed by one or more set(s) of adjacent substituents, such as R⁰ and R¹, R¹ and R², R² and R³, R⁴ and R⁵, and R⁵ and R⁶, independently together with a benzene ring to which the substituents are bonded. 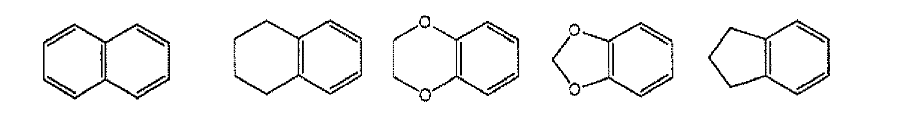 More preferred examples thereof include a hydrogen atom, a halogen atom, a nitro group, a C₁₋₃ alkyl group, a C₁₋₃ alkoxy group, a phenyl group, and a phenoxy group. Other more preferred examples thereof include the ring structures below formed by one or more set(s) of adjacent substituents, such as R⁰ and R¹, R¹ and R², R² and R³, R⁴ and R⁵, and R⁵ and R⁶, independently together with a benzene ring to which the substituents are bonded. 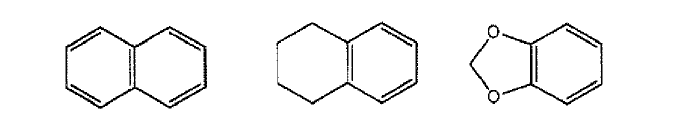 Particularly preferred examples thereof include an example in which R⁰, R¹, R², R⁴, R⁵, and R⁶ are a hydrogen atom and R³ is a methyl group, a methoxy group, or a bromine atom and an example in which R⁰, R¹, R⁴, R⁵, and R⁶ are a hydrogen atom and R⁷ and R³ form, together with a benzene ring to which R² and R³ are bonded, the ring structures below.

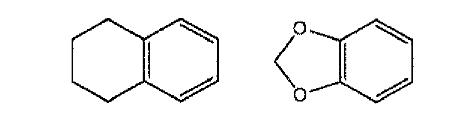

Preferred examples of R⁷ include a hydrogen atom, a C₁₋₁₀ alkyl group, a C₂₋₆ alkenyl group, a C₂₋₆ alkynyl group, and a C₂₋₁₄ aryl group.
More preferred examples thereof include a hydrogen atom and a C₁₋₃ alkyl group and particularly preferred examples thereof include a hydrogen atom.

In Formula (II) and Formula (II'), R⁰, R¹, R⁴, R⁵, R⁶, R⁸, R⁹, R¹⁰, and R¹¹ are a substituent on a phenyl ring and preferred examples thereof include a hydrogen atom, a halogen atom, a C₁₋₁₀ alkyl group, a C₂₋₆ alkenyl group, a C₂₋₆ alkynyl group, a C₁₋₁₀ alkoxy group, a C₂₋₁₄ aryl group, and a C₂₋₁₄ aryloxy group. Other preferred examples thereof include the ring structures below formed by one or more set(s) of adjacent substituents, such as R⁰ and R¹, R⁴ and R⁵, R⁵ and R⁶, R⁸ and R⁹, R⁹ and R¹⁰, and R¹⁰ and R¹¹ independently together with a benzene ring to which the substituents are bonded. 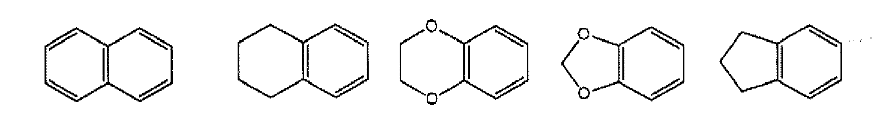 More preferred examples thereof include a hydrogen atom, a halogen atom, a nitro group, a C₁₋₃ alkyl group, a C₁₋₃ alkoxy group, a phenyl group, and a phenoxy group. Other more preferred examples thereof include the ring structures below formed by one or more set(s) of adjacent substituents, such as R⁰ and R¹, R⁴ and R⁵, R⁵ and R⁶, R⁸ and R⁹, R⁹ and R¹⁰, and R¹⁰ and R¹¹, independently together with a benzene ring to which the substituents are bonded. 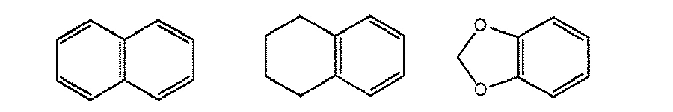 Particularly preferred is where R⁰, R¹, R⁴, R⁵, R⁶, R⁸, R⁹, R¹⁰, and R¹¹ are a hydrogen atom.

Preferred examples of R⁷ include a hydrogen atom, a C₁₋₁₀ alkyl group, a C₂₋₆ alkenyl group, a C₂₋₆ alkynyl group, and a C₂₋₁₄ aryl group.
More preferred examples thereof include a hydrogen atom and a C₁₋₃ alkyl group and particularly preferred examples thereof include a hydrogen atom.

Particularly preferred specific examples of Formula (I), Formula (I'), Formula (II), and Formula (II') are shown below.

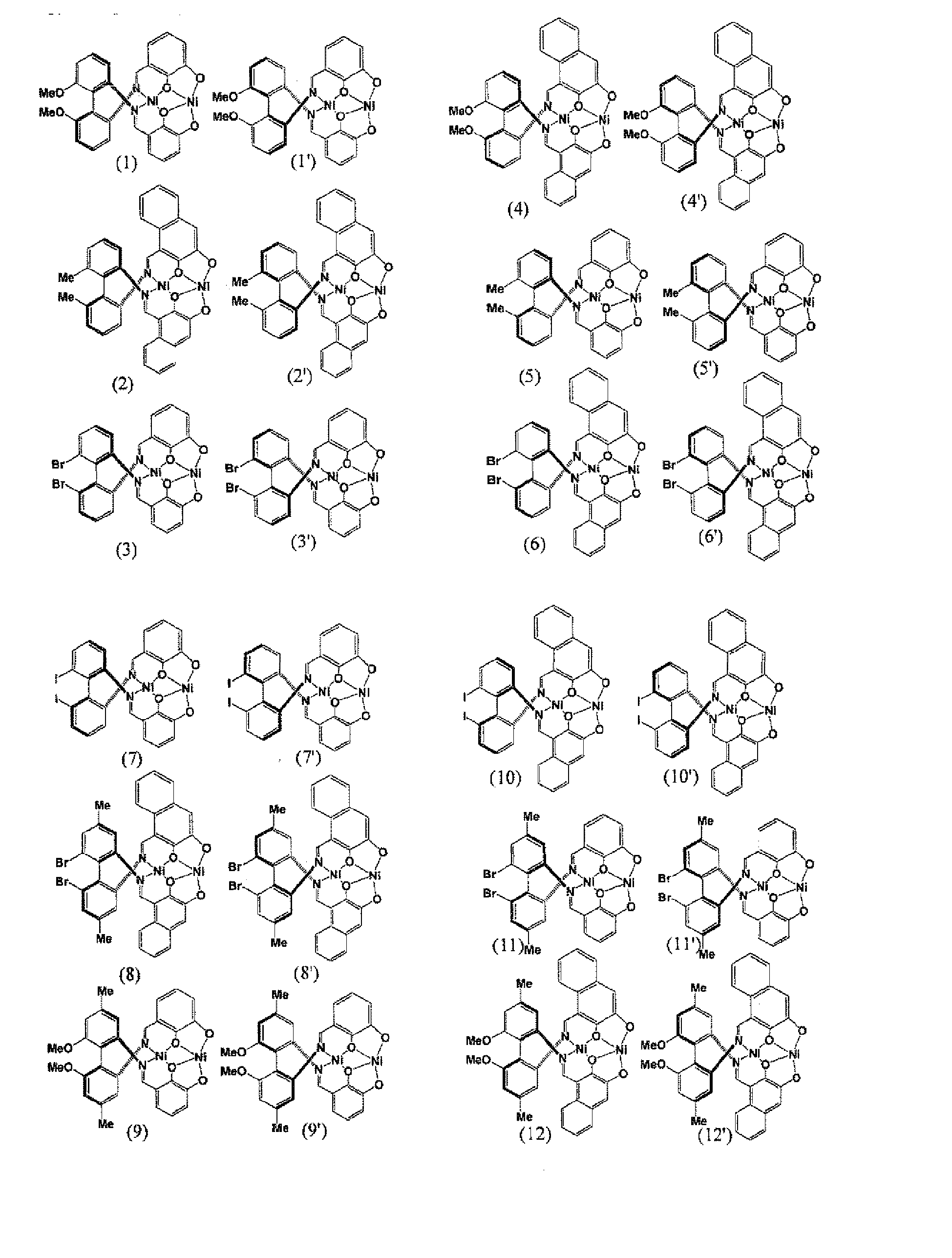

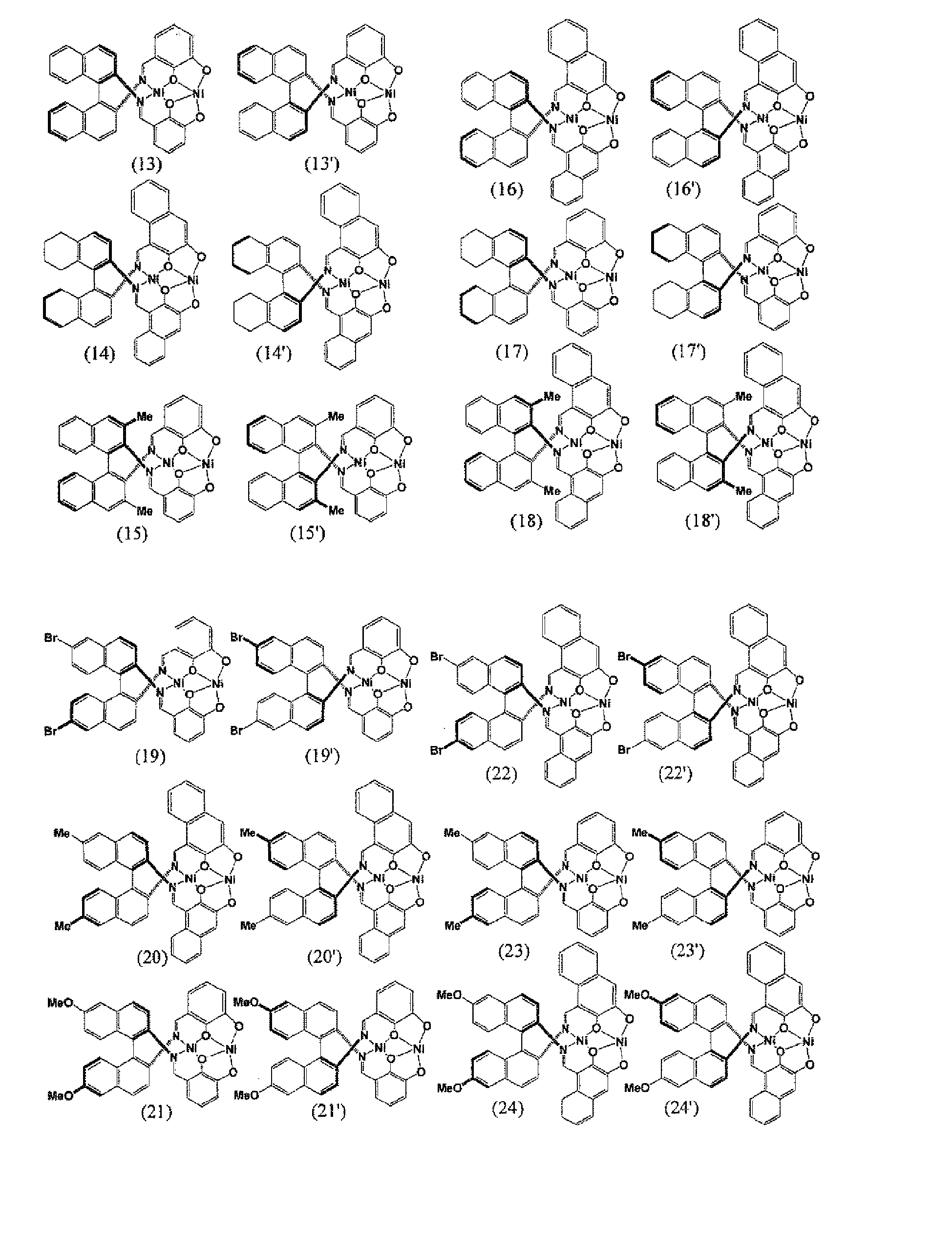

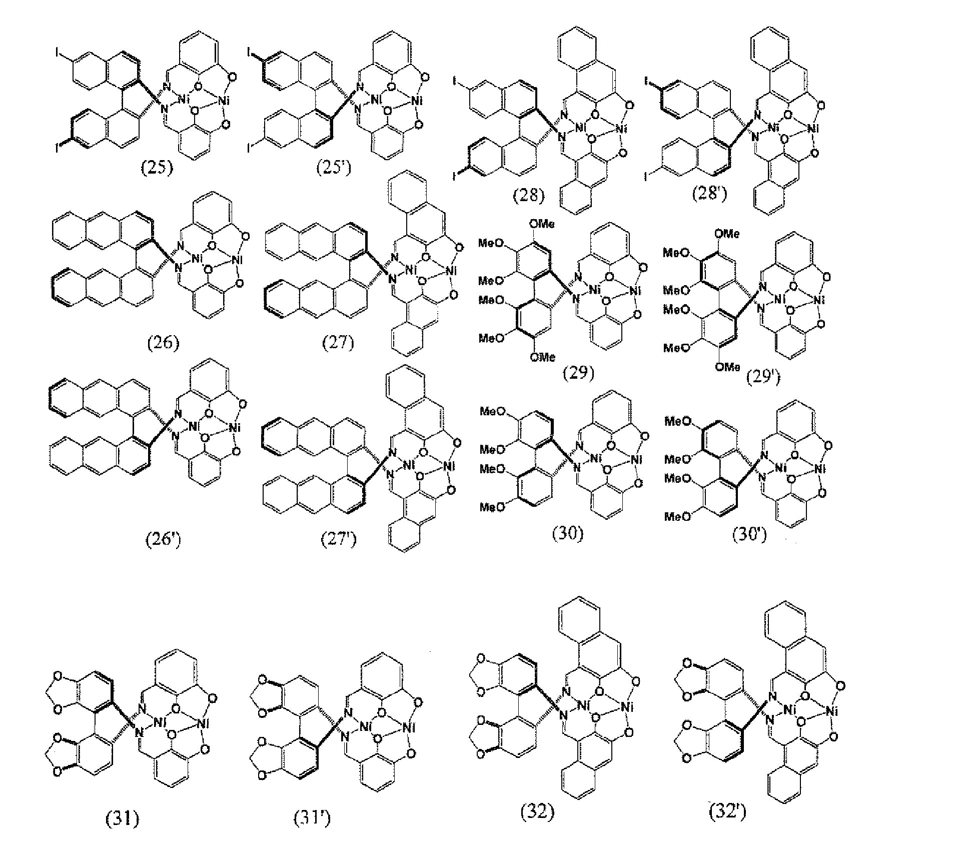

Hereinafter, the synthesis method of the optically active dinickel complex of the present invention is described. As a synthesis method of a salen skeleton that is a ligand, the salen skeleton is synthesized referring to a method described in a literature (Organic Synthesis Collection: Vol. 10, p. 96 (2004); Vol. 75, p. 1 (1998)) or the like. An optically active dinickel complex can be synthesized by adding two equivalents of a nickel source to the salen skeleton in a solvent.

The solvent used in the present reaction is not particularly limited, provided that the solvent is stable and inactive under conditions of the present reaction so as not to inhibit the reaction. Examples of such a solvent include: sulfoxide solvents represented by dimethylsulfoxide; amide solvents represented by dimethylformamide and dimethylacetamide; ether solvents represented by ethyl ether, dimethoxyethane, tetrahydrofuran, dioxane, and cyclopentyl methyl ether; halogen solvents represented by dichloromethane, chloroform, 1,2-dichloroethane, and chlorobenzene; nitrile solvents represented by acetonitrile and propionitrile; aromatic hydrocarbon solvents represented by benzene and toluene; hydrocarbon solvents represented by hexane and heptane; ester solvents represented by ethyl acetate; alcohol solvents represented by methanol, ethanol, 1-propanol, 2-propanol, and ethyleneglycol; and water. The reaction can also be effected under a condition where these solvents are optionally mixed. Preferred examples of the solvent include alcohol solvents represented by methanol, ethanol, 1-propanol, 2-propanol, and ethyleneglycol and more preferred examples thereof include methanol, ethanol, 1-propanol, and 2-propanol.
The reaction temperature is not particularly limited. However, the reaction is effected at a temperature in a range of preferably -40°C to the reflux temperature of a used solvent, more preferably 20°C to the reflux temperature of a used solvent. The present reaction can also be effected under such conditions as under reduced pressure, under normal pressure, or under pressurized pressure and/or under microwave irradiation. The reaction time is not particularly limited and is accordingly selected corresponding to the reaction temperature.

Examples of the nickel source include: nickel (II) carboxylates represented by nickel (II) formate, nickel (II) acetate, nickel (II) octanoate, nickel (II) 2-ethylhexanoate, nickel (II) oxalate, and nickel (II) naphthenate, and hydrates thereof; nickel nitrate, nickel (II) perchlorate, nickel (II) sulfate, nickel (II) phosphate, and hydrates thereof; nickel halides represented by nickel (II) chloride, nickel (II) bromide, and nickel (II) iodide, and hydrates thereof; and nickel (II) hydroxide. Among them, preferred are nickel (II) carboxylates, more preferred are nickel (II) octanoate dihydrate, nickel (II) 2-ethylhexanoate, nickel (II) oxalate dihydrate, nickel (II) acetate, and nickel (II) acetate tetrahydrate, and most preferred are nickel (II) acetate and nickel (II) acetate tetrahydrate.
When the optically active dinickel complex of the present invention is extracted, it exists stably. Therefore, the dinickel complex is desirably used in an extracted state. However, the optically active dinickel complex in the reaction system as it is, that is, in situ, can also be used.

In the method of the present invention, with respect to the equivalent relationship between the substrate (VI) and the substrate (VII) and between the substrate (VI) and the substrate (IX), the amount of the substrate (VII) and the amount of the substrate (IX) are in a range of preferably 0.1 to 10 equivalents, more preferably 0.5 to 2.0 equivalents, relative to 1 equivalent of the substrate (VI).
The used amount of the catalyst is in a range of preferably 0.01 to 100 mol%, more preferably 1 to 20 mol%, relative to 100 mol% of the substrate (VI).

In the present invention, by effecting the reaction in the presence of an additive having a dehydration activity, the reaction time can be reduced. Examples of the dehydrating agent applicable in the present invention include synthetic zeolites such as molecular sieves MS3A, MS4A, and MS5A, magnesium sulfate anhydride, and calcium sulfate anhydride. Among them, preferred are MS3A, MS4A, and calcium sulfate anhydride, and most preferred are MS3A and MS4A. The additive amount of the dehydrating agent may be in a range not hindering the stirring during the reaction, and may be in a range of 0.1 to 1,000 mg, preferably 1 to 500 mg, relative to 1 mmol of the substrate.

The production method of the optically active amine of the present invention is generally performed in an organic solvent. The solvent in the description of the production method according to the present invention is not particularly limited, so long as the solvent is stable and is inactive under conditions of the present invention not inhibiting the reaction. Examples of such a solvent include: sulfoxide solvents represented by dimethylsulfoxide; amide solvents represented by dimethylformamide and dimethylacetamide; ether solvents represented by ethyl ether, dimethoxyethane, tetrahydrofuran, dioxane, and cyclopentyl methyl ether; halogen solvents represented by dichloromethane, chloroform, 1,2-dichloroethane, and chlorobenzene; nitrile solvents represented by acetonitrile and propionitrile; aromatic hydrocarbon solvents represented by benzene and toluene; hydrocarbon solvents represented by hexane and heptane; ester solvents represented by ethyl acetate; and alcohol solvents represented by methanol, ethanol, 1-propanol, 2-propanol, and ethyleneglycol. The reaction can also be effected under a condition where these solvents are optionally mixed.
As the organic solvent, aprotic organic solvents are preferred and specific examples of the aprotic organic solvent include: halogenated hydrocarbons such as dichloromethane (CH₂Cl₂), 1,2-dichloroethane (CH₂ClCH₂Cl), and chlorobenzene; aromatic hydrocarbons such as toluene; esters such as ethyl acetate; and ethers such as tetrahydrofuran (THF).

The reaction temperature for the production method of the optically active amine compound of the present invention is not particularly limited. However, the production method is performed at a temperature in a range of preferably -60°C to the reflux temperature of a used solvent, more preferably -60°C to 40°C. The present reaction can also be effected under such conditions as under reduced pressure, under normal pressure, or under pressurized pressure and/or under microwave irradiation. The reaction time is not particularly limited and is accordingly selected corresponding to the reaction temperature.

In the production method of the present invention, if necessary, by column chromatography, thin-layer chromatography, high performance liquid chromatography (HPLC), high performance liquid chromatography-mass spectrometry (LC/MS), recrystallization, washing with a solvent, or a combination of these purification methods, a high-purity product can also be obtained.

Each structure in Formula (VI) to Formula (X) is described.

R¹² is preferably a C₁₋₁₀ alkyl group, a C₂₋₉ heterocyclyl group, a C₇₋₁₄ aralkyl group, or a C₂₋₁₄ aryl group (where the C₁₋₁₀ alkyl group, the C₂₋₉ heterocyclyl group, the C₇₋₁₄ aralkyl group, and the C₂₋₁₄ aryl group are unsubstituted or substituted with a halogen atom, a C₁₋₆ alkyl group, or a C₁₋₆ alkoxy group), more preferably a C₁₋₁₀ alkyl group, a C₇₋₁₄ aralkyl group, or a C₂₋₁₄ aryl group (where the C₂₋₁₄ aryl group is unsubstituted or substituted with a halogen atom, a C₁₋₆ alkyl group, or a C₁₋₆ alkoxy group).

R¹³ is not particularly limited so long as it has a structure that can stably maintain a N-substituted imine and does not influence the asymmetric Mannich reaction. R¹³ is preferably a protective group for an amino group. Examples of the protective group for an amino group include a protective group described in Protective Groups in Organic Synthesis, Fourth edition, authored by T.W. Greene, John Wiley & Sons Inc. (2006). Preferred examples of the protective group include a C₁₋₁₀ alkyloxycarbonyl group (the C₁₋₁₀ alkyloxycarbonyl group is unsubstituted or substituted with one or more halogen atom(s) that are the same as or different from each other), a C₁₋₁₀ alkenyloxycarbonyl group, and a C₁₋₁₀ aralkyloxycarbonyl group.
More preferred examples thereof include a tert-butoxycarbonyl group (Boc group), a benzyloxycarbonyl group (Cbz group), a 9-fluorenylmethyloxycarbonyl group (Fmoc group), a 2,2,2-trichloroethoxycarbonyl group (Troc group), and an allyloxycarbonyl group (Alloc group). Particularly preferred example thereof include a tert-butoxycarbonyl group (Boc group) and a benzyloxycarbonyl group (Cbz group). More particularly preferred is a tert-butoxycarbonyl group (Boc group).

X is preferably a nitro group, a cyano group, -C(O)R¹⁵, -C(O)OR¹⁵, or - P(O)(OR¹⁵)(OR¹⁶).

Y is preferably -C(O)R¹⁵ or -C(O)OR¹⁵.

Z is preferably a nitro group, a cyano group, -C(O)OR¹⁵, or - P(O)(OR¹⁵)(OR¹⁶).

W is preferably an oxygen atom or -CH₂-.

n is preferably 1 or 2.

R¹⁴ is preferably a C₁₋₁₀ alkyl group or a C₇₋₁₄ aralkyl group (where the C₁₋₁₀ alkyl group and the C₇₋₁₄ aralkyl group are unsubstituted or substituted with a halogen atom, a C₁₋₆ alkyl group, or a C₁₋₆ alkoxy group), more preferably a C₁₋₆ alkyl group or a C₇₋₁₄ aralkyl group, further preferably a C₁₋₃ alkyl group or a benzyl group.

R¹⁵ and R¹⁶ are preferably a C₁₋₁₀ alkyl group (the C₁₋₁₀ alkyl group is unsubstituted or substituted with a halogen atom or a C₁₋₆ alkoxy group), more preferably a C₁₋₁₀ alkyl group, further preferably a C₁₋₆ alkyl group.

R¹⁷, R¹⁸, R¹⁹, R²⁰, R²¹, R²², R²³, R²⁴, and R²⁵ are preferably a hydrogen atom or a C₁₋₁₀ alkyl group, more preferably a hydrogen atom.

G is preferably -CH₂- or an oxygen atom.

m1 and m2 are preferably 1 or 2, where m1+m2 is 3 or 4.

According to the production method of the present invention, only one type of the enantiomers can be produced with a high selectivity. In the production method of the present invention, by selectively using the complex of Formula (I) or Formula (I'), and the complex of Formula (II) or the complex of Formula (II'), both enantiomers of the optically active amine compound can be selectively produced.

### Examples

Hereinafter, Reference Synthesis Examples and Reaction Examples of the present invention will be described, which should not be construed as limiting the scope of the present invention. Here, ¹H-NMR and ¹³C-NMR were measured at 500 MHz and 125.62 MHz, respectively. The ratio of anti/syn was measured and determined by ¹H-NMR of the reaction composition.
In Examples, MS, ESI-MS, HRMS, IR, HPLC, dr, ee, anti, and syn mean mass spectrometry, an electro spray ionization method mass spectrometry method, high resolution mass spectrometry, infrared spectroscopy, high performance liquid chromatography, diastereomer ratio, enantiomer excess, anti form, and syn form, respectively. Numerical value % described in the results of HPLC analysis indicates a surface area percentage unless otherwise defined.

### (Reference Synthesis Example 1)

### Synthesis of optically active ligand (R)-3,3'-(1E, 1E')-(1,1'-binaphthyl-2,2'-diylbis(azane-1-yl-1-ylidene))bis(methane-1-yl-1-ylidene)dibenzene-1,2-diol (33)

Into a reaction vessel, 1.82 g (6.4 mmol) of (R)-1,1'-binaphthyl-2,2'-diamine and 1.95 g (14.1 mmol) of 2,3-dihydroxybenzaldehyde were charged, and to the resultant mixture, 85 mL of ethanol was added at room temperature. The mixed solution was heated to reflux for 12 hours and the disappearance of (R)-binaphthyldiamine was confirmed, followed by cooling down the solution to room temperature and by distilling off ethanol under reduced pressure. To the resultant residue, a mixture of ethanol/hexane=1/2 was added and the resultant mixture was stirred at room temperature for 2 hours to dissolve unreacted aldehyde. The solid in the mixture was filtered off under reduced pressure and was washed with a mixture of ethanol/hexane=1/2 to produce 3.30 g of the objective optically active ligand (R)-3,3'-(1E, 1E')-(1,1'-binaphthyl-2,2-diylbis(azane-1-yl-1-ylidene))bis(methane-1-yl-1-ylidene)dibenzene-1,2-diol (33) as an orange solid (yield: 98%). 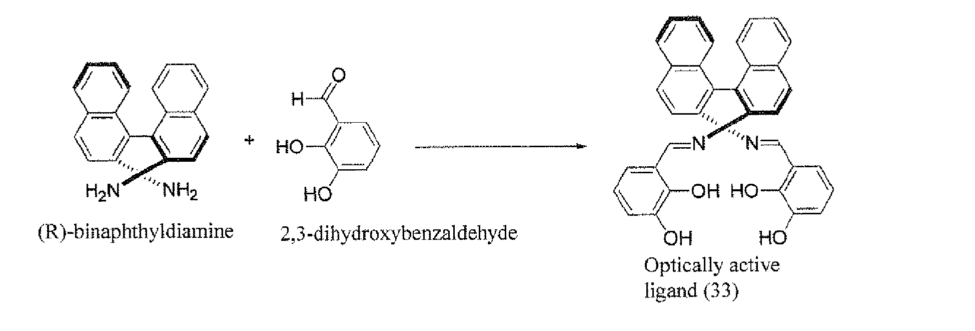 Form orange solid
¹H-NMR (ppm in CDCl₃) δ 5.34 (s, 2H), 6.66 (dd, J=7.6, 7.6 Hz, 2H), 6.77 (dd, J=1.5, 7.6 Hz, 2H), 6.84 (dd, J=1.5, 7.6 Hz, 2H), 7.22-7.32 (m, 4H), 7.44-7.49 (m, 2H), 7.63 (d, J=8.8 Hz, 2H), 7.97 (d, J=7.8 Hz, 2H), 8.09 (d, J=8.8 Hz, 2H), 8.62 (s, 2H), 12.5
(s, 2H)
¹³C-NMR (ppm in CDCl₃) δ 116.7, 117.4, 118.5, 118.6, 122.8, 126.1, 126.3, 127.1, 128.2, 129.4, 130.1, 132.5, 133.1, 143.0, 144.7, 48.7, 161.3
ESI-MS m/z 547[M+Na]⁺.

### (Synthesis Example 1)

### Preparation of optically active dinickel complex (13)

To a solution of 1.049 g (2.0 mmol) of the optically active ligand (R)-3,3'-(1E, 1E')-(1,1'-binaphthyl-2,2'-diylbis(azane-1-yl-1-ylidene))bis(methane-1-yl-1-ylidene)dibenzene-1,2-diol (33) synthesized in Reference Synthesis Example 1 in 10 mL of ethanol, 995.4 mg (4.0 mmol) of nickel acetate tetrahydrate was added at room temperature. The resultant mixture was heated to reflux for 12 hours and was cooled down to room temperature to filter off a deposited precipitation, followed by washing the obtained dark yellow solid with 3 mL of water three times, with 3 mL of ethanol three times, and with 3 mL of diethyl ether. The obtained solid was dried under reduced pressure to produce 1.187 g of the objective optically active dinickel complex (13) as a dark yellow solid (yield: 91%). 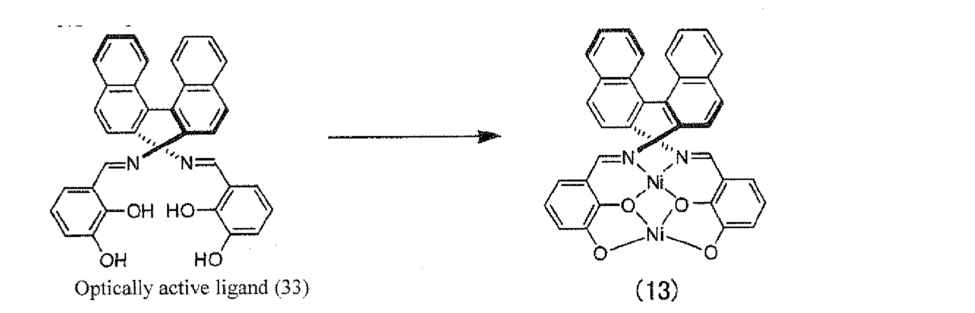 ESI-MS m/z 637[M+H]⁺.

### (Reference Synthesis Example 2)

### Synthesis of optically active ligand (R)-3,3'-(1E, 1E')-(6,6'-dibromo-1,1'-binaphthyl-2,2'-diyl)bis(azane-1-yl-1-ylidene)bis(methane-1-yl-1-ylidene)dibenzene-1,2-diol (52)

Into a reaction vessel, 0.154 g (0.347 mmol) of (R)-6,6'-dibromobinaphthyldiamine and 0.101 g (0.729 mmol) of 2,3-dihydroxybenzaldehyde were charged and, to the resultant mixture, 10 mL of ethanol was added at room temperature. The mixed solution was heated to reflux for 12 hours and was cooled down to room temperature and ethanol was distilled off under reduced pressure. The resultant residue was washed with ethanol/hexane=1/2 and the solid was filtered off under reduced pressure to produce 0.113 g of the objective optically active ligand (R)-3,3'-(1E, 1E')-(6,6'-dibromo-1,1'-binaphthyl-2,2'-diyl)bls(azane-1-yl-1-ylidene)bis(methane-1-yl-1-ylidene)dibenzene-1,2-diol (52) as an orange solid (yield: 48%).
Form orange solid
¹H-NMR (ppm in CDCl₃) δ 5.39 (s, 2H), 6.71 (dd, J=7.8, 7.8 Hz, 2H), 6.80 (dd, J=1.5, 7.8 Hz, 2H), 6.89 (dd, J=1.5, 7.8 Hz, 2H), 7.07 (d, J=9.0 Hz, 2H), 7.37 (dd, J=1.8, 9.0 Hz, 2H), 7.67 (d, J=8.9 Hz, 2H), 8.02 (d, J=8.9 Hz, 2H), 8.14 (d, J=1.8 Hz, 2H), 8.63 (s, 2H), 12.4 (s, 2H)

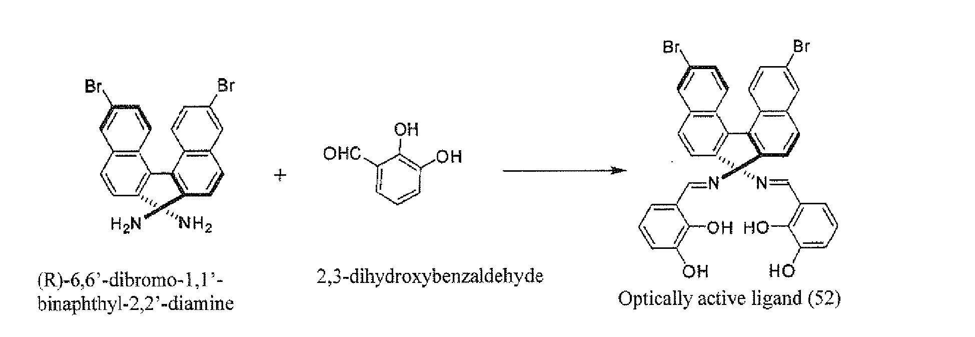

### (Synthesis Example 2)

### Preparation of optically active dinickel complex (19)

To a solution of 83.1 mg (0.122 mmol) of the optically active ligand (R)-3,3'-(1E, 1E')-(6,6'-dibromo-1,1'-binaphthyl-2,2'-diyl)bis(azane-1-yl-1-ylidene))bis(methane-1-yl-1-ylidene)dibenzene-1,2-diol (52) synthesized in Reference Synthesis Example 2 in 5 mL of ethanol, 63.6 mg (0.256 mmol) of nickel acetate tetrahydrate was added at room temperature. The resultant mixture was heated to reflux for 18 hours and was cooled down to room temperature to filter off a deposited precipitation, followed by washing the obtained dark yellow solid with 1 mL of water three times, with 0.5 mL of ethanol three times, and with 1 mL of diethyl ether. The obtained solid was dried under reduced pressure to produce 60.6 mg of the objective optically active dinickel complex catalyst (19) as a dark yellow solid (yield: 62%). ESI-MS m/z 794[M]⁺, 796[M]⁺.

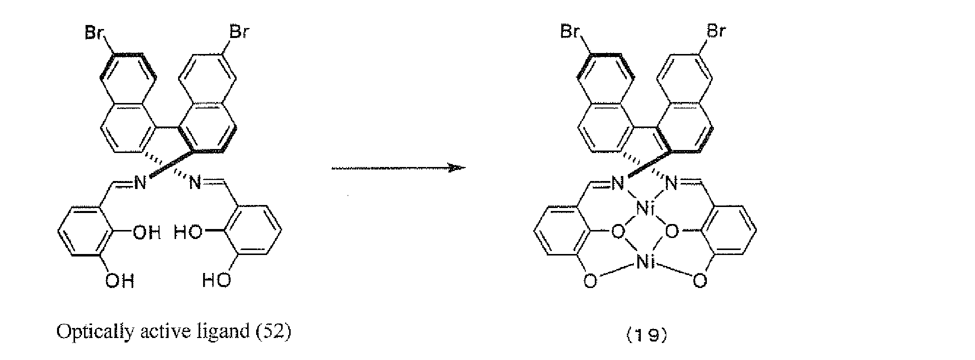

### (Synthesis Example 3)

### Preparation of optically active dinickel complex (17)

Into a reaction vessel, 1.706 g (5.836 mmol) of (R)-octahydrobinaphthyldiamine and 1.773 g (12.83 mmol) of 2,3-dihydroxybenzaldehyde were charged and, to the resultant mixture, 30 mL of ethanol was added at room temperature. The mixed solution was heated to reflux for 12 hours and was cooled down to room temperature and ethanol was distilled off under reduced pressure. 0.325 g of the resultant residue (containing about 0.60 mmol of an optically active ligand (53)) as it was was used in a reaction with nickel acetate tetrahydrate. To a solution of 0.325 g of the residue in 3 mL of ethanol, 298.6 mg (1.20 mmol) of nickel acetate tetrahydrate was added at room temperature. The resultant mixture was heated to reflux for 21 hours and was cooled down to room temperature to filter off a deposited precipitation, followed by washing the obtained dark yellow solid with 1 mL of water three times, with 1 mL of ethanol three times, and with 1 mL of diethyl ether. The obtained solid was dried under reduced pressure to produce 334.8 mg of the objective optically active dinickel complex catalyst (17) as a dark yellow solid (yield: 85%).
Optically active ligand (53)
Form red brown solid
¹H-NMR (CDCl₃) δ 1.66-1.81 (m, 8H), 2.23-2.36 (m, 4H), 2.81-2.94 (m, 4H), 5.63 (s, 2H), 6.71 (dd, J=8.0, 8.0 Hz, 2H), 6.82 (dd, J=1.2, 8.0 Hz, 2H), 6.90 (dd, J=1.2, 8.0 Hz, 2H), 7.14 (d, J=8.1 Hz, 2H), 7.20 (d, J=8.1 Hz, 2H), 8.51 (s, 2H), 12.9 (s, 2H).
Optically active dinickel complex catalyst (17)
ESI-MS m/z 645[M+H]⁺.

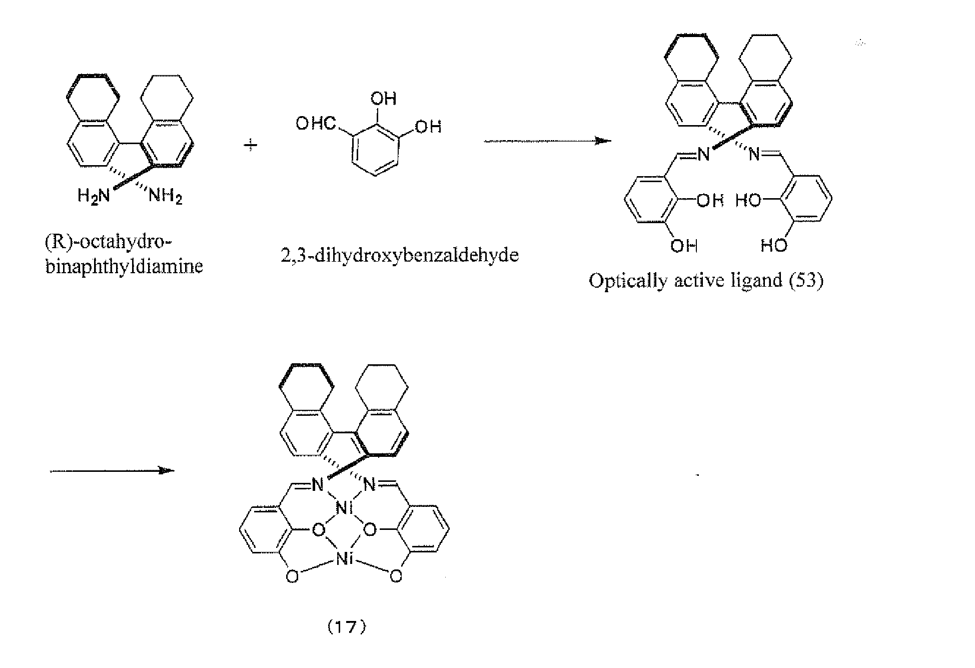

### (Synthesis Example 4)

### Preparation of optically active dinickel complex (5)

Into a reaction vessel, 0.212 g (1.00 mmol) of (R)-6,6'-dimethylbiphenyldiamine and 0.304 g (2.20 mmol) of 2,3-dihydroxybenzaldehyde were charged and, to the resultant mixture, 5 mL of ethanol was added at room temperature. The mixed solution was heated to reflux for 12 hours and was cooled down to room temperature and 398 mg (1.60 mmol) of nickel acetate tetrahydrate was added to the mixed solution at room temperature. The resultant mixture was heated to reflux for 18 hours and was cooled down to room temperature to filter off a deposited precipitation, followed by washing the resultant dark yellow solid with 0.5 mL of ethanol three times and with 1 mL of diethyl ether. The resultant solid was dried under reduced pressure to produce 470.7 mg of the objective optically active dinickel complex catalyst as a dark yellow solid.
ESI-MS m/z 567[M+H]⁺

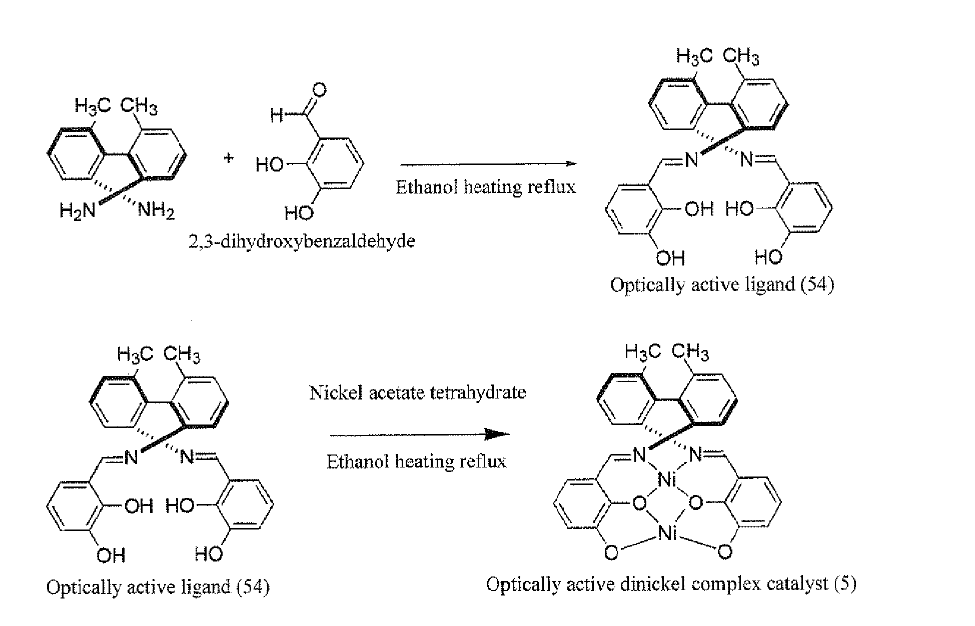

### (Reaction Example 1)

### Synthesis of (2R, 3R)-tert-butyl 3-(tert-butoxycarbonylamino)-2-methyl-2-nitro-3-phenylpropanoic acid ester (36aa)

Into a flask, 75 mg of molecular sieves 4A was charged and it was dried by heating for 5 minutes under reduced pressure and was cooled down to room temperature, followed by filling the inside of the flask with argon. Into the flask, 9.6 mg (0.015 mmol, 5 mol%) of the optically active dinickel complex (13) and 750 µL of dry tetrahydrofuran were added at room temperature, and thereto further, 57.8 mg (0.33 mmol) of tert-butyl 2-nitropropanoic acid ester (35a) was added, followed by cooling down the mixed solution to 0°C. The mixed solution was stirred at 0°C for 15 minutes and thereto, 61.6 mg (0.3 mmol) of tert-butyl benzylidenecarbamate (34a) was added, followed by stirring the resultant mixture at 0°C for further 12 hours. After the completion of the reaction, the reaction solvent was distilled off under reduced pressure and the resultant residue was purified by silica gel column chromatography (eluting with hexane/ethyl acetate=20/1 to 10/1) to produce 108.4 mg of the objective substance as a colorless solid (yield: 95%, anti/syn=91/9, optical purity: 98%ee). 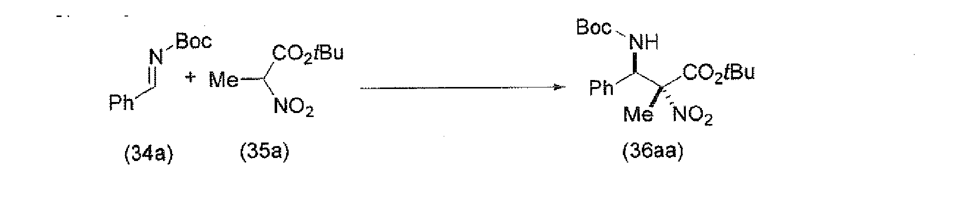 Form colorless solid
IR (KBr) ν 2981, 1724, 1555, 1155 cm⁻¹
¹H-NMR (ppm in CDCl₃) δ 1.38 (s, 9H), 1.50 (s, 9H), 1.68 (s, 3H), 5.35 (d, J=9.6 Hz, 1H), 6.53 (d, J=9.6 Hz, 1H), 7.20-7.32 (m, 5H)
¹³C-NMR (ppm in CDCl₃) δ 22.7, 27.4, 28.1, 59.7, 79.9, 85.0, 94.8, 128.45, 128.49, 128.6, 136.0, 154.6, 164.4
ESI-MS m/z 403[M+Na]⁺
HRMS calcd. for C₁₉H₂₈N₂O₆Na[M+Na]⁺: 403. 1845,
found 403. 1833
[α]_{D}^{22.5} -6.3 (c 1.04, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=9.2 min (R,R, 99.1%) and retention time=5.8 min (S,S, 0.9%).

### (Reaction Example 2)

### Synthesis of (2R, 3R)-tert-butyl 3-(tert-butoxycarbonylamino)-2-methyl-2-nitro-3-phenylpropanoic acid ester (36aa)

The reaction was effected under the same reaction conditions as in Reaction Example 1, except that the amount of the optically active dinickel complex (13) was reduced to 1 mol%, at room temperature for 12 hours to produce 106.1 mg (yield: 93%, anti/syn=88/12, optical purity: 98%ee) of the objective substance as a colorless solid.
The spectrum data corresponded to that of Reaction Example 1.

### (Reaction Example 3)

### Synthesis of (2R, 3R)-tert-butyl 3-(tert-butoxycarbonylamino)-2-methyl-2-nitro-3-phenylpropanoic acid ester (36aa)

Into a flask, 9.6 mg (0.015 mmol, 5 mol%) of the optically active dinickel complex (13) and 750 µL of dry tetrahydrofuran were charged and thereto, further 57.8 mg (0.33 mmol) of tert-butyl 2-nitropropanoic acid ester (35a) was added in an argon atmosphere, followed by cooling down the mixed solution to 0°C The mixed solution was stirred at 0°C for 15 minutes and thereto, 61.6 mg (0.3 mmol) of tert-butyl benzylidenecarbamate (34a) was added, followed by stirring the resultant mixture at 0°C for further 18 hours. After the completion of the reaction, the reaction solvent was distilled off under reduced pressure and the resultant residue was purified by silica gel column chromatography (eluting with hexane/ethyl acetate=20/1 to 10/1) to produce 104.8 mg of the objective substance (yield: 92%, anti/syn=90/10, optical
purity: 98%ee) as a colorless solid.
The spectrum data corresponded to that of Reaction Example 1.

### (Reaction Examples 4 to 14)

### Synthesis of (2R, 3R)-tert-butyl 3-(tert-butoxycarbonylamino)-2-substituted-2-nitro-3-substituted-propanoic acid ester

By the same operation as in Reaction Example 1, the reaction was effected with the substrate, the catalyst amount, and the reaction time as shown in Table 1 and the objective substance was obtained with the yield and the optical purity shown in Table 1.

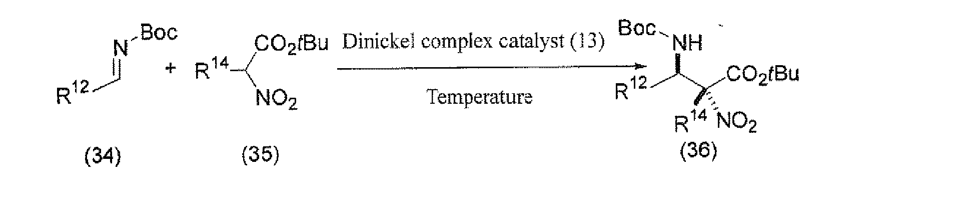

**[Table 1]**

| Example | Imine | | Nitroacetic acid ester | | Catalyst (14) | Temp. | Time | Product | yield | dr | %ee |
|---|---|---|---|---|---|---|---|---|---|---|---|
| | R¹² | | R¹⁴ | | (×mol%) | (°C) | (h) | | (%) | (anti/syn) | (anti) |
| 4 | 4-MeO-C₆H₄- | (34b) | Me | (35a) | 5 | 0 | 12 | (36ba) | 92 | 87:13 | 98 |
| 5 | 4-Me-C₆H₄- | (39c) | Me | (35a) | 5 | 0 | 12 | (36ca) | 90 | 89:11 | 97 |
| 6 | 4-Cl-C₆-H₄- | (34d) | Me | (35a) | 5 | 0 | 12 | (36da) | 87 | 86:14 | 97 |
| 7 | 4-F- C₆H₄- | (34e) | Me | (35a) | 5 | 0 | 12 | (36ea) | 91 | 90:10 | 91 |
| 8 | 3-thienyl | (34f) | Me | (35a) | 5 | 0 | 12 | (36a) | 96 | 91:9 | 99 |
| 9 | Ph- | (34a) | Et | (35b) | 5 | 0 | 12 | (36ab) | 91 | 94:6 | 99 |
| 10 | Ph- | (34a) | n-Pr | (35c) | 5 | 0 | 12 | (36ac) | 92 | 92:8 | >99 |
| 11 | Ph- | (34a) | Bn | (35d) | 5 | 0 | 12 | (36ad) | 94 | 88:12 | 94 |
| 12 | PhCH₂CH₂- | (34g) | Me | (35a) | 10 | -40 | 36 | (36ga) | 73 | >97:3 | 95 |
| 13 | *n*-butyl | (34h) | Me | (35a) | 10 | -40 | 36 | (36ha) | 67 | >97:3 | 93 |
| 14 | *i*-butyl | (34i) | Me | (35a) | 10 | -20 | 24 | (36ia) | 85 | >97:3 | 91 |

The structures of the products 36ba to 36ia are shown as follows.

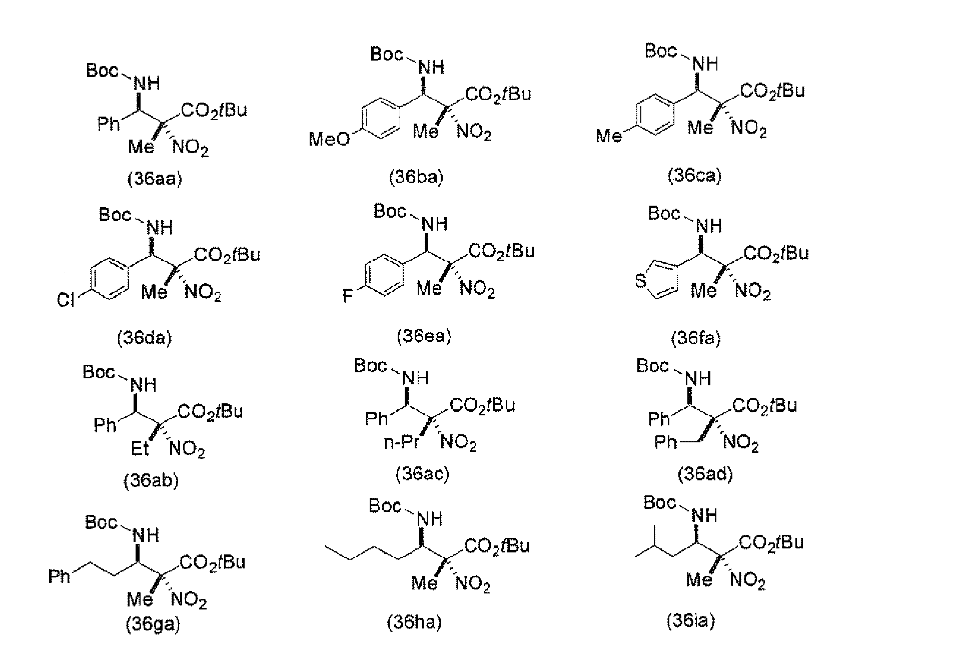

Data of the objective substance of Reaction Example 4 (2R, 3R)-tert-butyl 3-(tert-butoxycarbonylamino)-3-(4-methoxyphenyl)-2-methyl-2-nitro-propanoic acid ester (36ba)
Form colorless solid
1R (KBr) ν 2984, 1726, 1560 cm⁻¹
¹H-NMR (ppm in CDCl₃) δ 1.36 (s, 9H), 1.46 (s, 9H), 1.63 (s, 3H), 3.80 (s, 3H), 5.40 (brs, 1H), 6.39 (brs, 1H), 6.81-6.85 (m, 2H), 7.23-7.27 (m, 2H)
¹³C-NMR (ppm in CDCl₃) δ 22.6, 27.4, 28.1, 55.1, 59.2, 79.8, 84.9, 94.9, 113.7, 127.9, 129.7, 154.5, 1.59.5, 164.4
ESI-MS m/z 433[M+Na]⁺
HRMS calcd. for C₂₀H₃₀N₂O₇Na[M+Na]⁺: 433. 1951, found 433. 963
[α]_{D}^{23.6} -11.4 (c 1.05, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=12.7 min (R,R, 98.8%) and 7.1 min (S,S, 1.2%).

Data of the objective substance of Reaction Example 5
(2R, 3R)-tert-butyl 3-(tert-butoxycarbonylamino)-2-methyl-3-(4-methylphenyl)-2-nitro-propanoic acid ester (36ca)
Form colorless solid
IR (neat) ν 2979, 1722, 1557 cm⁻¹
¹H-NMR (ppm in CDCl₃) δ 1.35 (s, 9H), 1.46 (s, 9H), 1.63 (s, 3H), 2.31 (s, 3H), 5.42 (d, J=9.8 Hz, 1H), 6.42 (brs, 1H), 7.08-7.15 (m, 2H), 7.18-7.23 (m, 2H)
¹³C-NMR (ppm in CDCl₃) δ 21.0, 22.8, 27.5, 28.2, 59.5, 79.9, 85.0, 94.9, 128.5, 129.2, 133.0, 138.3, 154.6, 164.5
ESI-MS m/z 417[M+Na]⁺
HRMS calcd. for C₂₀H₃₀N₂O₆Na[M+Na]⁺: 417.2002, found 417.2006
[α]_{D}^{26.0} -9.72 (c 1.23, CHCl₃)
HPLC (Daicel Chemical Industries, Ltd., CHIRALPAK (registered trademark) AD-H, hexane/2-propanol=90/10, flow rate 1 mL/min, detection UV 254 mn), retention time=8.2 min (R,R, 98.4%) and retention time=5.3 min (S,S, 1.6%)

### Data of the objective substance of Reaction Example 6

(2R, 3R)-tert-butyl 3-(tert-butoxycarbonylamino)-3-(4-chlorophenyl)-2-methyl-2-nitro-propanoic acid ester (36da)
Form colorless foam
IR (KBr) ν 2981, 1720, 1559, 1163 cm⁻¹
¹H-NMR (ppm in CDCl₃) δ 1.35 (s, 9H), 1.46 (s, 9H), 1.63 (s, 3H), 5.41 (brd, J=7.0 Hz, 1H), 6.42 (brs, 1H), 7.26-7.29 (m, 4H)
¹³C-NMR (ppm in CDCl₃) δ 22.6, 27.5, 28.1, 59.2, 80.3, 85.4, 94.6, 128.7, 130.0, 134.5, 134.7, 154.6, 164.3
ESI-MS m/z 437[M+Na]⁺
[α]_{D}^{25.9} -10.4 (c 1.09, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=14.3 min (R,R) and retention time=7.7 min (S,S).

### Data of the objective substance of Reaction Example 7

(2R, 3R)-tert-butyl 3-(tert-butoxycarbonylamino)-3-(4-fluorophenyl)-2-methyl-2-nitro-propanoic acid ester (36ea)
Form colorless oily substance
IR (neat) ν 2980, 1723, 1557 cm⁻¹
¹H-NMR (ppm in CDCl₃) δ 1.36 (s, 9H), 1.45 (s, 9H), 1.64 (s, 3H), 5.42 (brs, 1H), 6.40 (brs, 1H), 6.98-7.105 (m, 2H), 7.31-7.36 (m, 2H)
¹³C-NMR (ppm in CDCl₃) δ 22.7, 27.5, 28.2, 59.2, 80.3, 85.3, 94.8, 115.4 (d, J=20.9 Hz), 130.5 (d, J=8.3 Hz), 132.0, 154.6, 162.6 (d, J=248 Hz), 164.4
ESI-MS m/z 421[M+Na]⁺
HRMS calcd. for C₁₉H₂₇CFN₂O₆Na[M+Na]⁺: 421. 1751, found 421. 1744 [α]_{D}^{26.0} -3.33 (c 1.22, CHCl₃)
HPLC (Daicel Chemical Industries, Ltd., CHIRALPAK (registered trademark) AD-H, hexane/2-propanol=90/10, flow rate 0.3 mL/min, detection UV 254 nm), retention time=39.3 min (R,R, 95.1%) and retention time=20.5 min (S,S, 4.9%)

Data of the objective substance of Reaction Example 8
(2R, 3R)-tert-butyl 3-(tert-butoxycarbonylamino)-2-methyl-2-nitro-3-(3-thienyl)propanoic acid ester (36fa)
Form colorless solid
IR(KBr) ν 2981, 1754, 1677, 1557 cm⁻¹
¹H-NMR (ppm in CDCl₃) δ 1.38 (s, 9H), 1.44 (s, 9H), 1.70 (s, 3H), 5.59 (d, J=9.4 Hz, 1H), 6.15 (d, J=9.4 Hz, 1H), 7.04-7.08 (m, 1H), 7.25-7.29 (m, 2H)
¹³C-NMR (ppm in CDCl₃) δ 22.4, 27.4, 28.2, 55.5, 80.2, 84.9, 95.0, 124.8, 126.0, 127.5, 136.8, 154.7, 164.6
ESI-MS m/z 409[M+Na]⁺
HRMS calcd. for C₁₇H₂₆N₂O₆SNa[M+Na]⁺: 409. 1409, found 409. 1406
[α]_{D}^{23.1} -36.3 (c 1.05, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=10.6 min (R,R, 99.7%) and retention time=6.5 min (S,S, 0.3%).

Data of the objective substance of Reaction Example 9
(2R, 3R)-tert-butyl 3-(tert-butoxycarbonylamino)-2-ethyl-2-nitro-3-phenylpropanoic acid ester (36ab)
Form colorless solid
IR (KBr) ν 2978, 717, 1557 cm^{-1 1}H-NMR (ppm in CDCl₃) δ 0.95 (dd, J=7.4, 7.4 Hz, 3H), 1.36 (s, 9H), 1.46 (s, 9H), 1.89-2.05 (m, 2H), 5.50 (d, J=8.8 Hz, 1H), 6.52 (d, J=8.8 Hz, 1H), 7.27-7.32 (m, 5H) ¹³C-NMR (ppm in CDCl₃) δ 8.8, 27.6, 28.2, 29.4, 58.7, 79.9, 85.3, 98.3, 128.2, 128.5, 128.6, 136.2, 154.6, 164.3
ESI-MS m/z 417[M+Na]⁺
HRMS calcd. for C₂₀H₃₀N₂O₆Na[M+Na]⁺: 417. 2002, found 417. 2002
[α]_{D}^{25.9} +5.54 (c 1.25, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=5.7 min (R,R, 99.8%) and retention time=4.9 min (S,S, 0.2%).

### Data of the objective substance of Reaction Example 10

(2R, 3R)-tert-butyl 3-(tert-butoxycarbonylamino)-2-nitro-3-phenyl-2-propylpropanoic acid ester (36ac)
Form colorless oily substance
IR (neat) ν 2976, 1723, 1557 cm⁻¹
¹H-NMR (ppm in CDCl₃) δ 0.84 (dd, J=6.9, 7.5 Hz, 3H), 1.31-1.39 (m, 2H), 1.35 (s, 9H), 1.45 (s, 9H), 1.78-1.90 (m, 2H), 5.50 (d, J=9.4 Hz, 1H), 6.51 (d, J=9.4 Hz, 1H), 7.24-7.32 (m, 5H)
¹³C-NMR (ppm in CDCl₃) δ 13.8, 17.6, 27.5, 28.2, 37.8, 58.8, 79.9, 85.2, 97.8, 128.2, 128.5, 128.6, 136.1, 154.6, 164.4
ESI-MS m/z 431[M+Na]⁺
HRMS calcd. for C₂₁H₃₂N₂O₆Na[M+Na]⁺: 431. 2158, found 431. 2162
[α]_{D}^{21.0} +11.6 (c 0.950, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=5.1 min (main component, 99.9%) and retention time=4.6 min (sub component, 0.1 %).

### Data of the objective substance of Reaction Example 11

(2R, 3R)-tert-butyl 3-(tert-butoxycarbonylamino)-2-nitro-2-phenylmethyl-3-phenylpropanoic acid ester (36ad)
Form colorless foam
IR(KBr) ν 2980, 1721, 1558 cm^{-1 1}H-NMR (ppm in CDCl₃) δ 1.38 (s, 9H), 1.41 (s, 9H), 3.31 (d, J=14.3 Hz, 1H), 3.39 (d, J=14.3 Hz, 1H), 5.62 (brs, 1H), 6.34 (brs, 1H), 7.10-7.16 (m, 2H), 7.21-7.26 (m, 3H), 7.32-7.38 (m, 5H)
¹³C-NMR (ppm in CDCl₃) δ 27.6, 28.2, 41.3, 59.3, 80.1, 85.9, 98.8, 127.8, 128.2, 128.5, 128.6, 128.7, 130.2, 133.3, 136.2, 154.5, 163.9
ESI-MS m/z 479[M+Na]⁺
HRMS calcd. for C₂₅H₃₂N₂O₆Na[M+Na]⁺: 479. 2158, found 479.2158
[α]_{D}^{21.4} +32.5 (c 0.985, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=7.7 min (R,R, 97.1%) and retention time=5.6 min (S,S, 2.9%).

### Data of the objective substance of Reaction Example 12

(2R, ³R)-tert-butyl 3-(tert-butoxycarbonylamino)-2-methyl-2-nitro-5-phenylpentanoic acid ester (36ga)
Form colorless oily substance
IR (neat) ν 2979, 1715, 1556 cm⁻¹
¹H-NMR (ppm in CDCl₃) δ 1.42 (s, 9H), 1.43 (s, 9H), 1.74 (s, 3H), 1.81-1.97 (m, 2H), 2.62 (ddd, J=6.9, 9.7, 14.0 Hz, 1H), 2.75 (ddd, J=5.7, 9.7, 14.0 Hz, 1H), 4.34 (ddd, J=2.3, 10.9, 10.9 Hz, 1H), 4.94 (d, J=10.9 Hz, 1H), 7.15-7.20 (m, 3H), 7.25-7.30
(m, 2H)
¹³C-NMR (ppm in CDCl₃) δ 21.4, 27.5, 28.2, 32.8, 33.8, 55.0, 80.1, 84.7, 96.0, 126.1, 128.46, 128.47, 140.9, 155.6, 164.9
ESI-MS m/z 431[M+Na]⁺
HRMS calcd. for C₂₁H₃₂N₂O₆Na[M+Na]⁺: 431. 2158, found 431. 2158
[α]_{D}^{25.9} +19.3 (c 1.11, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=8.7 min (R,R, 97.7%) and retention time=5.3 min (S,S, 2.3%).

### Data of the objective substance of Reaction Example 13

(2R, 3R)-tert-butyl 3-(tert-butoxycarbonylamino)-2-methyl-2-nitro-heptanoic acid ester (36ha)
Form colorless solid
IR(KBr) ν 2983, 1745, 1715, 1547, 1503 cm⁻¹
¹H-NMR (ppm in CDCl₃) δ 0.87 (t, J=7.0 Hz, 3H), 1.23-1.64 (m, 6H), 1.41 (s, 9H), 1.48 (s, 9H), 1.77 (s, 3H), 4.26 (ddd, J=2.1, 10.7, 10.7 Hz, 1H), 4.79 (d, J=10.7 Hz, 1H)
¹³C-NMR (ppm in CDCl₃) δ 13.9, 21.3, 22.1, 27.6, 28.2, 28.6, 31.2, 55.2, 79.8, 84.5, 96.0, 155.6, 165.0
ESI-MS m/z 383[M+Na]⁺
HRMS calcd. for C₁₇H₃₂N₂O₆Na[M+Na]⁺: 383. 2158, found 383. 2160
[α]_{D}^{24.0} +23.7 (c 1.05, CHCl₃)
HPLC (Daicel Chemical Industries, Ltd., CHIRALPAK (registered trademark) AD-H, hexane/2-propanol=90/10, flow rate 0.5 mL/min, detection UV 220 nm), retention time=15.5 min (R,R, 95.9%) and retention time=10.2 min (S,S, 4.1%).

### Data of the objective substance of Reaction Example 14

(2R, 3R)-tert-butyl 3-(tert-butoxycarbonylamino)-2,5-dimethyl-2-nitro-hexanoic acid ester (36ia)
Form colorless oily substance
IR (neat) ν 2977, 1716, 1557 cm⁻¹
¹H-NMR (ppm in CDCl₃) δ 0.92 (d, J=6.8 Hz, 3H), 0.95 (d, J=6.4 Hz, 3H), 1.31-1.41 (m, 2H), 1.40 (s, 9H), 1.48 (s, 9H), 1.63-1.69 (m, 1H), 1.77 (s, 3H), 4.35 (ddd, J=2.1, 10.5, 10.5 Hz, 1H), 4.76 (d, J=10.5 Hz, 1H)
¹³C-NMR (ppm in CDCl₃) δ 21.3, 21.4, 23.6, 25.3, 27.6, 28.2, 40.7, 53.6, 79.9, 84.5, 96.3, 155.5, 165.0
ESI-MS m/z 383[M+Na]⁺
HRMS calcd. for C₁₇H₃₂N₂O₆Na[M+Na]⁺: 383. 2158, found 383.2158
[α]_{D}^{25.9} +25.9 (c 1.11, CHCl₃)
HPLC (Daicel Chemical Industries, Ltd., CHIRALPAK (registered trademark) AD-H, hexane/2-propanol=90/10, flow rate 0.5 mL/min, detection UV 254 nm), retention time=15.7 min (R,R, 95.6%) and retention time=10.4 min (S,S, 4.4%).

### (Reaction Example 15)

### Synthesis of methyl (3R)-3-(tert-butoxycarbonylamino)-2-methoxycarbonyl-2-methyl-3-phenylpropanoic acid ester (38)

Into a flask, 4.8 mg (7.5 µmol, 2.5 mol%) of the optically active dinickel complex (13) and 750 µL of dry tetrahydrofuran were charged at room temperature and thereto, further 48.2 mg (0.33 mmol) of dimethyl 2-methylmalonic acid ester (37) was added, followed by stirring the mixed solution at room temperature for 15 minutes. Thereto, further 61.6 mg (0.3 mmol) of tert-butyl benzylidenecarbamate (34a) was added and the resultant mixture was stirred at room temperature for further 12 hours. After the completion of the reaction, the reaction solvent was distilled off under reduced pressure and the resultant residue was purified by silica gel column chromatography (eluting with hexane/ethyl acetate=10/1 to 5/1) to produce 101.2 mg of the objective substance (38) (yield: 96%, optical purity: 96%ee). 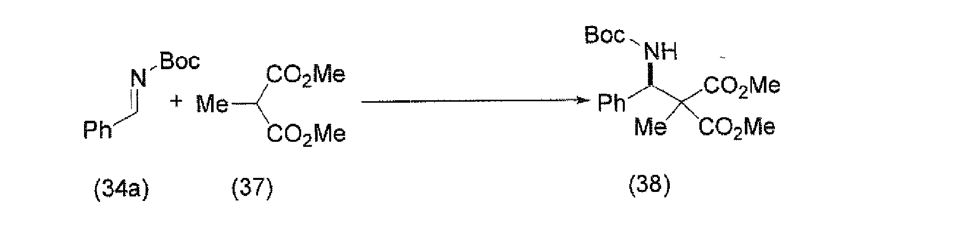 Form colorless solid
IR (KBr) ν 3001, 2953, 1733, 1698, 1507, 1237 cm⁻¹
¹H-NMR (ppm in CDCl₃) δ 1.36 (s, 9H), 1.59 (s, 3H), 3.68 (s, 3H), 3.75 (s, 3H), 5.13 (d, J=9.7 Hz, 1H), 6.46 (d, J=9.7 Hz, 1H), 7.23-7.29 (m, 5H)
¹³C-NMR (ppm in CDCl₃) δ 20.2, 28.2, 52.4, 52.8, 59.0, 59.0, 79.4, 127.8, 128.1, 128.2, 137.8, 1.54.7, 171.0, 171.6
ESI-MS m/z 374[M+Na]⁺
HRMS calcd. for C₁₈H₂₅NO₆Na[M+Na]⁺: 374. 1574, found 374. 1580
[α]_{D}^{21.2} -5.46 (c 1.04, CHCl₃)
HPLC (Daicel Chemical Industries, Ltd., CHIRALPAK (registered trademark) AD-H, hexane/2-propanol=90/10, flow rate 1 mL/min, detection UV 254 nm), retention time=8.6 min (R, 98.1 %) and retention time=7.7 min (S, 1.9%).

### (Reaction Example 16)

### Synthesis of methyl (3R)-3-(tert-butoxycarbonylamino)-2-methoxycarbonyl-3-phenylpropanoic acid ester (40)

The reaction was effected in the same manner as in Reaction Example 15, except that the reaction substrate was dimethyl malonic acid ester (39), to produce 84.2 mg (yield: 83%, optical purity: 96%ee) of the objective substance. 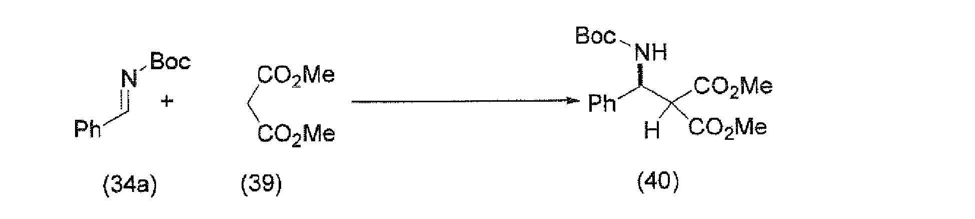 Form colorless solid
The spectrum data corresponded well to a data known in a literature (J. Chem. Commun. 2006, 1191).
HPLC (Daicel Chemical Industries, Ltd., CHIRALPAK (registered trademark) AD-H, hexane/2-propanol=90/10, flow rate 1 mL/min, detection UV 254 nm), retention time=23.3 min (main component, 98.3%) and retention time-31.9 min (sub component, 1.7%).

### (Reaction Example 17)

### Synthesis of optically active methyl 1-((tert-butoxycarbonylamino)(phenyl)methyl)-2-oxocyclopentanecarboxylic acid ester (42)

Into a flask, 3.2 mg (5.0 µmol, 2.5 mol%) of the optically active dinickel complex (13) and 750 µL of dry tetrahydrofuran were charged at room temperature and thereto, further 31.2 mg (0.22 mmol) of methyl 2-oxocyclopentanecarboxylic acid ester (41) was added, followed by stirring the mixed solution at room temperature for 15 minutes. Thereto, further 41 mg (0.2 mmol) of tert-butyl benzylidenecarbamate (34a) was added and the resultant mixture was stirred at room temperature for further 12 hours. After the completion of the reaction, the reaction solvent was distilled off under reduced pressure and the resultant residue was purified by silica gel column chromatography (eluting with hexane/ethyl acetate=10/1 to 5/1) to produce 62.1 mg of the objective substance (yield: 92%, optical purity: 99%ee). 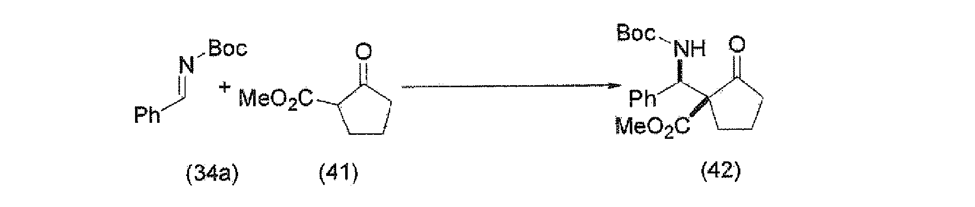 Form colorless solid
The spectrum data corresponded well to a data known in a literature. HPLC (Daicel Chemical Industries, Ltd., CHIRALPAK (registered trademark) AD-H, hexane/2-propanol=90/10, flow rate 1 mL/min, detection UV 254 nm), retention time=20.0 min (main component, >99.8%) and retention time=58.4 min (sub component, <0.2%).

### (Reaction Example 18)

### Synthesis of optically active methyl 1-((tert-butoxycarbonylamino)(furan-2-yl)methyl)-2-oxocyclopentanecarboxylic acid ester (43)

The reaction was effected in the same manner as in Reaction Example 15, except that the reaction substrate was tert-butyl furan-2-ylmethylenecarbamate (34j) to produce 55.3 mg (yield: 82%, optical purity: 91 %ee) of the objective substance. 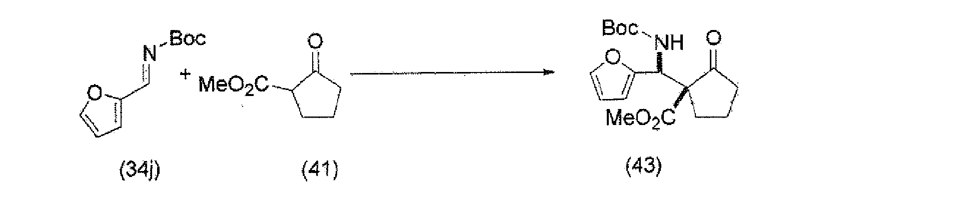 Form colorless oily substance
The spectrum data corresponded well to a data known in a literature (J. Chem. Commun. 2006, 1191).
HPLC (Daicel Chemical Industries, Ltd., CHIRALPAK (registered trademark) AD-H, hexane/2-propanol=90/10, flow rate 1 mL/min, detection UV 254 nm), retention time=13.4 min (main component, 95.7%) and retention time=7.6 min (sub component, 4.3%).

### (Reaction Example 19)

### Synthesis of optically active tert-butyl α-[1-(diethoxyphosphoryl)-2-oxocyclopentyl]benzylcarbamate (45)

Into a flask, 25.7 mg (40 µmol, 10 mol%) of the optically active dinickel complex (13) was charged and the flask was cooled down to 0°C. Into the flask, further 500 µL of dry toluene and 96.9 mg (0.44 mmol) of diethyl 2-oxocyclopentyl phosphonic acid ester (44) were added, and the mixed solution was stirred at 0°C for 15 minutes. Thereto, further 82.1 mg (0.4 mmol) of tert-butyl benzylidene carbamate (34a) was added and the resultant mixture was stirred at 0°C for further 48 hours. After the completion of the reaction, 70 mg of silica gel was charged into the flask and the resultant mixture was stirred at room temperature for 10 minutes. The mixture was filtered and the silica gel was washed with ethyl acetate to combine the filtrate and the washings, followed by distilling off the solvent under reduced pressure. The resultant residue was purified by silica gel column chromatography (eluting with hexane/ethyl acetate=2/1) to produce 139.8 mg of the objective substance (yield: 83%, optical purity: 96%ee). 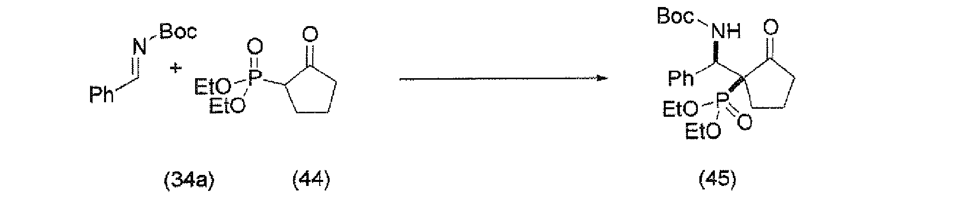 Form colorless solid
ESI-MS m/z 448[M+Na]⁺
HPLC (Daicel Chemical Industries, Ltd., CHIRALPAK (registered trademark) AD-H, hexane/2-propanol=90/10, flow rate 1 mL/min, detection UV 254 nm), retention time=58.9 min (main component, 98.0%) and retention time=2.0 min (sub component, 1.9%).

### (Reaction Example 20)

### Synthesis of optically active tert-butyl (2-acetyl-2-methyl-3-oxo-1-phenylbutyl)carbamic acid ester (47)

Into a flask, 6.4 mg (10 µmol, 10 mol%) of the optically active dinickel complex (13) and 500 µL of dry tetrahydrofuran were charged and the flask was cooled down to -40°C. Into the flask, further 14.7 mg (0.129 mmol) of 3-methylpentane-2,4-dione (46) was added and the mixed solution was stirred at -40°C for 15 minutes. Thereto, 20.5 mg (0.1 mmol) of tert-butyl benzylidene carbamate (34a) was added and the resultant mixture was stirred at -40°C for 24 hours. After the completion of the reaction, 50 mg of silica gel was charged into the flask and the resultant mixture was stirred at room temperature for 10 minutes. The mixture was filtered and the silica gel was washed with diethyl ether to combine the filtrate and the washings, followed by distilling off the solvent under reduced pressure. The yield was determined by analyzing the obtained crude product by NMR (objective substance: 86%). A portion of the obtained crude product was purified by thin-layer chromatography and the optical purity thereof was determined by HPLC (83%ee). 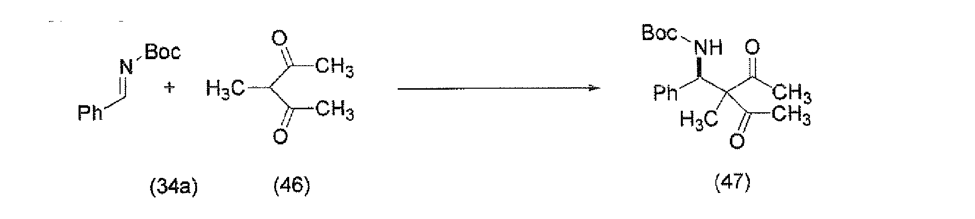 Form colorless solid
ESI-MS m/z 342[M+Na]⁺
HPLC (Daicel Chemical Industries, Ltd., CHIRALPAK (registered trademark) AD-H, hexane/2-propanol=90/10, flow rate 1 mL/min, detection UV 254 nm), retention time=10.1 min (main component, 91.4%) and retention time=6.8 min (sub component, 8.6%).

### (Reaction Example 21)

### Synthesis of optically active ethyl 2-acetyl-3-(tert-butoxycarbonylamino)-2-methyl-3-phenylpropanoic acid ester (49)

Into a flask, 1.5 mg (2.5 µmol, 2.5 mol%) of the optically active dinickel complex (13) and 500 µL of dry tetrahydrofuran were charged and thereto, further 17.8 mg (0.124 mmol) of ethyl 2-methyl-3-oxobutanoic acid ester (48) was added at 25°C, followed by stirring the mixed solution at 25°C for 15 minutes. Thereto, 20.5 mg (0.1 mmol) oftert-butyl benzylidenecarbamate (34a) was added and the resultant mixture was stirred at 25°C for 1.5 hours. After the completion of the reaction, 50 mg of silica gel was charged into the flask and the resultant mixture was stirred at room temperature for 10 minutes. The mixture was filtered and the silica gel was washed with diethyl ether to combine the filtrate and the washings, followed by distilling off the solvent under reduced pressure. The yield was determined by analyzing the obtained crude product by NMR (objective substance: 99%, diastereomer ratio: 42:1). A portion of the obtained crude product was purified by thin-layer chromatography and the optical purity thereof was determined by HPLC (99%ee). 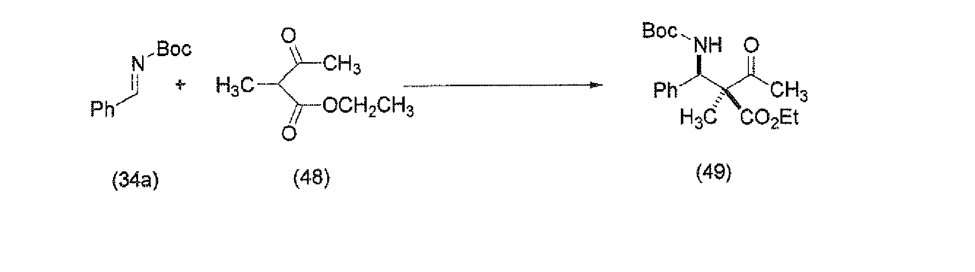 Form colorless solid
ESI-MS m/z 372[M+Na]⁺
HPLC (Daicel Chemical Industries, Ltd., CHIRALPAK (registered trademark) AD-H, hexane/2-propanol=90/10, flow rate 1 mL/min, detection UV 254 nm), retention time=17.1 min (main component, 99.4%) and retention time=11.9 min (sub component, 0.6%).

### (Reaction Example 22)

### Synthesis of optically active tert-butyl (3-acetyl-2-oxo-tetrahydrofuran-3-yl)(phenyl-)methylcarbamic acid ester (51)

Into a flask, 3.2 mg (5 µmol, 5 mol%) of the optically active dinickel complex (13) and 500 µL of dry tetrahydrofuran were charged and the flask was cooled down to -40°C. Into the flask, further 14.9 mg (0.116 mmol) of 3-acetyldihydrofuran-2(3H)-one (50) was added and the mixed solution was stirred at -40°C for 15 minutes. Thereto, 20.5 mg (0.1 mmol) of tert-butyl benzylidenecarbamate (34a) was added and the resultant mixture was stirred at -40°C for 12 hours. After the completion of the reaction, 50 mg of silica gel was charged into the flask and the resultant mixture was stirred at room temperature for 10 minutes. The mixture was filtered and the silica gel was washed with diethyl ether to combine the filtrate and the washings, followed by distilling off the solvent under reduced pressure. The yield was determined by analyzing the obtained crude product by NMR (objective substance: 91%, diastereomer ratio: 8:1). A portion of the obtained crude product was purified by thin-layer chromatography and the optical purity thereof was determined by HPLC (92%ee). 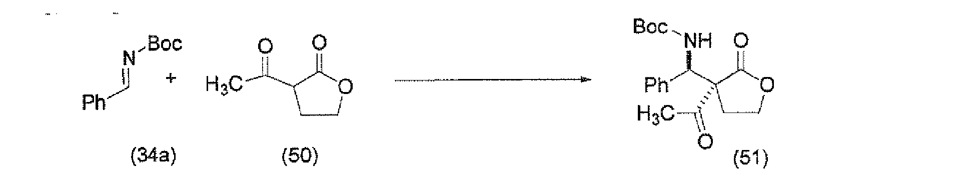 Form colorless solid
ESI-MS m/z 356[M+Na]⁺
HPLC (Daicel Chemical Industries, Ltd., CHIRALPAK (registered trademark) AD-H, hexane/2-propanol=90/10, flow rate 1 mL/min, detection UV 254 nm), retention time=36.2 min (main component, 95.9%) and retention time=12.1 min (sub component, 4.1%).

### INDUSTRIAL APPLICABILITY

The present invention provides a novel optically active dinickel complex that is stable in air for a long period and is easily handled, and the present invention further provides a novel production method of an optically active amine exhibiting high diastereoselectivity and high enantioselectivity by an asymmetric Mannich reaction using the complex of the present invention as a catalyst.

## Claims

1. An optically active dinickel complex of Formula (I) or Formula (I'): 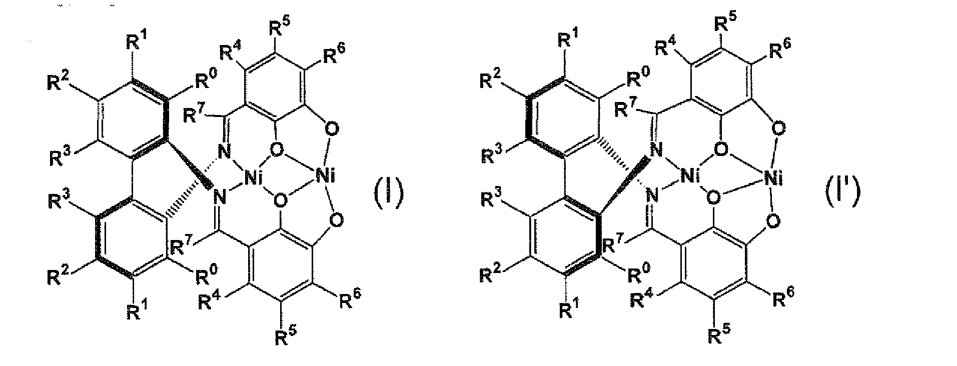 [where R⁰, R¹, R², R³, R⁴, R⁵, R⁶, and R⁷ are each independently a hydrogen atom, a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group,
a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, the C₂₋₉ heterocyclyl group, the C₂₋₁₄ aryl group, and the C₂₋₁₄ aryloxy group are unsubstituted or substituted with one or more substituent(s) that are the same as or different from each other selected from a group V¹ of substituents and a group V² of substituents) or
two adjacent substituents of R⁰ and R¹, R¹ and R², R² and R³, R⁴ and R⁵, or R⁵ and R⁶ together with a benzene ring to which these substituents are bonded form a C₆₋₁₄ polycyclic fused ring (the C₆₋₁₄ polycyclic fused ring is unsubstituted or substituted with one or more substituent(s) that are the same as or different from each other selected from a group V¹ of substituents and a group V² of substituents),
where the group V¹ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfozayl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V² of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V¹ of substituents)].

2. The optically active dinickel complex according to claim 1 of Formula (II) or Formula (II'): 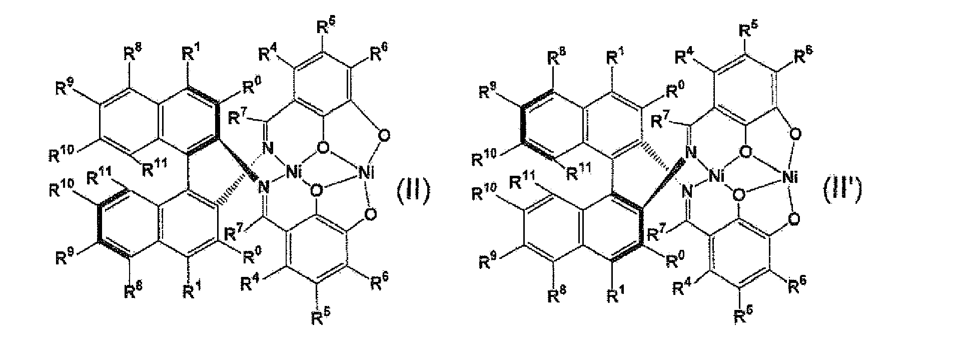 [where R⁰, R¹, R⁴, R⁵, R⁶, R⁷, R⁸, R⁹, R¹⁰, and R¹¹ are each independently a hydrogen atom, a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, the C₂₋₉ heterocyclyl group, the C₂₋₁₄ aryl group, and the C₂₋₁₄ aryloxy group are unsubstituted or substituted with one or more substituent(s) that are the same as or different from each other selected from the group V¹ of substituents and the group V² of substituents) or
two adjacent substituents of R⁰ and R¹, R⁴ and R⁵, R⁵ and R⁶, R⁸ and R⁹, R⁹ and R¹⁰, or R¹⁰ and R¹¹ together with a benzene ring to which these substituents are bonded form a C₆₋₁₄ polycyclic fused ring (the C₆₋₁₄ polycyclic fused ring is unsubstituted or substituted with one or more substituent(s) that are the same as or different from each other selected from the group V¹ of substituents and the group V² of substituents)
and the group V¹ of substituents and the group V² of substituents are the same as those defined in claim 1],
wherein two adjacent substituents of R² and R³ together with a benzene ring to which R² and R³ are bonded form a naphthalene ring.

3. The optically active dinickel complex according to claim 2, wherein R⁰, R¹, R⁸, R⁹, R¹⁰, and R¹¹ are each independently a hydrogen atom, a halogen atom, a C₁₋₁₀ alkyl group, or a C₁₋₁₀ alkoxy group.

4. The optically active dinickel complex according to claim 3, wherein R⁰, R¹, R⁸, R⁹, R¹⁰, and R¹¹ are a hydrogen atom.

5. The optically active dinickel complex according to any one of claims 1 to 4, wherein R⁴, R⁵, R⁶, and R⁷ are a hydrogen atom.

6. The optically active dinickel complex according to claim 1 or 2, wherein R⁴ and R⁵ together form, together with a benzene ring to which R⁴ and R⁵ are bonded, a ring structure of Formula (III) below; and R⁶ and R⁷ are a hydrogen atom.

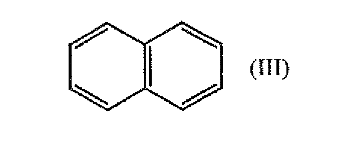

7. The optically active dinickel complex according to claim 2, wherein R⁴ and R⁵ together form, together with a benzene ring to which R⁴ and R⁵ are bonded, a ring structure of Formula (III) below; R⁶ and R⁷ are a hydrogen atom; and R⁰, R¹, R², R⁹, R¹⁰, and R¹¹ are each independently a hydrogen atom, a halogen atom, a C₁₋₁₀ alkyl group, or a C₁₋₁₀ alkoxy group.

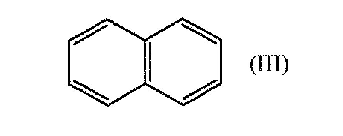

8. The optically active dinickel complex according to claim 7, wherein R⁰, R¹, R⁸, R⁹, R¹⁰, and R¹¹ are a hydrogen atom.

9. The optically active dinickel complex according to claim 1, wherein R⁴, R⁵, R⁶, and R⁷ are a hydrogen atom; and R⁰, R¹, R², and R³ are each independently a hydrogen atom, a halogen atom, a C₁₋₁₀ alkyl group, or a C₁₋₁₀ alkoxy group.

10. The optically active dinickel complex according to claim 1, wherein R⁰, R¹, R⁴, R⁵, R⁶, and R⁷ are a hydrogen atom; and R² and R³ together form, together with a benzene ring to which R² and R³ are bonded, a ring structure of Formula (IV) or Formula (V) below.

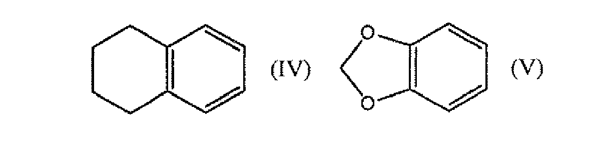

11. The optically active dinickel complex according to claim 1, wherein
R⁰, R¹, R⁶, and R⁷ are a hydrogen atom,
R⁴ and R⁵ together form, together with a benzene ring to which R⁴ and R⁵ are bonded, a ring structure of Formula (III) below, and
R² and R³ together form, together with a benzene ring to which R² and R³ are bonded, a ring structure of Formula (IV) or Formula (V) below.

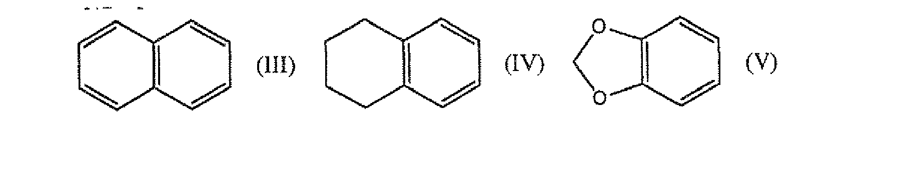

12. A production method of an optically active amide (VIII), **characterized by** comprising reacting a N-substituted imine of Formula (VI) with a compound of Formula (VII) using an optically active dinickel complex of Formula (I), (I'), (II), or (II') as a catalyst. 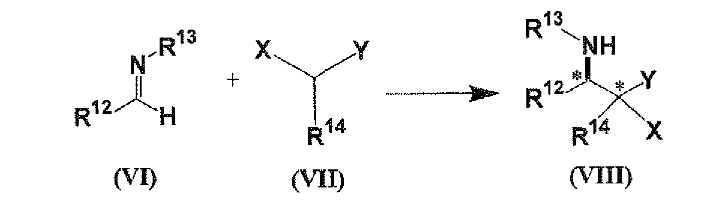 [where
R¹² is a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, a C₇₋₁₄ aralkyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, the C₇₋₁₄ aralkyl group, and the C₂₋₁₄ aryl group are unsubstituted or substituted with one or more substituent(s) that are the same as or different from each other selected from a group V³ of substituents and a group V⁴ of substituents);
R¹³ is a protective group for an amino group, a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents);
R¹⁴ is a hydrogen atom, a halogen atom, a C₁₋₁₀ alkyl group, a C₂₋₆ alkenyl group, a C₂₋₆ alkynyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents);
X and Y are each independently a nitro group, a cyano group, -C(O)R¹⁵, -C(O)OR¹⁵, - C(O)SR¹⁵, -C(S)OR¹⁵, -C(O)NR¹⁵R¹⁶, -S(O)₂R¹⁵, -S(O)₂NR¹⁵R¹⁶, -S(O)R¹⁵, or - P(O)(OR¹⁵)(OR¹⁶)
(where R¹⁵ and R¹⁶ are each independently a hydrogen atom, a C₁₋₁₀ alkyl group, a C₂₋₆ alkenyl group, a C₂₋₆ alkynyl group, a C₁₋₃ halogen-substituted alkyl group, a C₁₋₃ halogen-substituted alkoxy group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₃ halogen-substituted alkyl group, the C₁₋₃ halogen-substituted alkoxy group, the C₂₋₉ heterocyclyl group, and the C₂₋₁₄ aryl group are unsubstituted or substituted with one or more substituent(s) that are the same as or different from each other selected from the group V³ of substituents and the group V⁴ of substituents), or
R¹⁵ and R¹⁶ together are -(CR¹⁷R¹⁸)ₘ₁-G-(CR¹⁹R²⁰)ₘ₂- (where R¹⁷, R¹⁸, R¹⁹, and R²⁰ are each independently a hydrogen atom, a C₁₋₁₀ alkyl group, a C₁₋₃ halogen-substituted alkyl group, a C₁₋₃ halogen-substituted alkoxy group, a C₂₋₁₄ aryl group, a C₁₋₁₀ alkoxy group, a C₂₋₁₄ aryloxy group, a hydroxy group, or a protected hydroxy group; G is an oxygen atom, a sulfur atom, -CR²¹R²²- (where R²¹ and R²² are each independently a hydrogen atom, a C₁₋₁₀ alkyl group, a C₂₋₁₄ aryl group, a C₁₋₁₀ alkoxy group, a C₂₋₁₄ aryloxy group, a hydroxy group, or a protected hydroxy group), or - NR²³ (where R²³ is a hydrogen atom, a hydroxy group, a formyl group, a protective group for an amino group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (where the C₁₋₁₀ alkyl group, the C₂₋₆ alkenyl group, the C₂₋₆ alkynyl group, the C₁₋₁₀) alkoxy group, the C₁₋₁₀ alkylcarbonyloxy group, the C₁₋₁₀ alkoxycarbonyl group, and the C₁₋₁₀ alkylcarbonyl group are unsubstituted or substituted with a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, or a C₂₋₁₄ aryloxy group (where the C₂₋₁₄ aryl group and the C₂₋₁₄ aryloxy group are unsubstituted or substituted with a C₁₋₆ alkyl group (the C₁₋₆ alkyl group is unsubstituted or substituted with a halogen atom), or a halogen atom)), or a C₂₋₁₄ aryl group (the C₂₋₁₄ aryl group is unsubstituted or substituted with a C₁₋₁₀ alkyl group (the C₁₋₁₀ alkyl group is unsubstituted or substituted with a halogen atom), a C₂₋₆ alkenyl group, a C₂₋₆ alkynyl group, a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C1-10 alkylamino group, a hydroxy group, a protected hydroxy group, a C₂₋₁₄ aryl group, or a C₂₋₁₄ aryloxy group)); and m1 and m2 are each independently an integer of 0 to 5, where m1+m2 is 3, 4, or 5)),
where the group V³ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group. the C₁₋₁₀ alkylaminocarbonyl group, the C₁₋₁₀ alkylsulfonylamino group, the C₁₋₁₀ thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V⁴ of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V¹ of substituents), and the absolute configuration of a carbon atom marked with * means R or S].

13. The production method of the optically active amine according to claim 12, wherein
X is a nitro group, and
Y is -C(O)OR¹⁵ (where R¹⁵ is the same as defined in claim 12).

14. The production method of the optically active amine according to claim 12, wherein
X is -C(O) R¹⁵, and
Y is -C(O)OR¹⁵ (where R¹⁵ is the same as defined in claim 12 and R¹⁵ in X and R¹⁵ in Y are each independently defined).

15. The production method of the optically active amine according to claim 12, wherein
X is a cyano group, and
Y is -C(O)OR¹⁵ (where R¹⁵ is the same as defined in claim 12).

16. The production method of the optically active amine according to claim 12, wherein
X is -P(O)(OR¹⁵)(OR¹⁶), and
Y is -C(O)OR¹⁵ (where R¹⁵ and R¹⁶ are the same as defined in claim 12, and R¹⁵ in X and R¹⁵ in Y are each independently defined).

17. The production method of the optically active amine according to claim 12, wherein
X is -C(O)OR¹⁵, and
Y is -C(O)OR¹⁵ (where R¹⁵ is the same as defined in claim 12, and R¹⁵ in X and R¹⁵ in Y are each independently defined).

18. The production method of the optically active amine according to claim 12, wherein
X is a nitro group, and
Y is -C(O)R¹⁵ (where R¹⁵ is the same as defined in claim 12).

19. The production method of the optically active amine according to claim 12, wherein
X is a cyano group, and
Y is -C(O)R¹⁵ (where R¹⁵ is the same as defined in claim 12).

20. The production method of the optically active amine according to claim 12, wherein
X is -P(O)(OR¹⁵)(OR¹⁶), and
Y is -C(O)R¹⁵ (where R¹⁵ and R¹⁶ are the same as defined in claim 12, and R¹⁵ in X and R¹⁵ in Y are each independently defined).

21. The production method of the optically active amine according to claim 12, wherein
X is -C(O)R¹⁵, and
Y is -C(O)R¹⁵ (where R¹⁵ is the same as defined in claim 12, and R¹⁵ in X and R¹⁵ in Y are each independently defined).

22. A production method of an optically active β-amine-cyclic carbonyl compound (X), **characterized by** comprising reacting a N-substituted imine of Formula (VI) with a cyclic compound of Formula (IX) using an optically active dinickel complex of Formula (I), (I'), (II), or (II') as a catalyst. 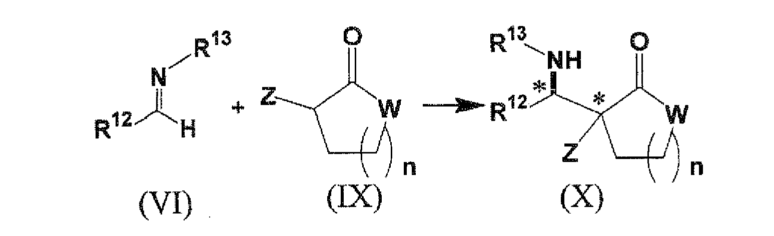 [where R¹² is a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, a C₇₋₁₄ aralkyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, the C₇₋₁₄ aralkyl group, and the C₂₋₁₄ aryl group are unsubstituted or substituted with one or more substituent(s) that are the same as or different from each other selected from a group V⁵ of substituents and a group V⁶ of substituents);
R¹³ is a protective group for an amino group, a C₁₋₁₀ alkyl group, a C₂₋₆ alkenyl group, a C₂₋₆ alkynyl group, a C₁₋₁₀ alkoxycarbonyl group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₁₀ alkoxycarbonyl group, the C₂₋₉ heterocyclyl group, and the C₂₋₁₄ aryl group are unsubstituted or substituted with one or more substituent(s) that are the same as or different from each other selected from the group V⁵ of substituents and the group V⁶ of substituents);
Z is a nitro group, a cyano group, -C(O)R¹⁵, -C(O)OR¹⁵, -C(O)SR¹⁵, -C(S)OR¹⁵, - C(O)NR¹⁵R¹⁶, -S(O)₂R¹⁵, -S(O)₂NR¹⁵R¹⁶, -S(O)R¹⁵, or -P(O)(OR¹⁵)(OR¹⁶) (where R¹⁵ and R¹⁶ are each independently a hydrogen atom, a C₁₋₁₀ alkyl group, a C₂₋₆ alkenyl group, a C₂₋₆ alkynyl group, a C₁₋₃ halogen-substituted alkyl group, a C₁₋₃ halogen-substituted alkoxy group, a C₂₋₉ heterocyclyl group, or a C₂₋₁₄ aryl group (where the C₁₋₁₀ alkyl group, the C₂₋₆ alkenyl group, the C₂₋₆ alkynyl group, the C₁₋₃ halogen-substituted alkyl group, the C₁₋₃ halogen-substituted alkoxy group, the C₂₋₉ heterocyclyl group, and the C₂₋₁₄ aryl group are unsubstituted or substituted with one or more substituent(s) that are the same as or different from each other selected from the group V⁵ of substituents and the group V⁶ of substituents),
or
R¹⁵ and R¹⁶ together are -(CR¹⁷R¹⁸)ₘ₁-G-(CR¹⁹R²⁰)ₘ₂- (where R¹⁷, R¹⁸, R¹⁹, and R²⁰ are each independently a hydrogen atom, a C₁₋₁₀ alkyl group, a C₂₋₁₄ aryl group, a C₁₋₁₀ alkoxy group, a C₂₋₁₄ aryloxy group, a hydroxy group, or a protected hydroxy group; G is an oxygen atom, a sulfur atom, -CR²¹R²²- (where R²¹ and R²² are each independently a hydrogen atom, a C₁₋₁₀ alkyl group, a C₂₋₁₄ aryl group, a C₁₋₁₀ alkoxy group, a C₂₋₁₄ aryloxy group, a hydroxy group, or a protected hydroxy group), or - NR²³ (where R²³ is a hydrogen atom, a hydroxy group, a formyl group, a C₁₋₁₀ alkyl group, a C₂₋₆ alkenyl group, a C₂₋₆ alkynyl group, a C₁₋₁₀ alkoxy group, a C₁₋₁₀ alkylcarbonyloxy group, a C₁₋₁₀ alkoxycarbonyl group, a C₁₋₁₀ alkylcarbonyl group (where the C₁₋₁₀ alkyl group, the C₂₋₆ alkenyl group, the C₂₋₆ alkynyl group, the C₁₋₁₀ alkoxy group, the C₁₋₁₀ alkylcarbonyloxy group, the C₁₋₁₀ alkoxycarbonyl group, and the C₁₋₁₀ alkylcarbonyl group are unsubstituted or substituted with a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, or a C₂₋₁₄ aryloxy group (where the C₂₋₁₄ aryl group and the C₂₋₁₄ aryloxy group are unsubstituted or substituted with a C₁₋₆ alkyl group (the C₁₋₆ alkyl group is unsubstituted or substituted with a halogen atom) or a halogen atom)), or a C₂₋₁₄ aryl group (the C₂₋₁₄ aryl group is unsubstituted or optionally substituted with one or a plurality of substituent(s) selected from a C₁₋₁₀ alkyl group (the C₁₋₁₀ alkyl group is unsubstituted or substituted with a halogen atom), a C₂₋₆ alkenyl group, a C₂₋₆ alkynyl group, a carboxyl group, a nitro group, a cyano group, a halogen atom, a C₁₋₁₀ alkoxy group, a C₁₋₁₀ alkylcarbonyl group, a C₁₋₁₀ alkylcarbonyloxy group, a C₁₋₁₀ alkoxycarbonyl group, a C₁₋₁₀ alkylcarbonylamino group, an amino group, a mono or di C₁₋₁₀ alkylamino group, a hydroxy group, a protected hydroxy group, a C₂₋₁₄ aryl group, and a C₂₋₁₄ aryloxy group)); and m1 and m2 are each independently an integer of 0 to 5, where m1+m2 is 3, 4, or 5));
W is an oxygen atom, a sulfur atom, -NR²⁴, or -C(R²⁴R²⁵)- (where R²⁴ and R²⁵ are a hydrogen atom, a C₁₋₁₀ alkyl group, a C₂₋₆ alkenyl group, a C₂₋₆ alkynyl group, a C₂₋₁₄ aryl group, or a C₁₋₁₀ alkoxycarbonyl group (where the C₁₋₁₀ alkyl group, the C₂₋₆ alkenyl group, the C₂₋₆ alkynyl group, the C₂₋₁₄ aryl group, and the C₁₋₁₀ alkoxycarbonyl group are unsubstituted or substituted with one or more substituent(s) that are the same as or different from each other selected from the group V⁵ of substituents and the group V⁶ of substituents)); and
n is 1, 2, or 3,
where the group V⁵ of substituents consists of a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, and a C₂₋₉ heterocyclyl group (where the C₁₋₃ halogen-substituted alkyl group, the C₁₋₃ halogen-substituted alkoxy group, the C₁₋₁₀ alkyl group, the C₂₋₆ alkenyl group, the C₂₋₆ alkynyl group, the C₁₋₁₀ alkylcarbonyloxy group, the C₁₋₁₀ alkoxycarbonyl group, the C₁₋₁₀ alkoxy group, the C₁₋₁₀ alkylcarbonyl group, the C₁₋₁₀ alkylcarbonylamino group, the mono or di C₁₋₁₀ alkylamino group, the C₁₋₁₀ alkylsulfonyl group, the C₁₋₁₀ alkylaminosulfonyl group, the C₁₋₁₀ alkylaminocarbonyl group, the C₁₋₁₀ alkylsulfonylamino group, the C₁₋₁₀ thioalkyl group, and the C₂₋₉ heterocyclyl group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, a C₂₋₉ heterocyclyl group, a C₂₋₁₄ aryl group, or a C₂₋₁₄ aryloxy group (where the C₂₋₁₄ aryl group and the C₂₋₁₄ aryloxy group are unsubstituted or substituted with a hydroxy group, a protected hydroxy group, an amino group, a protected amino group, a thiol group, a protected thiol group, a nitro group, a cyano group, a halogen atom, a carbamoyl group, a sulfamoyl group, a carboxy group, a sulfo group, a formyl group, a C₁₋₃ halogen-substituted alkyl group, a C₁₋₃ halogen-substituted alkoxy group, a C₁₋₁₀ alkyl group, a C₂₋₆ alkenyl group, a C₂₋₆ alkynyl group, a C₁₋₁₀ alkylcarbonyloxy group, a C₁₋₁₀ alkoxycarbonyl group, a C₁₋₁₀ alkoxy group, a C₁₋₁₀ alkylcarbonyl group, a C₁₋₁₀ alkylcarbonylamino group, a mono or di C₁₋₁₀ alkylamino group, a C₁₋₁₀ alkylsulfonyl group, a C₁₋₁₀ alkylaminosulfonyl group, a C₁₋₁₀ alkylaminocarbonyl group, a C₁₋₁₀ alkylsulfonylamino group, a C₁₋₁₀ thioalkyl group, or a C₂₋₉ heterocyclyl group)), and
the group V⁶ of substituents consists of a C₂₋₁₄ aryl group and a C₂₋₁₄ aryloxy group (where the C₂₋₁₄ aryl group and the C₂₋₁₄ aryloxy group are unsubstituted or substituted with one or more substituent(s) that are the same as or different from each other selected from the group V⁵ of substituents), and the absolute configuration of a carbon atom marked with * means R or S.]

23. The production method of the optically active amine according to claim 22, wherein Z is a nitro group.

24. The production method of the optically active amine according to claim 22, wherein Z is a cyano group.

25. The production method of the optically active amine according to claim 22, wherein Z is -C(O)R¹⁵ (where R¹⁵ is the same as defined in claim 22).

26. The production method of the optically active amine according to claim 22, wherein Z is -C(O)OR¹⁵ (where R¹⁵ is the same as defined in claim 22).

27. The production method of the optically active amine according to claim 22, wherein Z is -P(O)(OR¹⁵)(OR¹⁶) (where R¹⁵ and R¹⁶ are the same as defined in claim 22).

28. The production method according to any one of claims 22 to 27, wherein
W is -CH₂-, and
n is 1 or 2.

29. The production method according to any one of claims 22 to 27, wherein
W is an oxygen atom, and
n is 1 or 2.

30. The production method according to any one of claims 12 to 29, wherein, R¹³ in the N-substituted imine of Formula (VI), is a Boc group.

31. The production method according to any one of claims 12 to 29, wherein, R¹³ in the N-substituted imine of Formula (VI), is a Cbz group.

32. The production method according to any one of claims 12 to 31, **characterized in that** the reaction is carried out in the presence of an additive having a dehydration activity.

33. The production method according to any one of claims 12 to 32, wherein the catalyst used in the reaction is the optically active dinickel complex as claimed in claim 1.

34. The production method according to any one of claims 12 to 32, wherein the catalyst used in the reaction is the optically active dinickel complex as claimed in claim 2.

35. The production method according to any one of claims 12 to 32, wherein the catalyst used in the reaction is the optically active dinickel complex as claimed in claim 3.

36. The production method according to any one of claims 12 to 32, wherein the catalyst used in the reaction is the optically active dinickel complex as claimed in claim 4.

37. The production method according to any one of claims 12 to 32, wherein the catalyst used in the reaction is the optically active dinickel complex as claimed in claim 5.

38. The production method according to any one of claims 12 to 32, wherein the catalyst used in the reaction is the optically active dinickel complex as claimed in claim 6.

39. The production method according to any one of claims 12 to 32, wherein the catalyst used in the reaction is the optically active dinickel complex as claimed in claim 7.

40. The production method according to any one of claims 12 to 32, wherein the catalyst used in the reaction is the optically active dinickel complex as claimed in claim 8.

41. The production method according to any one of claims 12 to 32, wherein the catalyst used in the reaction is the optically active dinickel complex as claimed in claim 9.

42. The production method according to any one of claims 12 to 32, wherein the catalyst used in the reaction is the optically active dinickel complex as claimed in claim 10.

43. The production method according to any one of claims 12 to 32, wherein the catalyst used in the reaction is the optically active dinickel complex as claimed in claim 11.
